# EUROPEAN PATENT SPECIFICATION

(11) **EP 2 603 218 B1**
(45) Date of publication and mention of the grant of the patent: **02.03.2016**
(21) Application number: 11816959.8
(22) Date of filing: 10.08.2011
(51) Int. Cl.: A61K 31/4706, C07D 401/12, A61P 19/00, C07D 405/14

(54) **QUINOLYL AMINES AS KINASE INHIBITORS**
CHINOLYLAMINE ALS KINASEHEMMER
QUINOLYLAMINES EN TANT QU'INHIBITEURS DE KINASES

(30) Priority: 10.08.2010 US 372342 P
(43) Date of publication of application: 19.06.2013
(73) Proprietor: GlaxoSmithKline Intellectual Property Development Limited, Brentford Middlesex TW8 9GS (GB)
(72) Inventor: CASILLAS, Linda, N., Collegeville, PA 19426 (US); CHARNLEY, Adam, Kenneth, Collegeville, PA 19426 (US); HAILE, Pamela, A, Collegeville, PA 19426 (US); HUGHES, Terry, Vincent, Collegeville, PA 19426 (US); MARQUIS, Robert, W., Collegeville, PA 19426 (US); MEHLMANN, John, F, Collegeville, PA 19426 (US); REILLY, Michael, Collegeville, PA 19426 (US); ROMANO, Joseph, J., Collegeville, PA 19426 (US); SINGHAUS, Robert, R., Collegeville, PA 19426 (US)
(74) Representative: Price, Susanna Clare Hopley
(86) International application number: PCT/US2011/047183
(87) International publication number: WO 2012/021580

(56) References cited:
- EP-B1- 0 973 746
- WO-A1-02/092571
- US-A1- 2002 026 052
- US-B2- 7 282 504
- US-B2- 7 282 504
- CAVASOTTO ET AL.: 'In silico identification of novel EGFR inhibitors with antiproliferative activity against cancer cells' BIOORGANIC & MEDICINAL CHEMISTRY LETTERS vol. 16, 2006, pages 1969 - 1974, XP027966378

## Description

### BACKGROUND OF THE INVENTION

### Field of the Invention

The present invention relates to N-pyrazolyl, N-quinolyl amines that inhibit RIP2 kinase and methods of making and using the same. Specifically, the present invention relates to substituted N-pyrazolyl, N-quinolyl amines as RIP2 kinase inhibitors.

### Background of the Invention

Receptor interacting protein-2 (RIP2) kinase, which is also referred to as CARD3, RICK, CARDIAK, or RIPK2, is a TKL family serine/threonine protein kinase involved in innate immune signaling. RIP2 kinase is composed of an N-terminal kinase domain and a C-terminal caspase-recruitment domain (CARD) linked via an intermediate (IM) region ((1998) J. Biol. Chef. 273, 12296-12300; (1998) Current Biology 8, 885-889; and (1998) J. Biol. Chem. 273, 16968-16975). The CARD domain of RIP2 kinase mediates interaction with other CARD-containing proteins, such as NOD1 and NOD2 ((2000) J. Biol. Chem. 275, 27823-27831 and (2001) EMBO reports 2, 736-742). NOD1 and NOD2 are cytoplasmic receptors which play a key role in innate immune surveillance. They recognize both gram positive and gram negative bacterial pathogens and are activated by specific peptidoglycan motifs, diaminopimelic acid (i.e., DAP) and muramyl dipeptide (MDP), respectively ((2007) J Immunol 178, 2380-2386).

Following activation, RIP2 kinase associates with NOD1 or NOD2 and appears to function principally as a molecular scaffold to bring together other kinases (TAK1, IKKα/(β/γ) involved in NF-κB and mitogen-activated protein kinase activation ((2006) Nature Reviews Immunology 6, 9-20). RIP2 kinase undergoes a K63-linked polyubiquitination on lysine-209 which facilitates TAK1 recruitment ((2008) EMBO Journal 27, 373-383). This post-translational modification is required for signaling as mutation of this residue prevents NOD1/2 mediated NF-kB activation. RIP2 kinase also undergoes autophosphorylation on serine-176, and possibly other residues ((2006) Cellular Signalling 18, 2223-2229). Studies using kinase dead mutants (K47A) and non-selective small molecule inhibitors have demonstrated that RIP2 kinase activity is important for regulating the stability of RIP2 kinase expression and signaling ((2007) Biochem J 404, 179-190 and (2009) J. Biol. Chem. 284, 19183-19188).

Dysregulation of RIP2-dependent signaling has been linked to auto inflammatory diseases. Gain-of-function mutations in the NACHT-domain of NOD2 cause Blau Syndrome/Early-onset Sarcoidosis, a pediatric granulomateous disease characterized by uveitis, dermatitis, and arthritis ((2001) Nature Genetics 29, 19-20; (2005) Journal of Rheumatology 32, 373-375; (2005) Current Rheumatology Reports 7, 427-433; (2005) Blood 105, 1195-1197; (2005) European Journal of Human Genetics 13, 742-747; (2006) American Journal of Ophthalmology 142, 1089-1092; (2006) Arthritis & Rheumatism 54, 3337-3344; (2009) Arthritis & Rheumatism 60, 1797-1803; and (2010) Rheumatology 49, 194-196). Mutations in the LRR-domain of NOD2 have been strongly linked to susceptibility to Crohn's Disease ((2002) Am. J. Hum. Genet. 70, 845-857; (2004) European Journal of Human Genetics 12, 206-212; (2008) Mucosal Immunology (2008) 1 (Suppl 1), SS-S9. 1, S5-S9; (2008) Inflammatory Bowel Diseases 14, 295-302; (2008) Experimental Dermatology 17, 1057-1058; (2008) British Medical Bulletin 87, 17-30; (2009) Inflammatory Bowel Diseases 15, 1145 - 1154 and (2009) Microbes and Infection 11, 912-918). Mutations in NOD1 have been associated with asthma ((2005) Hum. Mol. Genet. 14, 935-941) and early-onset and extra-intestinal inflammatory bowel disease ((2005) Hum. Mol. Genet. 14, 1245-1250). Genetic and functional studies have also suggested a role for RIP2-dependent signaling in a variety of other granulomateous disorders, such as sarcoidosis ((2009) Journal of Clinical Immunology 29, 78-89 and (2006) Sarcoidosis Vasculitis and Diffuse Lung Diseases 23, 23-29) and Wegner's Granulomatosis ((2009) Diagnostic Pathology 4, 23).

A potent, selective, small molecule inhibitor of RIP2 kinase activity would block RIP2-dependent pro-inflammatory signaling and thereby provide a therapeutic benefit in autoinflammatory diseases characterized by increased and/or dysregulated RIP2 kinase activity.

WO2002/092571A1 describes 4-anilinoquinoline-3-carboxamides which are JAK3 kinase inhibitors, methods for their preparation and pharmaceutical compositions thereof. WO2002/092571 A1 also discloses their use in the treatment of diseases or conditions mediated by JAK3 kinases such as asthma, host versus graft rejection/transplantation and rheumatoid arthritis.

US 2002/026052A1 describes 3-cyanoquinolines, 3-cyano-1,6-naphthyridines and 3-cyano-1,7-naphthyridines as protein kinase inhibitors, which are useful as antineoplastic agents and in the treatment of osteoporosis and polycystic kidney disease.

US 7,282,504 B2 relates to pyrimidine derivatives which are kinase inhibitors, compositions comprising the inhibitors, and methods of using the inhibitors. The invention also provides for methods of making kinase inhibitor compounds and methods for treating disease or disease symptoms, particularly those mediated by an enzyme or polypeptide such as phosphoryl transferase mediated or kinase mediated disease or disease symptoms.

### SUMMARY OF THE INVENTION

The invention is directed to novel N-pyrazolyl, N-quinolyl amine compounds according to Formula (I): 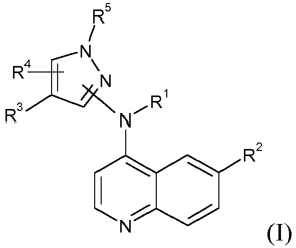 wherein:
R¹ is H, -SO₂(C₁-C₄)alkyl, -CO(C₁-C₄)alkyl, or (C₁-C₄)alkyl;
R² is - SR^{a}, -SOR^{a}, or -SO₂R^{a}, wherein R^{a} is (C₁-C₆)alkyl, halo(C₁-C₆)alkyl, (C₃-C₇)cycloalkyl, 4-7 membered heterocycloalkyl, aryl, or heteroaryl, wherein:
   said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄)alkyl, (C₃-C₇)cycloalkyl, phenyl, 5-6 membered heteroaryl, 9-10 membered heteroaryl, 4-7 membered heterocycloalkyl and (phenyl)(C₁-C₄ alkyl)amino-, wherein said (C₃-C₇)cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl and (C₁-C₄)alkoxy,
   said (C₃-C₇)cycloalkyl or 4-7 membered heterocycloalkyl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl-, oxo and (C₁-C₄)alkoxy, and
   said aryl is an aromatic, monovalent monocyclic or bicyclic hydrocarbon radical containing from 6 to 10 carbon ring atoms, which may be fused to one or more cycloalkyl rings; and said heteroaryl is an aromatic monovalent monocyclic or bicyclic radical, containing 5 to 10 ring atoms, including 1 to 4 heteroatoms selected from nitrogen, oxygen and sulfur, and wherein said aryl or heteroaryl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl- and (C₁-C₄)alkoxy;
   R³ is methyl, hydroxymethyl, trifluoromethyl, carboxy, or -CO₂(C₁-C₄)alkyl;
   R⁴ is H, methyl, hydroxymethyl, trifluoromethyl, carboxy, or -CO₂(C₁-C₄)alkyl;
   R⁵ is H or (C₁-C₃)alkyl;
   or a salt, particularly a pharmaceutically acceptable salt, thereof, or hydrate thereof

The compounds of the invention are inhibitors of RIP2 kinase and may be useful for the treatment of RIP2 kinase-mediated diseases and disorders particularly uveitis, Crohn's disease, ulcerative colitis, early-onset and extra-intestinal inflammatory bowel disease and granulomateous disorders, such as sarcoidosis, Blau syndrome/early-onset sarcoidosis and Wegner's Granulomatosis. Accordingly, the invention is further directed to a compound according to Formula (I), or a pharmaceutically acceptable salt thereof, for use in the treatment of a RIP2 kinase-mediated disease or condition in a patient (particularly, a human). The present invention is also directed to pharmaceutical compositions comprising a compound of the invention. The invention is still further directed to the use of a compound of the invention or a pharmaceutical composition comprising a compound of the invention in the manufacture of a medicament for use in the treatment of a RIP2 kinase-mediated disease or disorder.

### BRIEF DESCRIPTION OF THE DRAWINGS

Figure 1 shows the combined cytokine response in rat whole blood samples obtained after pre-dosing the rats with the compound of Example 1 or prednisolone, followed by dosing with L18-MDP.
Figure 2 shows the combined cytokine response in rat whole blood samples obtained after pre-dosing the rats with the compound of Example 3 or prednisolone, followed by dosing with L18-MDP.

### DETAILED DESCRIPTION OF THE INVENTION

The alternative definitions for the various groups and substituent groups of Formula (I) provided throughout the specification are intended to particularly describe each compound species disclosed herein, individually, as well as groups of one or more compound species. The scope of this invention includes any combination of these group and substituent group definitions. The compounds of the invention are only those which are contemplated to be "chemically stable" as will be appreciated by those skilled in the art.

It will be appreciated by those skilled in the art that the compounds of Formula (I) may be may be alternatively represented as Formula (Ia):

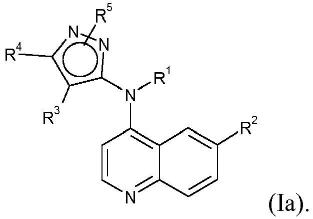

In another embodiment, the invention is directed to a compound of Formula (I). Specifically, the invention is directed to a compound according to Formula (I) wherein:
R¹ is H, -SO₂(C₁-C₄alkyl), -CO(C₁-C₄alkyl), or (C₁-C₄alkyl);
R² is -SR^{a} or -SO₂R^{a}, wherein R^{a} is (C₁-C₆)alkyl, (C₃-C₇)cycloalkyl, 4-7 membered heterocycloalkyl, aryl, or heteroaryl, wherein:
   said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), (C₃-C₇)cycloalkyl, phenyl, 5-6 membered heteroaryl, 9-10 membered heteroaryl, 4-7 membered heterocycloalkyl and (phenyl)(C₁-C₄ alkyl)amino-, wherein said (C₃-C₇)cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
   said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl-, oxo and (C₁-C₄)alkoxy, and
   said aryl is an aromatic, monovalent monocyclic or bicyclic hydrocarbon radical containing from 6 to 10 carbon ring atoms, which may be fused to one or more cycloalkyl rings; and said heteroaryl is an aromatic monovalent monocyclic or bicyclic radical, containing 5 to 10 ring atoms, including 1 to 4 heteroatoms selected from nitrogen, oxygen and sulfur, and wherein said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
   R³ is methyl or trifluoromethyl (-CH₃ or -CF₃);
   R⁴ is H, methyl or trifluoromethyl (-CH₃ or -CF₃);
   R⁵ is H or (C₁-C₃)alkyl;
   or a salt, particularly a pharmaceutically acceptable salt, thereof

It will be appreciated by those skilled in the art that the compounds of this invention may exist as pyrazole isomers represented by Formula (I-A) and Formula (I-B):

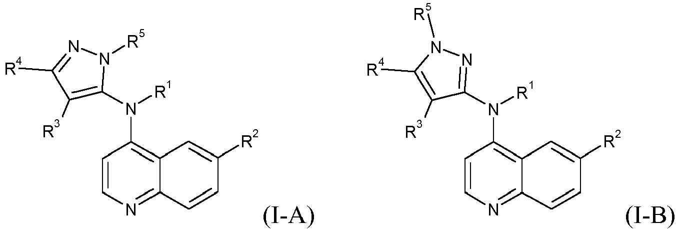

When R⁵ is H, the compounds of this invention may exist as tautomers. However, when R⁵ is (C₁-C₃)alkyl, the compounds of this invention, may exist as either one of the regioisomers represented by Formula (I-A) or Formula (I-B), or as a mixture thereof.

In addition, it will be appreciated by those skilled in the art that the compounds of this invention, depending on further substitution, may exist in other tautomeric forms. All tautomeric forms of the compounds described herein are intended to be encompassed within the scope of the present invention. It is to be understood that any reference to a named compound of this invention is intended to encompass all tautomers of the named compound and any mixtures of tautomers of the named compound.

In one embodiment of this invention, R¹ is H. In other embodiments, R¹ is -SO₂(C₁-C₄alkyl) or -CO(C₁-C₄alkyl); specifically, -SO₂CH; or -COCH₃. In other embodiments, R¹ is (C₁-C₂)alkyl; specifically, -CH₃. In specific embodiments, R¹ is H or -CH₃; generally, R¹ is H.

In another embodiment, R² is -SR^{a} or -SO₂R^{a}. In yet another embodiment, R² is -SOR^{a}. In further embodiment, R² is -SO₂R^{a}. In these embodiments, R^{a} is (C₁-C₆)alkyl, (C₃-C₆)cycloalkyl, 4-6-membered heterocycloalkyl, 5-6-membered heteroaryl or phenyl;
wherein said (C₁-C₆)alkyl is optionally substituted by one or two groups each independently selected from hydroxyl, (C₁-C₄)alkoxy, -CO₂(C₁-C₄)alkyl, -SO₂(C₁-C₄)alkyl, and a (C₃-C₆)cycloalkyl, phenyl, 4-6-membered heterocycloalkyl, 5-6-membered heteroaryl, or 9-10-membered heteroaryl, where said (C₃-C₆)cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy; and
wherein said (C₃-C₆)cycloalkyl, 4-6-membered heterocycloalkyl, 5-6-membered heteroaryl or phenyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy.

When R^{a} is a heterocycloalkyl or heteroaryl group, it is to be understood that the heterocycloalkyl or heteroaryl group is bonded to the sulfur atom of the -SR^{a} or -SO₂R^{a}, moiety by a ring carbon atom.

In a still further embodiment, R^{a} is (C₁-C₆)alkyl, 4-6-membered heterocycloalkyl, 5-6-membered heteroaryl or phenyl, wherein:
said (C₁-C₆)alkyl is optionally substituted by one or two groups each independently selected from hydroxyl, (C₁-C₂)alkoxy, (C₁-C₂)alkoxy(C₂-C₃)alkoxy-, -SO₂(C₁-C₂)alkyl, and a group selected from (C₃-C₆)cycloalkyl (optionally substituted by (C₁-C₄)alkyl or hydroxy(C₁-C₄)alkyl), 4-6-membered heterocycloalkyl (optionally substituted by (C₁-C₄)alkyl or halogen), 5-6-membered heteroaryl (optionally substituted by (C₁-C₄)alkyl or hydroxy(C₁-C₄)alkyl), phenyl, and 9-10-membered heteroaryl

In a still further embodiment, R^{a} is (C₁-C₆)alkyl or (C₃-C₆)cycloalkyl;
wherein said (C₁-C₆)alkyl is optionally substituted by one or two groups each independently selected from hydroxyl, (C₁-C₄)alkoxy, -SO₂(C₁-C₄)alkyl, and a (C₃-C₆)cycloalkyl, phenyl, 4-6-membered heterocycloalkyl, 5-6-membered heteroaryl, or 9-10-membered heteroaryl, where said (C₃-C₆)cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy; and
wherein said (C₃-C₆)cycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and(C₁-C₄)alkoxy.

In a specific embodiment, R^{a} is a (C₁-C₆)alkyl or a (C₁-C₆)alkyl substituted by one hydroxyl group.

In another embodiment, R^{a} is halo(C₁-C₄)alkyl containing 1-9 halogen atoms. In specific embodiments, R^{a} is halo(C₁-C₂)alkyl, specifically a halo(C₁-C₂)alkyl containing 1-5 halogen atoms, and more specifically a halo(C₁-C₂)alkyl containing 3 halogen atoms.

In a more specific embodiment, R^{a} is -CH₃, -CF₃, -CH₂CH₃, -CH₂CF₃, -CH₂CH₂CH₃, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CH₂CH₂OH, -C(CH₃)₂CH₂CH₂OCH₃, -CH₂CH₂SO₂CH₃ or -C(CH₃)₂CO₂CH₃.

In another specific embodiment, R^{a} is -CH₃, -CH₂CH₃, -CH₂CH₂CH₃, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CH₂CH₂OH, -C(CH₃)₂CH₂CH₂OCH₃, -CH_{2C}H₂SO₂CH₃ or -C(CH₃)₂CO₂CH₃.

In another embodiment, R^{a} is [1-(2-hydroxyethyl)cyclopropyl]methyl-, cyclopropyl, cyclopentyl, cyclohexyl, oxetan-3-yl, 3-methyl-oxetan-3-yl, tetrahydro-2H-pyran-4-yl, (4-fluoro)-tetrahydro-2H-pyran-4-yl, (4-methyl)-tetrahydro-2H-pyran-4-yl, -CH₂-tetrahydro-2*H-*furan-2-yl, tetrahydro-2H-furan-3-yl, 2-methyl-tetrahydro-2*H-*furan-3-yl, -CH₂-tetrahydro-2*H-*pyran-4-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, -CH₂-piperidin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂- imidazol-1-yl, -CH₂CH₂-imidazol-4-yl, (2S)-1-hydroxy-3-(1*H*-imidazol-4-yl)prop-2-yl, 3-[methyl(phenyl)amino]prop-1-yl or -CH₂CH₂CH₂-morpholin-4-yl.

In another embodiment, R^{a} is [1-(2-hydroxyethyl)cyclopropyl]methyl-, cyclopropyl, cyclopentyl, cyclohexyl, oxetan-3-yl, 3-methyl-oxetan-3-yl, tetrahydro-2H-pyran-4-yl, -CH₂-tetrahydro-2H-pyran-4-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, -CH₂-piperidin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, (2S)-1-hydroxy-3-(1*H*-imidazol-4-yl)prop-2-yl, 3-[methyl(phenyl)amino]prop-1-yl or -CH₂CH₂CH₂-morpholin-4-yl.

In a more specific embodiment, R^{a} is -CH₃, -CH₂CH₃, -CH₂CH₂CH₃, -CH(CH₃)₂, -CH(CH₃)CH₂CH₃, -C(CH₃)₃, or tetrahydro-2H-pyran-4-yl. In another specific embodiment, R^{a} is -CH₃, -CF₃, -CH₂CF₃, -CH(CH₃)₂, -C(CH₃)₃, -CH₂CH₂OH, (4-fluoro)-tetrahydro-2H-pyran-4-yl, (4-methyl)-tetrahydro-2H-pyran-4-yl, -CH₂-tetrahydro-2H-furan-2-yl, tetrahydro-2H-furan-3-yl, 2-methyl-tetrahydro-2H-furan-3-yl or tetrahydro-2H-pyran-4-yl. In a further specific embodiment, R^{a} is -C(CH₃)₃.

In another embodiment, R³ is methyl or trifluoromethyl (-CH₃ or -CF₃), R⁴ is methyl or trifluoromethyl (-CH₃ or -CF₃), and R⁵ is H or methyl (-CH₃). In another embodiment, R³ is methyl, hydroxymethyl (hydroxymethylene or HOCH₂-) or carboxy (-CO₂H), R⁴ is H, methyl or trifluoromethyl, and R⁵ is H or methyl. In another embodiment, R³ is methyl, R⁴ is H, methyl, trifluoromethyl, hydroxymethyl- or ethyl-carboxy-, and R⁵ is H or methyl. In a specific embodiment, the invention is directed to a compound wherein R³ is methyl; R⁴ is methyl or trifluoromethyl and R⁵ is H or (C₁-C₃)alkyl. More specifically, the invention is directed to a compound wherein R³ is methyl; R⁴ is methyl and R⁵ is H.

The invention is further directed to a compound of Formula (I), wherein:
R¹ is H, -CH₃, -SO₂CH₃ or -COCH₃;
R² is -SR^{a}, -SOR^{a}, or -SO₂R^{a}, wherein R^{a} is (C₁-C₆)afkyl or (C₃-C₆)cycloalkyl;
   wherein said (C₁-C₆)alkyl is optionally substituted by one or two groups each independently selected from hydroxyl, (C₁-C₄)alkoxy, -SO₂(C₁-C₄)alkyl, and a (C₃-C₆)cycloalkyl, phenyl, 4-6-membered heterocycloalkyl, 5-6-membered heteroaryl, or 9-10-membered heteroaryl, where said (C₃-C₆)cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy; and
   wherein said (C₃-C₆)cycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R³ is methyl or trifluoromethyl (-CH₃ or -CF₃);
R⁴ is methyl or trifluoromethyl (-CH₃ or -CF₃); and
R⁵ is H or (C₁-C₃)alkyl;
or a salt, particularly a pharmaceutically acceptable salt, thereof

In another embodiment, the invention is directed to a compound of Formula (I) wherein R¹ is H or -CH₃,; R² is -SR^{a}, -SOR^{a}, or -SO₂R^{a}, where R^{a} is -CH₃, -CF₃, -CH₂CH₃, -CH₂CF₃, -CH₂CH₂CH₃, -CH(CH₃)₂, -CH(CH₃)CH₂CH₃, -C(CH₃)₃, -CH₂CH₂OH, -CH₂CH₂OCH₃, -CH₂CH₂OCH₂CH₂OCH₃, -CH₂CH₂CH₂OH, -CH₂CH₂CH₂CH₂OH, -CH(CH₃)CH₂OH, -C(CH₃)₂CH₂OH, -C(CH₃)₂CH₂CH₂OH, -C(CH₃)₂CH₂CH₂OCH₃, -CH₂CH₂SO₂CH₃, -C(CH₃)₂CO₂CH₃, [1-(2-hydroxyethyl)cyclopropyl]methyl-, cyclopropyl, cyclopentyl, cyclohexyl, oxetan-3-yl, 3-methyl-oxetan-3-yl, tetrahydro-2H-pyran-4-yl, (4-fluoro)-tetrahydro-2H-pyran-4-yl, (4-methyl)-tetrahydro-2H-pyran-4-yl, -CH₂-tetrahydro-2H-furan-2-yl, tetrahydro-2H-furan-3-yl, 2-methyl-tetrahydro-2H-furan-3-yl, -CH₂-tetrahydro-2H-pyran-4-yl, phenyl, benzyl, -CH₂CH₂CH₂-phenyl, 4-amino-phenyl-, pyridin-4-yl, -CH₂-(6-methyl-pyridin-2-yl), piperidin-4-yl, 1-methyl-piperidin-4-yl-, -CH₂-piperidin-4-yl, -CH₂CH₂CH₂-morpholin-4-yl, pyrimidin-2-yl, -CH₂CH₂-indol-3-yl, 4,5-dimethyl-thiazol-2-yl, (3R)-1-benzyl-pyrrolidin-3-yl-, -CH₂CH₂-pyrrolidin-1-yl, -CH₂-benzimidazol-2-yl, -CH₂CH₂CH₂-imidazol-1-yl, -CH₂CH₂-imidazol-4-yl, (2S)-1-hydroxy-3-(1*H*-imidazol-4-yl)prop-2-yl, 3-[methyl(phenyl)amino]prop-1-yl or -CH₂CH₂CH₂-morpholin-4-yl; R³ is methyl; R⁴ is methyl; and R⁵ is H; or a salt, particularly a pharmaceutically acceptable salt, thereof

In another embodiment, the invention is directed to a compound of Formula (I) wherein R¹ is H or -CH₃; R² is -SR^{a}, -SOR^{a}, or -SO₂R^{a}, where R^{a} is -CH₃, -CH₂CH₃, -CH₂CH₂CH₃, -CH(CH₃)₂, -CH(CH₃)CH₂CH₃, -C(CH₃)₃, or tetrahydro-2H-pyran-4-yl.

In another specific embodiment, R^{a} is -CH₃, -CF₃, -CH₂CF₃, -CH(CH₃)₂, -C(CH₃)₃, -CH₂CH₂OH, (4-fluoro)-tetrahydro-2H-pyran-4-yl, (4-methyl)-tetrahydro-2H-pyran-4-yl, -CH₂-tetrahydro-2H-furan-2-yl, tetrahydro-2H-furan-3-yl, 2-methyl-tetrahydro-2H-furan-3-yl or tetrahydro-2H-pyran-4-yl; R³ is methyl; R⁴ is methyl; and R⁵ is H; or a salt, particularly a pharmaceutically acceptable salt, thereof, or hydrate thereof.

In another embodiment, the invention is directed to a compound of Formula (I) wherein R¹ is H or -CH₃; R² is -SR^{a}, -SOR^{a}, or -SO₂R^{a}; wherein R^{a} is (C₁-C₄)alkyl, halo(C₁-C₄)alkyl, hydroxy(C₁-C₄)alkyl, 5-6 membered heterocycloalkyl, or -(C₁-C₂)alkyl-(5-6 membered heterocycloalkyl), wherein any of said heterocycloalkyl contains one heteroatom selected from N, O and S; R³ is methyl, hydroxymethyl or carboxy (-CO₂H) and R⁴ is H, methyl or trifluoromethyl, or R³ is methyl and R⁴ is H, methyl, trifluoromethyl, hydroxymethyl- or ethyl-carboxy-; and R⁵ is H or (C₁-C₃)alkyl; or a salt, particularly a pharmaceutically acceptable salt, thereof

In another embodiment, the invention is directed to a compound of Formula (I) wherein R¹ is H; R² is -SR^{a} or -SO₂R^{a}, wherein R^{a} is (C₁-C₄)alkyl; R³ is methyl or hydroxymethyl and R⁴ is methyl or R³ is methyl and R⁴ is methyl, hydroxymethyl or trifluoromethyl; and R⁵ is H or (C₁-C₃)alkyl; or a salt, particularly a pharmaceutically acceptable salt, thereof, or hydrate thereof

In another embodiment, the invention is directed to a compound of Formula (I) wherein R¹ is H; R² is -SR^{a} or -SO₂R^{a}, wherein R^{a} is unsubstituted (C₁-C₄)alkyl; R³ is methyl; R⁴ is methyl or trifluoromethyl; and R⁵ is H or (C₁-C₃)alkyl; or a salt, particularly a pharmaceutically acceptable salt, thereof

In another embodiment, the invention is directed to a compound of Formula (I) wherein R¹ is H; R² is -SO₂R^{a}; wherein R^{a} is unsubstituted (C₁-C₄)alkyl; R³ is methyl; R⁴ is methyl or trifluoromethyl; and R⁵ is H or methyl; or a salt, particularly a pharmaceutically acceptable salt, thereof, or hydrate thereof

As used herein, the term "alkyl" represents a saturated, straight or branched hydrocarbon moiety. Exemplary alkyls include, but are not limited to methyl (Me), ethyl (Et), *n*-propyl, isopropyl, *n*-butyl, *s*-butyl, isobutyl, *t*-butyl and pentyl. The term "C₁-C₄ alkyl" refers to an alkyl group or moiety containing from 1 to 4 carbon atoms.

When the term "alkyl" is used in combination with other substituent groups, such as "haloalkyl" or "hydroxyalkyl" or "arylalkyl", the term "alkyl" is intended to encompass a divalent straight or branched-chain hydrocarbon radical. For example, "arylalkyl" is intended to mean the radical -alkylaryl, wherein the alkyl moiety thereof is a divalent straight or branched-chain carbon radical and the aryl moiety thereof is as defined herein, and is represented by the bonding arrangement present in a benzyl group (-CH₂-phenyl); "halo(C₁-C₄)alkyl" or "(C₁-C₄)haloalkyl" is intended to mean a radical having one or more halogen atoms, which may be the same or different, at one or more carbon atoms of an alkyl moiety containing from 1 to 4 carbon atoms, which a is straight or branched-chain carbon radical, and is represented by a trifluoromethyl group (-CF₃).

As used herein, the term "cycloalkyl" refers to a non-aromatic, saturated, cyclic hydrocarbon ring. The term "(C₃-C₈)cycloalkyl" refers to a non-aromatic cyclic hydrocarbon ring having from three to eight ring carbon atoms. Exemplary "(C₃-C₈)cycloalkyl" groups useful in the present invention include cyclopropyl, cyclobutyl, cyclopentyl, cyclohexyl, cycloheptyl, and cyclooctyl.

"Alkoxy" refers to a group containing an alkyl radical attached through an oxygen linking atom. The term "(C₁-C₄)alkoxy" refers to a straight- or branched-chain hydrocarbon radical having at least 1 and up to 4 carbon atoms attached through an oxygen linking atom. Exemplary "(C₁-C₄)alkoxy" groups useful in the present invention include, but are not limited to, methoxy, ethoxy, n-propoxy, isopropoxy, *n*-butoxy, *s*-butoxy, isobutoxy, and *t*-butoxy.

"Aryl" represents an aromatic, monovalent monocyclic or bicyclic hydrocarbon radical containing from 6 to 10 carbon ring atoms, which may be fused one or more cycloalkyl rings.

Generally, in the compounds of this invention, aryl is phenyl.

Heterocyclic groups may be heteroaryl or heterocycloalkyl groups.

"Heterocycloalkyl" represents a non-aromatic, monovalent monocyclic or bicyclic radical, which is saturated or partially unsaturated, containing 3 to 10 ring atoms, unless otherwise specified, which includes 1 to 4 heteroatoms selected from nitrogen, oxygen and sulfur. Illustrative examples of heterocycloalkyls include, but are not limited to, azetidinyl, oxetanyl, pyrrolidyl (or pyrrolidinyl), piperidinyl, piperazinyl, morpholinyl, tetrahydro-2H-1,4-thiazinyl, tetrahydrofuryl (or tetrahydrofuranyl), dihydrofuryl, oxazolinyl, thiazolinyl, pyrazolinyl, tetrahydropyranyl, dihydropyranyl, 1,3-dioxolanyl, 1,3-dioxanyl, 1,4-dioxanyl, 1,3-oxathiolanyl, 1,3-oxathianyl, 1,3-dithianyl, azabicylo[3.2.1]octyl, azabicylo[3.3.1]nonyl, azabicylo[4.3.0]nonyl, oxabicylo[2.2.1]heptyl and 1,5,9-triazacyclododecyl.

In some of the compounds of this invention, heterocycloalkyl groups include 4-membered heterocycloalkyl groups containing one heteroatom, such as oxetanyl, thietanyl and azetidinyl.

In other compounds of this invention, heterocycloalkyl groups include 5-membered heterocycloalkyl groups containing one heteroatom selected from nitrogen, oxygen and sulfur and optionally containing one or two an additional nitrogen atoms, or optionally containing one additional oxygen or sulfur atom, such as pyrrolidyl (or pyrrolidinyl), tetrahydrofuryl (or tetrahydrofuranyl), tetrahydrothienyl, dihydrofuryl, oxazolinyl, thiazolinyl, imidazolinyl, pyrazolinyl, 1,3-dioxolanyl, and 1,3-oxathiolan-2-on-yl.

In other compounds of this invention, heterocycloalkyl groups are 6-membered heterocycloalkyl groups containing one heteroatom selected from nitrogen, oxygen and sulfur and optionally containing one or two an additional nitrogen atoms or one additional oxygen or sulfur atom, such as piperidyl (or piperidinyl), piperazinyl, morpholinyl, thiomorpholinyl, 1,1 dioxoido-thiomorpholin-4-yl, tetrahydropyranyl, dihydropyranyl, tetrahydro-2H-1,4-thiazinyl, 1,4-dioxanyl, 1,3-oxathianyl, and 1,3-dithianyl.

"Heteroaryl" represents an aromatic monovalent monocyclic or bicyclic radical, containing 5 to 10 ring atoms, including 1 to 4 heteroatoms selected from nitrogen, oxygen and sulfur. This term also encompasses bicyclic heterocyclic-aryl compounds containing an aryl ring moiety fused to a heterocycloalkyl ring moiety, containing 5 to 10 ring atoms, including 1 to 4 heteroatoms selected from nitrogen, oxygen and sulfur. Illustrative examples of heteroaryls include, but are not limited to, thienyl, pyrrolyl, imidazolyl, pyrazolyl, furyl (or furanyl), isothiazolyl, isoxazolyl, oxazolyl, oxadiazolyl, thiazolyl, pyridyl (or pyridinyl), pyrazinyl, pyrimidinyl, pyridazinyl, triazinyl, tetrazinyl, triazolyl, tetrazolyl, benzo[b]thienyl, isobenzofuryl, 2,3-dihydrobenzofuryl, chromenyl, chromanyl, indolizinyl, isoindolyl, indolyl, indazolyl, purinyl, isoquinolyl, quinolyl, phthalazinyl, naphthridinyl, quinzolinyl, benzothiazolyl, benzimidazolyl, tetrahydroquinolinyl, cinnolinyl, pteridinyl, and isothiazolyl.

In some embodiments, the heteroaryl groups present in the compounds of this invention are 5-membered and/or 6-membered monocyclic heteroaryl groups. Selected 5-membered heteroaryl groups contain one nitrogen, oxygen or sulfur ring heteroatom, and optionally contain 1, 2 or 3 additional nitrogen ring atoms. Selected 6-membered heteroaryl groups contain 1, 2, 3 or 4 nitrogen ring heteroatoms. Selected 5- or 6-membered heteroaryl groups include thienyl, pyrrolyl, imidazolyl, pyrazolyl, furyl (furanyl), isothiazolyl, isoxazolyl, oxazolyl, oxadiazolyl, thiazolyl, triazolyl and tetrazolyl or pyridyl, pyrazinyl, pyrimidinyl, pyridazinyl and triazinyl.

In other embodiments, the heteroaryl groups present in the compounds of this invention are 9-membered or 10-membered monocyclic heteroaryl groups. Selected 9-10 membered heteroaryl groups contain one nitrogen, oxygen or sulfur ring heteroatom, and optionally contain 1, 2, 3 or 4 additional nitrogen ring atoms.

In some of the compounds of this invention, heteroaryl groups include 9-membered heteroaryl groups include benzothienyl, benzofuranyl, indolyl, indolinyl, isoindolyl, isoindolinyl, indazolyl, indolizinyl, isobenzofuryl, 2,3-dihydrobenzofuryl, benzoxazolyl, benzthiazolyl, benzimidazolyl, benzoxadiazolyl, benzthiadiazolyl, benzotriazolyl, 1,3-benzoxathiol-2-on-yl (2-oxo-1,3-benzoxathiolyl), purinyl and imidazopyridinyl.

In some of the compounds of this invention, heteroaryl groups include 10-membered heteroaryl groups include chromenyl, chromanyl, quinolyl, isoquinolyl, phthalazinyl, naphthridinyl, quinazolinyl, quinoxalinyl, 4H-quinolizinyl, tetrahydroquinolinyl, cinnolinyl, and pteridinyl.

It is to be understood that the terms heterocycle, heterocyclic, heteroaryl, heterocycloalkyl, are intended to encompass stable heterocyclic groups where a ring nitrogen heteroatom is optionally oxidized (e.g., heterocyclic groups containing an N-oxide, such as pyridine-N-oxide) or where a ring sulfur heteroatom is optionally oxidized (e.g., heterocyclic groups containing sulfones or sulfoxide moieties, such as tetrahydrothienyl-1-oxide (a tetrahydrothienyl sulfoxide) or tetrahydrothienyl-1,1-dioxide (a tetrahydrothienyl sulfone)).

"Oxo" represents a double-bonded oxygen moiety; for example, if attached directly to a carbon atom forms a carbonyl moiety (C = O). The terms "halogen" and "halo" represent chloro, fluoro, bromo or iodo substituents. "Hydroxy" or "hydroxyl" is intended to mean the radical -OH.

As used herein, the terms "compound(s) of the invention" or "compound(s) of this invention" mean a compound of Formula (I), as defined above, in any form, i.e., any salt or non-salt form (e.g., as a free acid or base form, or as a salt, particularly a pharmaceutically acceptable salt thereof) and any physical form thereof (e.g., including non-solid forms (e.g., liquid or semi-solid forms), and solid forms (e.g., amorphous or crystalline forms, specific polymorphic forms, solvate forms, including hydrate forms (e.g., mono-, di- and hemi- hydrates)), and mixtures of various forms.

As used herein, the term "optionally substituted" means unsubstituted groups or rings (e.g., cycloalkyl, heterocycloalkyl, and heteroaryl rings) and groups or rings substituted with one or more specified substituents.

Specific compounds of the invention are:
6-[(1,1-dimethylethyl)sulfonyl]-*N*-(4,5-dimethyl-1*H*-pyrazol-3-yl)-4-quinolinamine,
*N*-(4,5-dimethyl-1*H*-pyrazol-3-yl)-6-(isopropylsulfonyl) quinolin-4-amine,
*N*-(4,5-dimethyl-1*H*-pyrazol-3-yl)-6-((tetrahydro-2H-pyran-4-yl)sulfonyl)quinolin-4-amine,
*N*-(3,4-dimethyl-1*H*-pyrazol-5-yl)-6-(methylsulfonyl)-4-quinolinamine,
*N*-(4,5-dimethyl-1*H*-pyrazol-3-yl)-6-[(trifluoromethyl) sulfonyl]-4-quinolinamine,
6-[(1,1-dimethylethyl)sulfonyl]-*N*-[4-methyl-5-(trifluoromethyl)-1*H*-pyrazol-3-yl]-4-quinolinamine,
6-[(1,1-dimethylethyl)sulfonyl]-*N*-(1,3,4-trimethyl-1*H-*pyrazol-5-yl)-4-quinolinamine,
6-[(1-methylethyl)sulfonyl]-*N*-[4-methyl-5-(trifluoromethyl)-1*H*-pyrazol-3-yl]-4-quino linamine,
*N*-(4-methyl-5-(trifluoromethyl)-1*H-*pyrazol-3-yl)-6-((tetrahydro-2*H-*pyran-4-yl)sulfonyl)quinolin-4-amine,
*N*-(3,4-dimethyl-1*H*-pyrazol-5-yl)-6-(((tetrahydrofuran-2-yl)methyl)sulfonyl) quinolin-4-amine,
*N*-(3,'4-dimethyl-1*H*-pyrazol-5-yl)-6-[(2,2,2-trifluoroethyl)sulfonyl]-4-quinolinamine,
6-(tert-butylsulfonyl)-*N*-(4,5-dimethyl-1H-pyrazol-3-yl)-N-methylquinolin-4-amine,
(*R*)-6-(tert-butylsulfinyl)-*N*-(4,5-dimethyl-1H-pyrazol-3-yl)quinolin-4-amine,
(*S*)-6-(tert-butylsulfinyl)-*N*-(4,5-dimethyl-1H-pyrazol-3-yl)quinolin-4-amine,
6-(*tert*-butylsulfonyl)-*N-*(4,5-dimethyl-1H-pyrazol-3-yl)-3-dueteroquinolin-4-amine,
*N*-(4,5-dimethyl-1H-pyrazol-3-yl)-6-((4-fluorotetrahydro-2*H-*pyran-4-yl)sulfonyl)quinolin-4-amine,
*N*-(4,5-dimethyl-1*H*-pyrazol-3-yl)-6-((4-methyltetrahydro-2H-pyran-4-yl)sulfonyl)quinolin-4-amine,
*N*-(4,5-dimethyl-1*H*-pyrazol-3-yl)-6-(tetrahydro-3-furanylsulfonyl)-4-quinolinamine,
*N*-(4,5-dimethyl-1*H-*pyrazol-3-yl)-6-[(2-methyltetrahydro-3-furanyl)sulfonyl]-4-quinolinamine,
*N*-(3,4-dimethyl-1*H*-pyrazol-5-yl)-6-(tetrahydro-2H-pyran-4-ylthio)-4-quinolinamine,
2-({4-[(4,5-dimethyl-1*H*-pyrazol-3-yl)amino]-6-quinolinyl}sulfonyl) ethanol,
ethyl 3-({6-[(1,1-dimethylethyl)sulfonyl]-4-quinolinyl}amino)-4-methyl-1*H-*pyrazole-5-carboxylate,
3-({6-[(1,1-dimethylethyl)sulfonyl]-4-quinolinyl}amino)-4-methyl-1*H-*pyrazol-5-yl]methanol,
[3-({6-[(1,1-Dimethylethyl)sulfonyl]-4-quinolinyl}amino)-5-methyl-1*H-*pyrazol-4-yl]methanol,
3-({6-[(1,1-Dimethylethyl)sulfonyl]-4-quinolinyl}amino)-5-methyl-1*H*-pyrazole-4-carboxylic acid,
(*R*)-*N*-(3,4-dimethyl-1H-pyrazol-5-yl)-6-((tetrahydro-2H-pyran-4-yl)sulfinyl)quinolin-4-amine, and
(*S*)-*N*-(3,4-dimethyl-1H-pyrazol-5-yl)-6-((tetrahydro-2H-pyran-4-yl)sulfinyl)quinolin-4-amine,
or a salt, particularly a pharmaceutically acceptable salt, thereof, or hydrate thereof.

Representative compounds of this invention include the compounds of Examples 1-27.

Accordingly, a compound of the invention includes a compound of Formula (I), or a salt thereof, particularly a pharmaceutically acceptable salt thereof.

There is disclosed a method of inhibiting RIP2 kinase comprising contacting the kinase with a compound according to Formula (I), or a salt, particularly a pharmaceutically acceptable salt, thereof.

There is disclosed a method of treating a RIP2 kinase-mediated disease or condition in a human comprising administering a therapeutically effective amount of a compound according to Formula (I), or a pharmaceutically acceptable salt thereof, to said human.

The compounds according to Formula (I) may contain one or more asymmetric center (also referred to as a chiral center) and may, therefore, exist as individual enantiomers, diastereomers, or other stereoisomeric forms, or as mixtures thereof. Chiral centers, such as a chiral carbon, or particularly, a chiral -SO- moiety, may also be present in the compounds of this invention. Where the stereochemistry of a chiral center present in a compound of this invention, or in any chemical structure illustrated herein, is not specified the structure is intended to encompass all individual stereoisomers and all mixtures thereof. Thus, compounds according to Formula (I) containing one or more chiral center may be used as racemic mixtures, enantiomerically enriched mixtures, or as enantiomerically pure individual stereoisomers.

Individual stereoisomers of a compound according to Formula (I) which contain one or more asymmetric center may be resolved by methods known to those skilled in the art. For example, such resolution may be carried out (1) by formation of diastereoisomeric salts, complexes or other derivatives; (2) by selective reaction with a stereoisomer-specific reagent, for example by enzymatic oxidation or reduction; or (3) by gas-liquid or liquid chromatography in a chiral environment, for example, on a chiral support such as silica with a bound chiral ligand or in the presence of a chiral solvent. Such a resolution (separation of enantiomers) is described in Examples 26 and 27. The skilled artisan will appreciate that where the desired stereoisomer is converted into another chemical entity by one of the separation procedures described above, a further step is required to liberate the desired form. Alternatively, specific stereoisomers may be synthesized by asymmetric synthesis using optically active reagents, substrates, catalysts or solvents, or by converting one enantiomer to the other by asymmetric transformation.

It is to be understood that a solid form of a compound of the invention may exist in crystalline forms, non-crystalline forms or a mixture thereof. Such crystalline forms may also exhibit polymorphism (i.e. the capacity to occur in different crystalline forms). These different crystalline forms are typically known as "polymorphs." Polymorphs have the same chemical composition but differ in packing, geometrical arrangement, and other descriptive properties of the crystalline solid state. Polymorphs, therefore, may have different physical properties such as shape, density, hardness, deformability, stability, and dissolution properties. Polymorphs typically exhibit different melting points, IR spectra, and X-ray powder diffraction patterns, which may be used for identification. One of ordinary skill in the art will appreciate that different polymorphs may be produced, for example, by changing or adjusting the conditions used in crystallizing/recrystallizing the compound.

Because of their potential use in medicine, the salts of the compounds of Formula (I) are preferably pharmaceutically acceptable salts. Suitable pharmaceutically acceptable salts include those described by Berge, Bighley and Monkhouse J.Pharm.Sci (1977) 66, pp 1-19. Salts encompassed within the term "pharmaceutically acceptable salts" refer to non-toxic salts of the compounds of this invention.

When a compound of the invention is a base (contains a basic moiety), a desired salt form may be prepared by any suitable method known in the art, including treatment of the free base with an inorganic acid, such as hydrochloric acid, hydrobromic acid, sulfuric acid, nitric acid, phosphoric acid, and the like, or with an organic acid, such as acetic acid, trifluoroacetic acid, maleic acid, succinic acid, mandelic acid, fumaric acid, malonic acid, pyruvic acid, oxalic acid, glycolic acid, salicylic acid, and the like, or with a pyranosidyl acid, such as glucuronic acid or galacturonic acid, or with an alpha-hydroxy acid, such as citric acid or tartaric acid, or with an amino acid, such as aspartic acid or glutamic acid, or with an aromatic acid, such as benzoic acid or cinnamic acid, or with a sulfonic acid, such as p-toluenesulfonic acid, methanesulfonic acid, ethanesulfonic acid or the like.

Suitable addition salts are formed from acids which form non-toxic salts and examples include acetate, p-aminobenzoate, ascorbate, aspartate, benzenesulfonate, benzoate, bicarbonate, bismethylenesalicylate, bisulfate, bitartrate, borate, calcium edetate, camsylate, carbonate, clavulanate, citrate, cyclohexylsulfamate, edetate, edisylate, estolate, esylate, ethanedisulfonate, ethanesulfonate, formate, fumarate, gluceptate, gluconate, glutamate, glycollate, glycollylarsanilate, hexylresorcinate, hydrabamine, hydrobromide, hydrochloride, dihydrochloride, hydrofumarate, hydrogen phosphate, hydroiodide, hydromaleate, hydrosuccinate, hydroxynaphthoate, isethionate, itaconate, lactate, lactobionate, laurate, malate, maleate, mandelate, mesylate, methylsulfate, monopotassium maleate, mucate, napsylate, nitrate, N-methylglucamine, oxalate, oxaloacetate, pamoate (embonate), palmate, palmitate, pantothenate, phosphate/diphosphate, pyruvate, polygalacturonate, propionate, saccharate, salicylate, stearate, subacetate, succinate, sulfate, tannate, tartrate, teoclate, tosylate, triethiodide, trifluoroacetate and valerate.

Other exemplary acid addition salts include pyrosulfate, sulfite, bisulfite, decanoate, caprylate, acrylate, isobutyrate, caproate, heptanoate, propiolate, oxalate, malonate, suberate, sebacate, butyne-1,4-dioate, hexyne-1,6-dioate, chlorobenzoate, methylbenzoate, dinitrobenzoate, hydroxybenzoate, methoxybenzoate, phthalate, phenylacetate, phenylpropionate, phenylbutrate, lactate, γ-hydroxybutyrate, mandelate, and sulfonates, such as xylenesulfonate, propanesulfonate, naphthalene-1-sulfonate and naphthalene-2-sulfonate.

If an inventive basic compound is isolated as a salt, the corresponding free base form of that compound may be prepared by any suitable method known to the art, including treatment of the salt with an inorganic or organic base, suitably an inorganic or organic base having a higher pKₐ than the free base form of the compound.

When a compound of the invention is an acid (contains an acidic moiety), a desired salt may be prepared by any suitable method known to the art, including treatment of the free acid with an inorganic or organic base, such as an amine (primary, secondary, or tertiary), an alkali metal or alkaline earth metal hydroxide, or the like. Illustrative examples of suitable salts include organic salts derived from amino acids such as glycine and arginine, ammonia, primary, secondary, and tertiary amines, and cyclic amines, such as N-methyl-D-glucamine, diethylamine, isopropylamine, trimethylamine, ethylene diamine, dicyclohexylamine, ethanolamine, piperidine, morpholine, and piperazine, as well as inorganic salts derived from sodium, calcium, potassium, magnesium, manganese, iron, copper, zinc, aluminum, and lithium.

Certain of the compounds of the invention may form salts with one or more equivalents of an acid (if the compound contains a basic moiety) or a base (if the compound contains an acidic moiety). The present invention includes within its scope all possible stoichiometric and non-stoichiometric salt forms.

Compounds of the invention having both a basic and acidic moiety may be in the form of zwitterions, acid-addition salt of the basic moiety or base salts of the acidic moiety.

This invention also provides for the conversion of one pharmaceutically acceptable salt of a compound of this invention, e.g., a hydrochloride salt, into another pharmaceutically acceptable salt of a compound of this invention, e.g., a sodium salt.

For solvates of the compounds of Formula (I), including solvates of salts of the compounds of Formula (I), that are in crystalline form, the skilled artisan will appreciate that pharmaceutically acceptable solvates may be formed wherein solvent molecules are incorporated into the crystalline lattice during crystallization. Solvates may involve nonaqueous solvents such as ethanol, isopropanol, DMSO, acetic acid, ethanolamine, and ethyl acetate, or they may involve water as the solvent that is incorporated into the crystalline lattice. Solvates wherein water is the solvent that is incorporated into the crystalline lattice are typically referred to as "hydrates." Hydrates include stoichiometric hydrates as well as compositions containing variable amounts of water. The invention includes all such solvates. A specific example of a hydrate of this invention is 6-[(1,1-dimethylethyl)sulfonyl]-*N*-(4,5-dimethyl-1*H*-pyrazol-3-yl)-4-quinolinamine monohydrate. It is to be understood that the term "a salt, particularly a pharmaceutically acceptable salt, thereof, or hydrate thereof" encompasses a salt of a compound of Formula (I), a pharmaceutically acceptable salt of a compound of Formula (I), a hydrate of a compound of Formula (I), a hydrate of a salt of a compound of Formula (I), and a hydrate of a pharmaceutically acceptable salt of a compound of Formula (I).

Because the compounds of Formula (I) are intended for use in pharmaceutical compositions it will readily be understood that they are each preferably provided in substantially pure form, for example at least 60% pure, more suitably at least 75% pure and preferably at least 85%, especially at least 98% pure (% are on a weight for weight basis). Impure preparations of the compounds may be used for preparing the more pure forms used in the pharmaceutical compositions.

### GENERAL SYNTHETIC METHODS

The compounds of Formula (I) may be obtained by using synthetic procedures illustrated in the Schemes below or by drawing on the knowledge of a skilled organic chemist. The synthesis provided in these Schemes are applicable for producing compounds of the invention having a variety of different substituent groups employing appropriate precursors, which are suitably protected if needed, to achieve compatibility with the reactions outlined herein. Subsequent deprotection, where needed, affords compounds of the nature generally disclosed. While the Schemes are shown with compounds only of Formula (I), they are illustrative of processes that may be used to make the compounds of the invention.

Intermediates (compounds used in the preparation of the compounds of the invention) may also be present as salts. Thus, in reference to intermediates, the phrase "compound(s) of formula (number)" means a compound having that structural formula or a pharmaceutically acceptable salt thereof.

4-Chloro-6-iodoquinoline could be made through condensation of the appropriate aniline with Meldrum's acid followed by cyclization and chlorination. 6-Bromo-4-chloroquinoline could be purchased commercially.

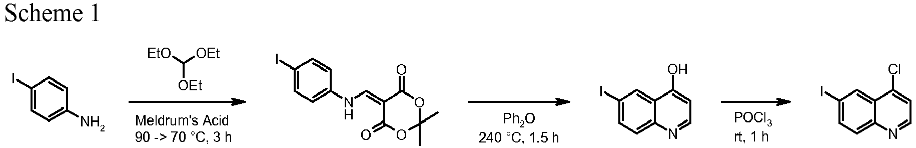

4-Chloro-6-sulfonylquinolines were synthesized from 4-chloro-6-haloquinoline via a palladium catalyzed coupling with a thiol followed by oxidation to the sulfone.

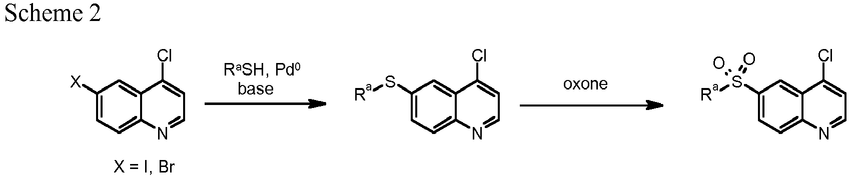

Alternatively, chloroquinolines could be made through condensation of the appropriate aniline with Meldrum's acid followed by cyclization and chlorination.

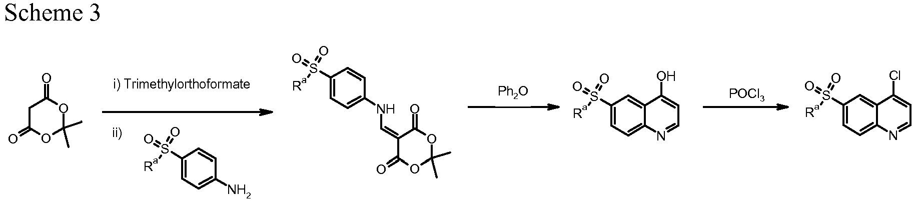

Formation of the pyrazole may be achieved through a two step process. Introduction of a methyl group could be accomplished via methylation of 3-amino-2-butenenitrile, followed by reaction with a source of hydrazine or substituted hydrazines in an appropriate solvent under elevated temperatures.

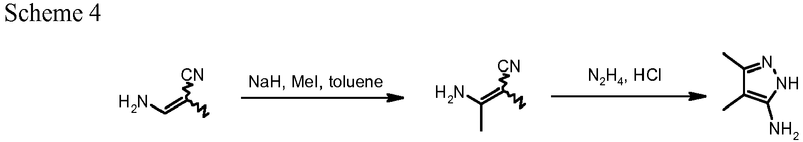

The pyrazole group may be installed on the quinoline core by heating the pyrazole and 4-chloroquinoline in ethanol in the presence of a catalytic amount of acid.

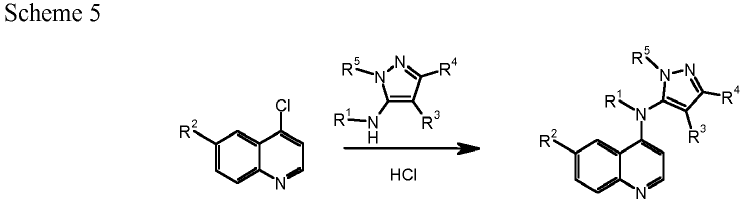

The pyrazole group may be installed prior to the installation of the sulfide/sulfone. Heating the pyrazole and 4-chloro-6-haloquinoline in the presence of acid in an appropriate solvent followed by the palladium catalyzed coupling of the thiol and arylhalide (e.g., aryl iodide) provides the 6-alkylthio-4-pyrazolylquinoline. Oxidation with oxone leads to the corresponding sulfone.

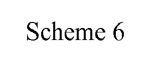

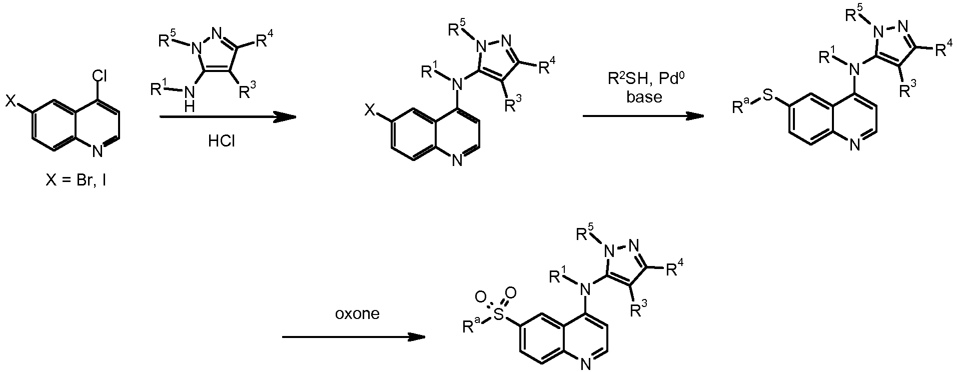

α-Substituted tetrahydro-pyranylsufones may be synthesized by deprotonation and alkylation of the unsubstituted tetrathydropyranylsufone.

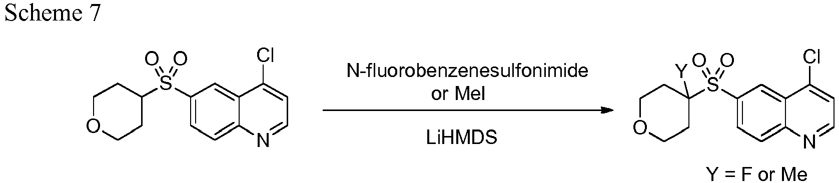

4-Chloro-6-iodoquinoline could be made through condensation of the appropriate aniline with Meldrum's acid followed by cyclization and chlorination. 6-Bromo-4-chloroquinoline could be purchased commercially.

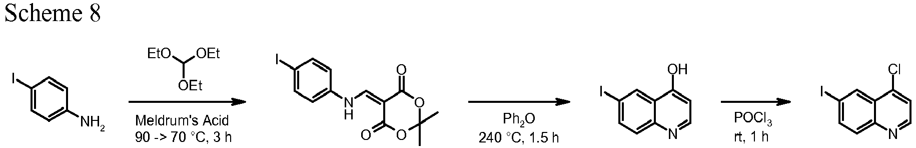

4-Chloro-6-sulfonylquinolines may be synthesized from 4-chloro-6-haloquinoline via a palladium catalyzed coupling with a thiol followed by oxidation to the sulfone. In some cases, sulfoxide may be observed.

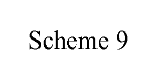

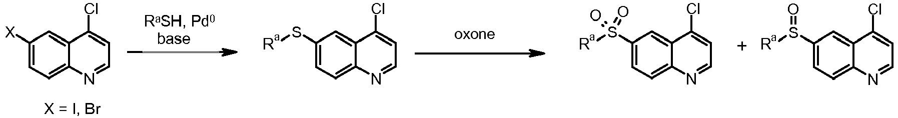

Alternatively, chloroquinolines could be made through condensation of the appropriate aniline with Meldrum's acid followed by cyclization and chlorination.

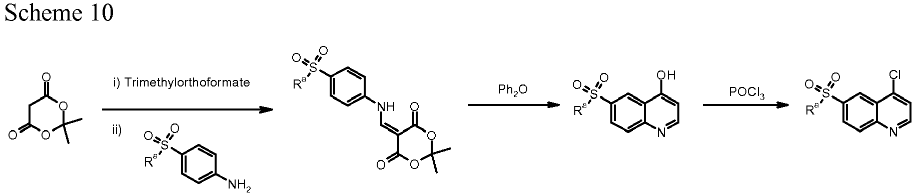

*N*,4,5-trimethyl-1H-pyrazol-3-amine could be obtained acetylation and reduction of the unsubstituted pyrazole.

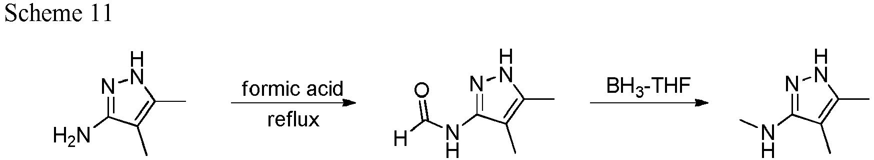

The pyrazole group may be installed prior to the installation of the sulfide/sulfone. Heating the pyrazole and 4-chloro-6-haloquinoline in the presence of acid in an appropriate solvent followed by the palladium catalyzed coupling of the thiol and arylhalide (e.g., aryl iodide) can provide the 6-alkylthio-4-pyrazolylquinoline. Oxidation with oxone leads to the corresponding sulfone.

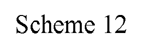

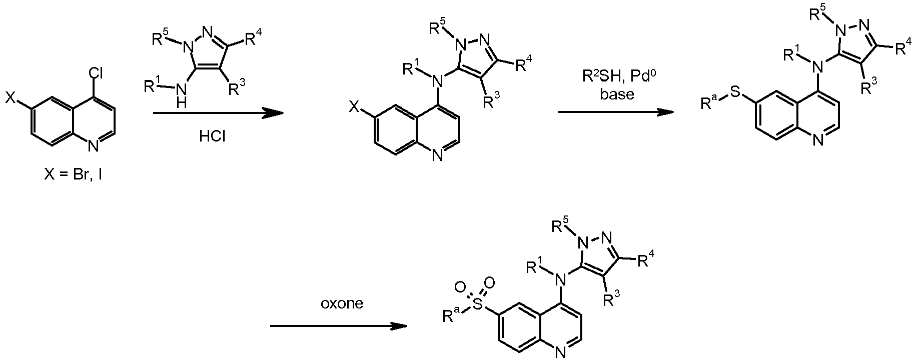

α-Substituted tetrahydro-pyranylsufones may be synthesized by deprotonation and alkylation of the unsubstituted tetrathydropyranylsufone.

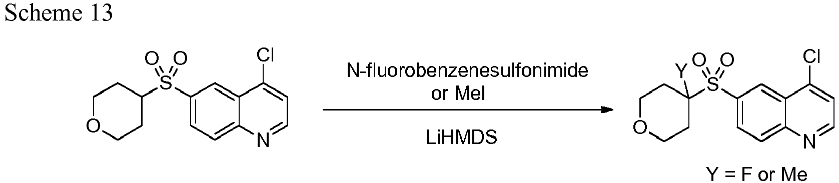

Ethyl 3-amino-4-methyl-1*H*-pyrazole-5-carboxylate could be synthesized via alkylation of an α-diketone with propanenitrile followed by condensation with hydrazine. Reaction with the 4-chloroquinoline provides the final product.

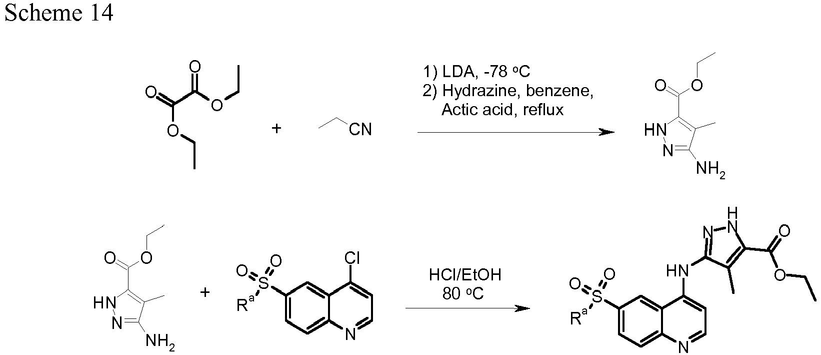

The ethyl carboxylate pyrazole of Scheme 9 could be reduced to the corresponding hydroxyethyl pyrazole by way of a lithium aluminum hydride mediated reduction.

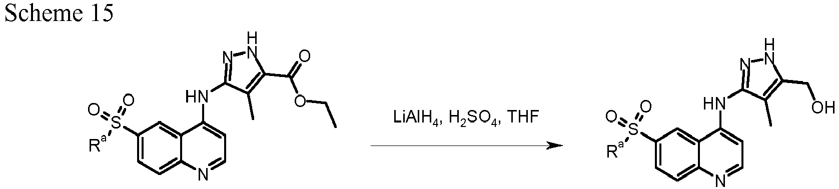

Ethyl 3-amino-5-mcthyl-1*H*-pyrazole-4-carboxylate could be formed similarly by condensation of ethyl (2*E*)-2-cyano-3-(ethyloxy)-2-butenoate with hydrazine. Again, reaction with the 4-chloro-6-sulfonylquinoline followed by a reduction of the ester provides the final product.

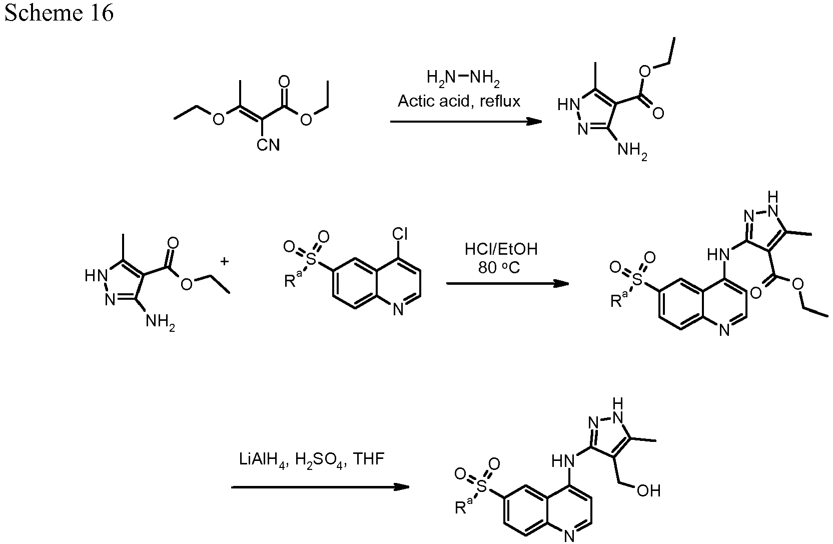

The ethyl carboxylate of Scheme 11 can be hydrolyzed to provide the carboxylic acid.

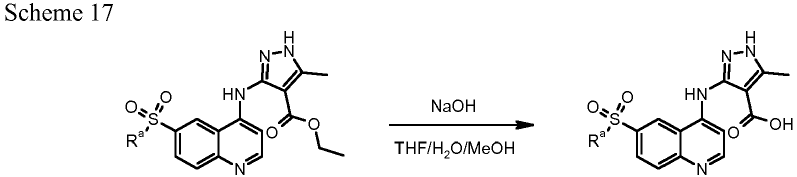

Reference deuterated quinolines can be synthesized via the 4-chloro-6-sulfonylquinoline. Iodination at C3 provides 3-iodoquinalinone which can be converted back to the 4-chloro-3-iodo-quinoline by treatment with POCl₃. A lithium/iodo exchange followed by a quench with deuterated methanol provides the deuterium incorporated quinoline.

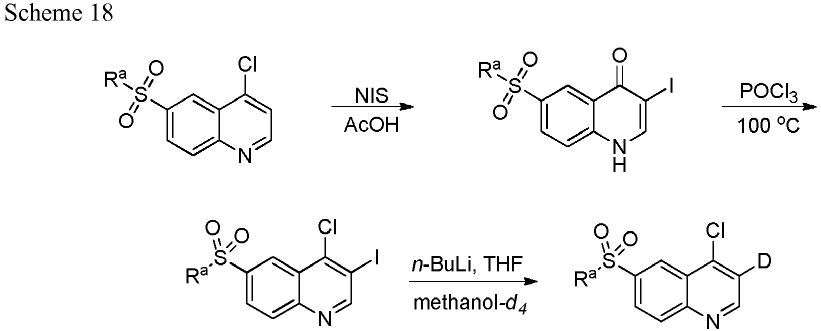

The invention also includes various deuterated forms of the compounds of Formula (I). Each available hydrogen atom attached to a carbon atom may be independently replaced with a deuterium atom. A person of ordinary skill in the art will know how to synthesize deuterated forms of the compounds of Formula (I). For example, deuterated pyrazole alkyl groups or deuterated alkyl-thioquinolines or alkyl-sulfonylquinolines may be prepared by conventional techniques (see for example: according to the method of Scheme 4 using iodomethane-*d*_{*3*,} available from Aldrich Chemical Co., Milwaukee, WI, Cat. No. 176036, or the method of Scheme 2 using methane-*d₃*-thiol, Cat. No. 614904, respectively). Employing such compounds will allow for the preparation of compounds of Formula (I) in which various hydrogen atoms are replaced with deuterium atoms.

Accordingly, there is provided a compound of Formula (I) wherein:
when R¹, R², R³, R⁴ and R⁵ are as defined above,
and at least one of:
   any alkyl group (including any methyl group) or moiety (e.g., (C₁-C₄)alkyl, (C₁-C₆)alkyl, etc., or the alkyl moiety of any alkoxy, phenyl(C₁-C₄)alkyl-, ((C₁-C₄)alkyl)((C₁-C₄)alkyl)amino-, etc.) optionally contains at least one deuterium atom (substituted for a hydrogen atom of the alkyl group or moiety); or
   any of said aryl, heteroaryl, (C₃-C₇)cycloalkyl, 5-6 membered heteroaryl, 9-10 membered heteroaryl, 4-7 membered heterocycloalkyl and phenyl (that is, any phenyl group or moiety (e.g. , the phenyl group of (phenyl)(C₁-C₄ alkyl)amino-) optionally contains at least one deuterium atom (substituted for a hydrogen atom of the aryl, heteroaryl, (C₃-C₇)cycloalkyl, 5-6 membered heteroaryl, 9-10 membered heteroaryl, 4-7 membered heterocycloalkyl and phenyl group or moiety); or
   the central quinolyl group/moiety contains at least one deuterium atom (substituted for a hydrogen atom of the quinolyl); or
   R⁴ is D (deuterium);
   or a salt, particularly a pharmaceutically acceptable salt, thereof.

An example of this particular embodiment of the invention is the compound 6-(*tert*-butylsulfonyl)-*N-*(4,5-dimethyl-1H-pyrazol-3-yl)-3-dueteroquinolin-4-amine, wherein the central quinolyl moiety contains a deuterium atom.

The present disclosure provides a method of inhibiting RIP2 kinase which comprises contacting the kinase with a compound according to Formula (I), or a salt, particularly a pharmaceutically acceptable salt, thereof. This disclosure also provides a method of treatment of a RIP2 kinase-mediated disease or disorder comprising administering a therapeutically effective amount of a compound of Formula (I), or a salt thereof, particularly a pharmaceutically acceptable salt thereof, to a patient, specifically a human, in need thereof. As used herein, "patient" refers to a human or other mammal.

The compounds of this invention may be particularly useful for treatment of RIP2 kinase-mediated diseases or disorders, particularly, uveitis, interleukin-1 converting enzyme (ICE, also known as Caspase-1) associated fever syndrome, dermatitis, acute lung injury, type 2 diabetes mellitus, arthritis (specifically rheumatoid arthritis), inflammatory bowel disorders (such as ulcerative colitis and Crohn's disease), early-onset and extra-intestinal inflammatory bowel disease, prevention of ischemia reperfusion injury in solid organs (specifically kidney) in response ischemia induced by cardiac surgery, organ transplant, sepsis and other insults, liver diseases (non-alcohol steatohepatitis, alcohol steatohepatitis, and autoimmune hepatitis), allergic diseases (such as asthma), transplant reactions (such as graft versus host disease), autoimmune diseases (such as systemic lupus erythematosus, and multiple sclerosis), and granulomateous disorders (such as sarcoidosis, Blau syndrome/early-onset sarcoidosis, Wegner's granulomatosis, and interstitial pulmonary disease).

The compounds of this invention may be particularly useful in the treatment of uveitis, ICE fever, Blau Syndrome/early-onset sarcoidosis, ulcerative colitis, Crohn's disease, Wegener's granulamatosis and sarcoidosis.

Treatment of RIP2 kinase-mediated disease conditions, or more broadly, treatment of immune mediated disease including, but not limited to, allergic diseases, autoimmune diseases, prevention of transplant rejection and the like, may be achieved using a compound of this invention as a monotherapy, or in dual or multiple combination therapy, particularly for the treatment of refractory cases, such as in combination with other anti-inflammatory and/or anti-TNF agents, which may be administered in therapeutically effective amounts as is known in the art.

For example, the compounds of this invention may be administered in combination with corticosteroids and/or anti-TNF agents to treat Blau syndrome/early-onset sarcoidosis; or in combination with anti-TNF biologies or other anti-inflammatory biologies to treat Crohn's Disease; or in combination with 5-ASA (mesalamine) or sulfasalazine to treat ulcerative colitis; or in combination with low-dose corticosteroids and/or methotrexate to treat Wegener's granulamatosis or sarcoidosis or interstitial pulmonary disease; or in combination with a biologic (e.g. anti-TNF, anti-IL-6, etc.) to treat rheumatoid arthritis; or in combination with anti-IL6 and/or methotrexate to treat ICE fever.

Examples of suitable anti-inflammatory agents include corticosteroids, particularly low-dose corticosteroids (such as Deltasone^{®} (prednisone)) and anti-inflammatory biologies (such as Acterma^{®} (anti-IL6R mAb) and Rituximab^{®} (anti-CD20 mAb)). Examples of suitable anti-TNF agents include anti-TNF biologies (such as Enbrel® (etanecerpt)), Humira^{®} (adalimumab), Remicade^{®} (infliximab) and Simponi^{®} (golimumab)).

This invention also provides a compound of Formula (I), or a salt thereof, particularly a pharmaceutically acceptable salt thereof, for use in therapy, specifically for use in the treatment of RIP2 kinase-mediated diseases or disorders, for example the diseases and disorders recited herein.

The invention also provides the use of a compound of Formula (I), or a salt thereof, particularly a pharmaceutically acceptable salt thereof, in the manufacture of a medicament for use in the treatment of RIP2 kinase-mediated diseases or disorders, for example the diseases and disorders recited herein.

A therapeutically "effective amount" is intended to mean that amount of a compound that, when administered to a patient in need of such treatment, is sufficient to effect treatment, as defined herein. Thus, e.g., a therapeutically effective amount of a compound of Formula (I), or a pharmaceutically acceptable salt thereof, is a quantity of an inventive agent that, when administered to a human in need thereof, is sufficient to modulate or inhibit the activity of RIP2 kinase such that a disease condition which is mediated by that activity is reduced, alleviated or prevented. The amount of a given compound that will correspond to such an amount will vary depending upon factors such as the particular compound (e.g., the potency (pIC₅₀), efficacy (EC₅₀), and the biological half-life of the particular compound), disease condition and its severity, the identity (e.g., age, size and weight) of the patient in need of treatment, but can nevertheless be routinely determined by one skilled in the art. Likewise, the duration of treatment and the time period of administration (time period between dosages and the timing of the dosages, e.g., before/with/after meals) of the compound will vary according to the identity of the mammal in need of treatment (e.g., weight), the particular compound and its properties (e.g., pharmaceutical characteristics), disease or condition and its severity and the specific composition and method being used, but can nevertheless be determined by one of skill in the art.

"Treating" or "treatment" is intended to mean at least the mitigation of a disease condition in a patient. The methods of treatment for mitigation of a disease condition include the use of the compounds in this invention in any conventionally acceptable manner, for example for prevention, retardation, prophylaxis, therapy or cure of a mediated disease. Specific diseases and conditions that may be particularly susceptible to treatment using a compound of this invention are described herein.

The compounds of the invention may be administered by any suitable route of administration, including both systemic administration and topical administration. Systemic administration includes oral administration, parenteral administration, transdermal administration, rectal administration, and administration by inhalation. Parenteral administration refers to routes of administration other than enteral, transdermal, or by inhalation, and is typically by injection or infusion. Parenteral administration includes intravenous, intramuscular, and subcutaneous injection or infusion. Inhalation refers to administration into the patient's lungs whether inhaled through the mouth or through the nasal passages. Topical administration includes application to the skin.

The compounds of the invention may be administered once or according to a dosing regimen wherein a number of doses are administered at varying intervals of time for a given period of time. For example, doses may be administered one, two, three, or four times per day. Doses may be administered until the desired therapeutic effect is achieved or indefinitely to maintain the desired therapeutic effect. Suitable dosing regimens for a compound of the invention depend on the pharmacokinetic properties of that compound, such as absorption, distribution, and half-life, which can be determined by the skilled artisan. In addition, suitable dosing regimens, including the duration such regimens are administered, for a compound of the invention depend on the condition being treated, the severity of the condition being treated, the age and physical condition of the patient being treated, the medical history of the patient to be treated, the nature of concurrent therapy, the desired therapeutic effect, and like factors within the knowledge and expertise of the skilled artisan. It will be further understood by such skilled artisans that suitable dosing regimens may require adjustment given an individual patient's response to the dosing regimen or over time as individual patient needs change.

For use in therapy, the compounds of the invention will be normally, but not necessarily, formulated into a pharmaceutical composition prior to administration to a patient. Accordingly, the invention also is directed to pharmaceutical compositions comprising a compound of the invention and a pharmaceutically acceptable excipient.

The pharmaceutical compositions of the invention may be prepared and packaged in bulk form wherein an effective amount of a compound of the invention can be extracted and then given to the patient such as with powders, syrups, and solutions for injection. Alternatively, the pharmaceutical compositions of the invention may be prepared and packaged in unit dosage form. For oral application, for example, one or more tablets or capsules may be administered. A dose of the pharmaceutical composition contains at least a therapeutically effective amount of a compound of this invention (i.e., a compound of Formula (I), or a salt, particularly a pharmaceutically acceptable salt, thereof). When prepared in unit dosage form, the pharmaceutical compositions may contain from 1 mg to 1000 mg of a compound of this invention.

The pharmaceutical compositions of the invention typically contain one compound of the invention. However, in certain embodiments, the pharmaceutical compositions of the invention contain more than one compound of the invention. In addition, the pharmaceutical compositions of the invention may optionally further comprise one or more additional pharmaceutically active compounds.

As used herein, "pharmaceutically acceptable excipient" means a material, composition or vehicle involved in giving form or consistency to the composition. Each excipient must be compatible with the other ingredients of the pharmaceutical composition when commingled such that interactions which would substantially reduce the efficacy of the compound of the invention when administered to a patient and interactions which would result in pharmaceutical compositions that are not pharmaceutically acceptable are avoided. In addition, each excipient must of course be of sufficiently high purity to render it pharmaceutically acceptable.

The compounds of the invention and the pharmaceutically acceptable excipient or excipients will typically be formulated into a dosage form adapted for administration to the patient by the desired route of administration. Conventional dosage forms include those adapted for (1) oral administration such as tablets, capsules, caplets, pills, troches, powders, syrups, elixirs, suspensions, solutions, emulsions, sachets, and cachets; (2) parenteral administration such as sterile solutions, suspensions, and powders for reconstitution; (3) transdermal administration such as transdermal patches; (4) rectal administration such as suppositories; (5) inhalation such as aerosols and solutions; and (6) topical administration such as creams, ointments, lotions, solutions, pastes, sprays, foams, and gels.

Suitable pharmaceutically acceptable excipients will vary depending upon the particular dosage form chosen. In addition, suitable pharmaceutically acceptable excipients may be chosen for a particular function that they may serve in the composition. For example, certain pharmaceutically acceptable excipients may be chosen for their ability to facilitate the production of uniform dosage forms. Certain pharmaceutically acceptable excipients may be chosen for their ability to facilitate the production of stable dosage forms. Certain pharmaceutically acceptable excipients may be chosen for their ability to facilitate the carrying or transporting the compound or compounds of the invention once administered to the patient from one organ, or portion of the body, to another organ, or portion of the body. Certain pharmaceutically acceptable excipients may be chosen for their ability to enhance patient compliance.

Suitable pharmaceutically acceptable excipients include the following types of excipients: diluents, fillers, binders, disintegrants, lubricants, glidants, granulating agents, coating agents, wetting agents, solvents, co-solvents, suspending agents, emulsifiers, sweeteners, flavoring agents, flavor masking agents, coloring agents, anti-caking agents, humectants, chelating agents, plasticizers, viscosity increasing agents, antioxidants, preservatives, stabilizers, surfactants, and buffering agents. The skilled artisan will appreciate that certain pharmaceutically acceptable excipients may serve more than one function and may serve alternative functions depending on how much of the excipient is present in the formulation and what other ingredients are present in the formulation.

Skilled artisans possess the knowledge and skill in the art to enable them to select suitable pharmaceutically acceptable excipients in appropriate amounts for use in the invention. In addition, there are a number of resources that are available to the skilled artisan which describe pharmaceutically acceptable excipients and may be useful in selecting suitable pharmaceutically acceptable excipients. Examples include Remintgon's Pharmaceutical Sciences (Mack Publishing Company), The Handbook of Pharmaceutical Additives (Gower Publishing Limited), and The Handbook of Pharmaceutical Excipients (the American Pharmaceutical Association and the Pharmaceutical Press).

The pharmaceutical compositions of the invention are prepared using techniques and methods known to those skilled in the art. Some of the methods commonly used in the art are described in Remington's Pharmaceutical Sciences (Mack Publishing Company).

In one aspect, the invention is directed to a solid oral dosage form such as a tablet or capsule comprising an effective amount of a compound of the invention and a diluent or filler. Suitable diluents and fillers include lactose, sucrose, dextrose, mannitol, sorbitol, starch (e.g. corn starch, potato starch, and pre-gelatinized starch), cellulose and its derivatives (e.g. microcrystalline cellulose), calcium sulfate, and dibasic calcium phosphate. The oral solid dosage form may further comprise a binder. Suitable binders include starch (e.g. corn starch, potato starch, and pre-gelatinized starch), gelatin, acacia, sodium alginate, alginic acid, tragacanth, guar gum, povidone, and cellulose and its derivatives (e.g. microcrystalline cellulose). The oral solid dosage form may further comprise a disintegrant. Suitable disintegrants include crospovidone, sodium starch glycolate, croscarmelose, alginic acid, and sodium carboxymethyl cellulose. The oral solid dosage form may further comprise a lubricant. Suitable lubricants include stearic acid, magnesium stearate, calcium stearate, and talc.

### EXAMPLES

The following examples illustrate the invention. These examples are not intended to limit the scope of the present invention, but rather to provide guidance to the skilled artisan to prepare and use the compounds, compositions, and methods of the present invention. While particular embodiments of the present invention are described, the skilled artisan will appreciate that various changes and modifications can be made without departing from the scope of the invention defined by claims.

Names for the intermediate and final compounds described herein were generated using the software naming program ACD/Name Pro V6.02 available from Advanced Chemistry Development, Inc., 110 Yonge Street, 14^{th} Floor, Toronto, Ontario, Canada, M5C 1T4 (http://www.acdlabs.com/) or the naming program in ChemDraw, Struct=Name Pro 12.0, as part of ChemBioDraw Ultra, available from CambridgeSoft. 100 CambridgePark Drive, Cambridge, MA 02140 USA (www.cambridgesoft.com). It will be appreciated by those skilled in the art that in certain instances this program will name a structurally depicted compound as a tautomer of that compound. It is to be understood that any reference to a named compound or a structurally depicted compound is intended to encompass all tautomers of such compounds and any mixtures of tautomers thereof.

In the following experimental descriptions, the following abbreviations may be used:

| **Abbreviation** | **Meaning** |
|---|---|
| AcOH | acetic acid |
| aq | aqueous |
| brine | saturated aqueous NaCl |
| CH₂Cl₂, DCM | methylene chloride |
| CH₃CN or MeCN | acetonitrile |
| CH₃NH₂ | methylamine |
| d | day |
| DMF | *N*,*N-*dimethylformamide |
| DMSO | dimethylsulfoxide |
| EDC | 1-ethyl-3-(3-dimethylaminopropyl) carbodiimide |
| equiv | equivalents |
| Et | ethyl |
| Et₃N | triethylamine |
| Et₂O | diethyl ether |
| EtOAc | ethyl acetate |
| h, hr | hour |
| HATU | O-(7-Azabenzotriazol-1yl)-*N*,*N*,*N*',*N*'-tetramethylyronium hexafluorophosphate |
| HCl | hydrochloric acid |
| *i*-Pr₂NEt | *N*',*N*'-diisopropylethylamine |
| KO*t*-Bu | potassium *tert*-butoxide |
| LCMS | liquid chromatography-mass spectroscopy |
| LiHDMS | lithium hexamethyldisilazide |
| Me | methyl |
| MeOH or CH₃OH | methanol |
| MSO₄ | magnesium sulfate |
| min | minute |
| MS | mass spectrum |
| µW | microwave |
| NaBH₄ | sodium borohydride |
| Na₂CO₃ | sodium carbonate |
| NaHCO₃ | sodium bicarbonate |
| NaOH | sodium hydroxide |
| Na₂SO₄ | sodium sulfate |
| N₂H₂ | hydrazine |
| NH₄Cl | ammonium chloride |
| NiCb•6H₂O | nickel (II) chloride hexahydrate |
| NMP | *N*-methyl-2-pyrrolidone |
| Ph | phenyl |
| POCl₃ | phosphoryl chloride |
| rt | room temperature |
| satd. | saturated |
| SCX | strong cation exchange |
| SPE | solid phase extraction |
| TFA | trifluoroacetic acid |
| THF | tetrahydrofuran |
| *t*_{R} | retention time |

### Preparation 1

### 4-chloro-6-iodoquinoline

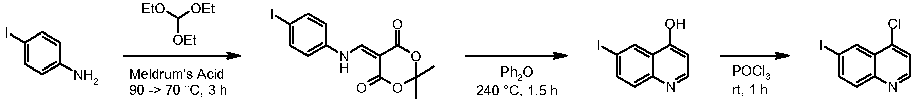

Step 1.5-{[(4-iodophenyl)amino]methylidene}-2,2-dimethyl-1,3-dioxane-4,6-dione: A mixture of Meldrum's acid (227 g, 1.58 mol) and triethyl orthoformate (262 mL, 1.58 mol) was heated to 90 °C for 1.5 hours before being cooled to 70 °C where 4-iodoaniline (300 g, 1.37 mol) was added in portions. In order for the reaction to be continually stirred via mechanical stirrer, MeOH was added (500 mL). Once the addition was complete, the reaction was stirred at 70 °C for another 1 hour before it was diluted with MeOH (1.5 L) and the suspension was filtered. The cake was broken up and washed with MeOH (2 x 1 L) and dried under vacuum overnight to afford the title compound as a tan solid (389 g, 75 %). ¹H NMR (400 MHz, DMSO-*d*₆) δ 11.22 (d, *J* = 14.4 Hz, 1H), 8.55 (d, *J* = 14.7 Hz, 1H), 7.76 (d, *J* = 8.8 Hz, 2H), 7.41 (d, *J* = 8.8 Hz, 2H), 1.67 (s, 6H).

Step 2. 6-iodo-4-quinolinol: To diphenyl ether (1.3 L, 8.0 mol) at 240 °C was added 5-{[(4-iodophenyl)amino]methylidene}-2,2-dimethyl-1,3-dioxane-4,6-dione (120 g, 322 mmol) portion-wise. The reaction was heated for 1.5 hours before being cooled to rt and poured into 1.5 L of hexanes. The resulting suspension was then filtered. The cake was broken up and rinsed with hexanes (2 x 500 mL). The solid was dried under vacuum to afford the title compound as a brown solid (80 g, 82%). ¹H NMR (400 MHz, DMSO-*d*₆) δ 11.89 (d, *J* = 4.0 Hz, 1H), 8.36 (d, *J* = 2.3 Hz, 1H), 7.89 - 7.98 (m, 2H), 7.37 (d, *J* = 8.6 Hz, 1H), 6.07 (dd, *J* = 7.5, 1.1 Hz, 1H); MS (m/z) 272.0 (M+H⁺).

Step 3. 4-chloro-6-iodoquinoline: 6-Iodo-4-quinolinol (100 g, 369 mmol) was suspended in POCl₃ (340 mL, 3.7 mol) at rt. After 1 hour it was concentrated, and the resulting residue was placed in an ice water bath and carefully neutralized using satd. aqueous Na₂CO₃. The resulting brown suspension was filtered, and the solid was rinsed with water (2 x 500 mL) and dried under vacuum overnight. 4-chloro-6-iodoquinoline was obtained as a brown solid (103 g, 92 %). ¹H NMR (400 MHz, DMSO-*d*₆) δ 8.88 (d, *J* = 4.8 Hz, 1H), 8.54 (d, *J* = 1.8 Hz, 1H), 8.15 (dd, *J* = 8.8, 2.0 Hz, 1H), 7.88 (d, *J* = 8.8 Hz, 1H), 7.82 (d, *J* = 4.8 Hz, 1H); MS (m/z) 289.9 (M+H⁺).

### Preparation 2

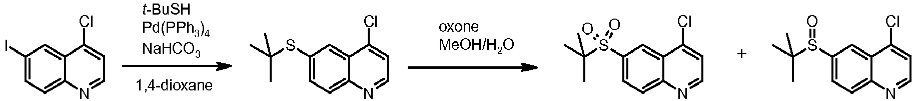

Step 1: 4-chloro-6-[(1,1-dimethylethyl)thio]quinoline: To a flask was added 4-chloro-6-iodo-quinoline (25 g, 86 mmol), tetrakis(triphenylphosphonium)palladium(0) (5.0 g, 4.3 mmol), and sodium carbonate (23 g, 216 mmol). The flask was then evacuated and backfilled with nitrogen three times. 1,4-Dioxane (200 mL) was then added followed by thiol (2-methyl-2-propane thiol, 10.2 mL, 91 mmol). The reaction was then heated to 50 °C overnight. The reaction was not complete and heating was continued at 70 °C for an additional 20 hours. Upon completion, the reaction was cooled to rt and poured into 200 mL of 2M aq 5:1 Na₂S₂O₃:NaHCO₃. The organics were collected and the aqueous layer was backextracted with EtOAc (2 x 200 mL). The combined organics were dried over sodium sulfate, filtered, and concentrated. The crude material was purified by flash chromatography (0 -> 20 % EtOAc in hexanes) and desired fractions were combined and concentrated to an oil which solidified upon standing to provide 9.4 g (43%) of the desired product. MS (m/z) 252.1 (M+H⁺). On some occasions, the sulfoxide intermediate was observed as a minor byproduct (<2%) and carried through to the final step (see example 1). Alternatively, triethylamine (TEA) may be used in place of sodium carbonate, and dioxane or acetonitrile may be used as the solvent in other examples. The sodium thiolate may also be used in place of the thiol when available. See table below for intermediates using these alternate conditions.

*Couplings may also be accomplished with the 6-bromo-4-chloroquinoline which can be purchased from ECA International.

Step 2: 4-chloro-6-[(1,1-dimethylethyl)sulfonyl]quinoline: 4-Chloro-6-[(1,1-dimethylethyl)thio]quinoline (9.4 g, 37 mmol) was suspended in MeOH (100 mL) and water (100 mL) before oxone (25 g, 41 mmol) was added and the reaction was stirred at rt until complete by LCMS (3 hours). The MeOH was removed in vacuo and the heterogeneous aqueous solution was extracted 3x with 100 mL EtOAc. The combined organics were concentrated to provide 8.5 g (80%) of a yellow powder. ¹H NMR (DMSO-*d*₆) δ: 9.08 (d, *J* = 4.8 Hz, 1H), 8.63 (d, *J* = 2.0 Hz, 1H), 8.36 (d, *J* = 8.8 Hz, 1H), 8.20 (dd, *J =* 8.8, 2.0 Hz, 1H), 8.00 (d, *J =* 4.8 Hz, 1H), 1.32 (s, 9H); MS (m/z) 284.1 (M+H⁺). Alternatively, THF or EtOAc may also be used as cosolvents with water in various ratios. See table below for conditions.

The following intermediates were prepared by similar methods:

| Prep # | Structure | Name | MS (M+H)⁺ | Step 1 Base/solvent | Step 2 Cosolvents |
|---|---|---|---|---|---|
| 3 | 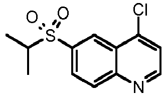 | 4-chloro-6-[(1-methylethyl) sulfonyl] quinoline | 270 | Sodium carbonate/1,4-dioxane; sodium propanethiolate | THF:Water |
| | | | | | 1.25:1 |
| 4 | 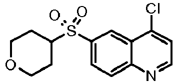 | 4-chloro-6-(tetrahydro-2*H-*pyran-4-ylsulfonyl) quinoline | 312 | TEA/acetonitrile | THF:Water |
| | | | | | 8:1 |
| 5 | 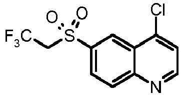 | 4-chloro-6-[(2,2,2-trifluoroethyl) sulfonyl] quinoline | 310 | TEA/1,4-Dioxane | THF:Water |
| | | | | | 7:1 |

### Preparation 6

### 4-Chloro-6-(methylsulfonyl)quinoline

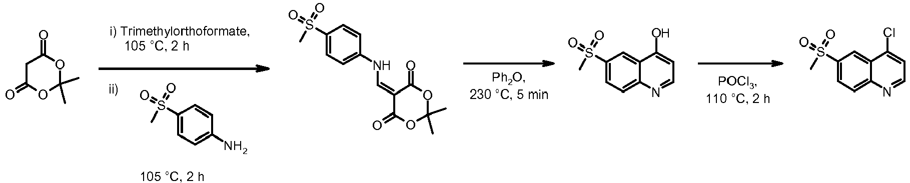

Step 1: 2,2-Dimethyl-5-({[4-(methylsulfonyl)phenyl]amino}methylidene)-1,3-dioxane-4,6-dione: A mixture of 2,2-dimethyl-1,3-dioxane-4,6-dione (51 g, 350 mmol) and trimethyl orthoformate (500 mL) was heated at reflux for 2h at which time 4-(methylsulfonyl)aniline (50 g, 290 mmol) was added. The reaction was stirred at 105 °C for 2h, cooled to rt, and filtered. The filter cake was washed with MeOH and dried to provide pure 2,2-dimethyl-5-({[4-(methylsulfonyl)phenyl]amino}methylidene)-1,3-dioxane-4,6-dione in quantitative yield. ¹H NMR (400 MHz, DMSO-*d*₆) δ 11.36 (d, *J* = 14.4 Hz, 1H), 8.68 (d, *J* = 14.4 Hz, 1H), 7.91 - 8.00 (m, 2H), 7.84 (d, *J* = 8.8 Hz, 2H), 3.25 (s, 3H), 1.69 (s, 6H); MS (m/z) 326 (M+H⁺).

Step 2: 6-(Methylsulfonyl)-4-quinolinol: To a 3-neck round bottom flask containing diphenylether heated to 245 °C (internal temperature) was added 2,2-dimethyl-5-({[4-(methylsulfbnyl)phenyl]amino}methylidene)-1,3-dioxane-4,6-dione (21 g, 13 mmol) over 5 minutes. The internal temperature dropped to 230 °C over the course of the addition. The reaction was allowed to cool to 60 °C and the mixture was diluted with hexanes (300 mL) and filtered to provide the desired product (∼15 g) which contained some residual diphenylether. The reaction was repeated 3 additional times. The 4 batches were combined to provide the desired product (50 g) w/ some diphenylether present. The crude product was suspended in refluxing MeOH (1.5 L), diluted with hexanes (500 mL) and filtered to provide pure 6-(methylsulfonyl)-4-quinolinol (47.3 g, 212 mmol, 80 % combined yield). ¹H NMR (400 MHz, DMSO-*d₆*) δ 12.14 (br. s., 1H), 8.58 (d, *J* = 2.3 Hz, 1H), 8.11 (dd, *J* = 8.8, 2.3 Hz, 1H), 8.03 (d, *J* = 7.6 Hz, 1H), 7.75 (d, *J* = 8.6 Hz, 1H), 6.17 (d, *J=* 7.3 Hz, 1H), 3.25 (s, 3H); MS (m/z) 224 (M+H⁺).

Step 3: 4-Chloro-6-(methylsulfonyl)quinoline: 6-(Methylsulfonyl)-4-quinolinol (23 g, 103 mmol) and phosphorus oxychloride (380 mL, 4.1 mol) were combined and heated at 110 °C for 2 h. The reaction was concentrated to dryness. The residue was treated with satd. sodium carbonate (CAUTION: gas evolution) to quench any residual POCl₃. The suspension was diluted with water and filtered to provide pure 4-chloro-6-(methylsulfonyl)quinoline (23 g, 95 mmol, 92 % yield). ¹H NMR (500 MHz, DMSO-*d*₆) δ 9.05 (d, *J* = 4.9 Hz, 1H), 8.74 (s, 1H), 8.29 - 8.40 (m, 2H), 7.98 (d, *J* = 4.6 Hz, 1H), 3.39 (s, 3H); MS (m/z) 242 (M+H⁺).

The following intermediates can also be made in an analogous manner beginning with the appropriate commercial aniline:

| Prep # | Structure | Name | MS (M+H)⁺ | NMR |
|---|---|---|---|---|
| 7 | 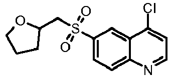 | 4-chloro-6-[(tetrahydro-2-furanylmethyl) sulfonyl] quino line | 312 | ¹H NMR (CHLOROFORM-*d*) δ: 8.97 (d, *J* = 4.5 Hz, 1H), 8.93 (s, 1H), 8.28 - 8.36 (m, *J* = 8.8 Hz, 1H), 8.24 (d, *J* = 8.8, 2.0 Hz, 1H), 7.66 (d, *J* = 4.8 Hz, 1H), 4.29 - 4.45 (m, 1H), 3.63 - 3.82 (m, 2H), 3.46 - 3.60 (m, 1H), 3.30 - 3.42 (m, 1H), 2.10 - 2.26 (m, 1H), 1.83 - 1.97 (m, 2H), 1.70 - 1.75 (m, 1H) |
| 8 | 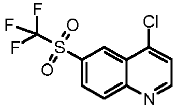 | 4-chloro-6-[(trifluoromethyl) sulfonyl] quino line | 296 | ¹H NMR (CHLOROFORM-*d*) δ: 9.05 (m, 2 H), 8.43 (d, *J* = 8.8 Hz, 1H), 8.28 (dd, *J* = 8.8, 1.5 Hz, 1H), 7.73 (d, *J* = 4.5 Hz, 1H) |

### Preparation 9

### N-(4,5-dimethyl-1H-pyrazol-3-yl)-6-iodoquinolin-4-amine

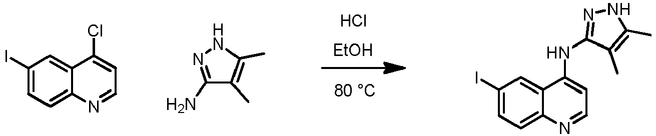

4-Chloro-6-iodoquinoline (5.0 g, 17 mmol) and 3,4-dimethyl-1*H*-pyrazol-5-amine (1.9 g, 17 mmol) were mixed in a microwave vial and taken up in EtOH (35 mL) and a drop of HCl was added. The reaction was heated to 80 °C overnight. After cooling to rt, the mixture was diluted with diethylether. The precipitate was collected by filtration and dried under vacuum to give the title compound (4.5 g, 60%). ¹H NMR (DMSO-*d*₆) δ: 12.67 (s, 1H), 10.47 (br. s., 1H), 9.18 (d, *J* = 1.5 Hz, 1H), 8.53 (d, *J* = 6.8 Hz, 1H), 8.26 (dd, *J* = 8.8, 1.5 Hz, 1H), 7.78 (d, *J* = 8.8 Hz, 1H), 6.80 (d, *J* = 6.8 Hz, 1H), 2.24 (s, 3H), 1.85 (s, 3H); MS (m/z) 365 (M+H⁺).

### Preparation 10

### 3,4-Dimethyl-1H-pyrazol-5-amine

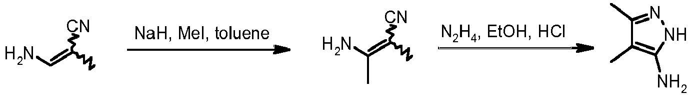

Step1: 3-amino-2-methyl-2-butenenitrile: To a suspension of NaH (11.7 g, 292 mmol) in toluene (100 mL) at 30 °C was added a solution of (2Z)-3-amino-2-butenenitrile (20 g, 244 mmol) in toluene (400 mL) and the reaction mixture was stirred for 10 min. MeI (15.23 mL, 244 mmol) was added and the reaction was cooled with cold water to maintain a temperature of 40 °C. The reaction was then cooled to 30 °C and stirred overnight. An orange solid formed and was collected via filtration washing with toluene. The solid was suspended in water (400 mL) and stirred for 1 hour. The solid was then filtered washing with water and air dried for 15 min, then placed under vacuum overnight (6.7 g, 29%). The mother liquor was concentrated under vacuum and the resulting residue dissolved in EtOAc to give a biphasic solution with mineral oil. The layers were separated and the EtOAc was removed under vacuum; the resulting solid was recrystallized from benzene to give the title compound (2.8 g, 12%). ¹H NMR (400 MHz, DMSO-*d*₆) δ ppm 1.57 (s, 3 H) 1.92 (s, 3 H) 6.12 (br. s., 2 H); MS (m/z) 97 (M+H⁺).

Step 2: 3,4-dimethyl-1*H-*pyrazol-5-amine: To a solution of 3-amino-2-methyl-2-butenenitrile (1.0 g, 10.4 mmol) in ethanol (10.4 mL) was added hydrazine (0.60 mL, 10.4 mmol). The resulting mixture was heated to 75 °C for 16 hours open to atmosphere. The reaction was concentrated onto silica gel and purified via flash chromatography eluting with 0-10% MeOH in DCM over 37 min to give the title compound as a yellow oil (710 mg, 61%). ¹H NMR (400 MHz, DMSO-*d*₆) δ ppm 1.72 (s, 3 H) 1.99 (s, 3 H) 3.99 - 4.50 (m, 2 H) 10.72 - 11.07 (m, 1 H); MS (m/z) 112 (M+H⁺).

### Preparation 11 N,4,5-trimethyl-1H-pyrazol-3-amine

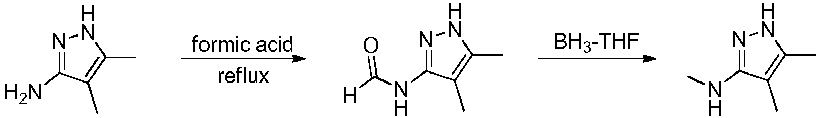

Step 1: (4,5-dimethyl-1H-pyrazol-3-yl)formamide. A mixture of 4,5-dimethyl-1H-pyrazol-3-amine (1.92 g, 17.3 mmol) in formic acid (10 mL) was stirred under nitrogen at reflux for 2 h. The reaction mixture was cooled to rt and concentrated to yield the title compound as a solid. LCMS (m/z): 140 (M+H⁺).

Step 2: *N*,4,5-trimethyl-1H-pyrazol-3-amine. A mixture of (4,5-dimethyl-1H-pyrazol-3-yl)formamide (2.47 g, 17.7 mmol) and BH₃THF (53.1 ml of a 1.0 M solution in THF, 53.1 mmol, 3.0 eq.) was stirred under nitrogen at rt for 3 hours. The mixture was then cooled to 0°C and quenched with MeOH (dropwise addition). The crude product was purified via column chromatography using 0-7% MeOH:DCM gradient, 80 g Isco column to yield 0.70 g of the title compound as a colorless viscous oil. ¹H NMR (400 MHz, DMSO-d₆) δ 10.27 - 11.36 (m, 1H), 4.56 (br. s., 1H), 2.64 (s, 3H), 2.00 (s, 3H), 1.71 (s, 3H); LCMS (m/z): 126 (M+H⁺).

### Preparation 12

### 6-(tert-butylsulfonyl)-4-chloro-3-deuteroquinoline

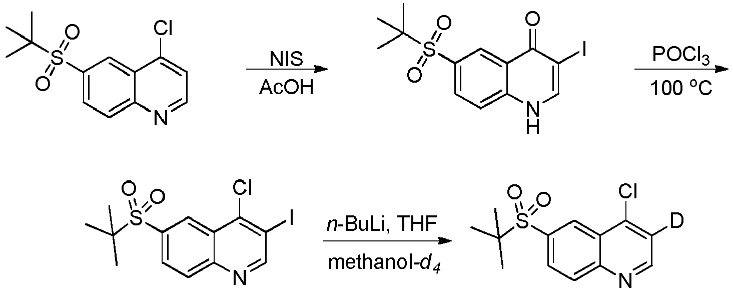

Step 1: 6-(tert-butylsulfonyl)-3-iodoquinolin-4(1H)-one. A mixture of 6-(tert-butylsulfonyl)-4-chloroquinoline (3.00 g, 10.57 mmol) and *N*-Iodosuccinimide (NIS) (2.62 g, 11.63 mmol) in acetic acid (25 mL) was stirred at 70 °C for 2 h. Remaining starting material was observed and the temperature was elevated to 100 °C and stirred for several hours adding NIS several times (an additional 1.1 eq was added). The mixture was cooled to rt and diluted with ether. The resulting solid was filtered and dried to yield the title compound as a tan solid. LCMS (m/z): 392 (M+H⁺).

Step 2: 6-(tert-butylsulfonyl)-4-chloro-3-iodoquinoline. A mixture of 6-(tert-butylsulfonyl)-3-iodoquinolin-4(1H)-one (4.14 g, 10.57 mmol) in POCl₃ (25 mL) was stirred under nitrogen at 100 °C for 2 h. A solid precipitated during the course of the reaction. The reaction was cooled to rt and diluted with DCM, and the solid was filtered. The mother liquor was concentrated *in vacuo,* and the resulting material was purified via column chromatography using 0-5% MeOH:DCM gradient to yield the title compound as a light tan solid (3.11 g, 72% over two steps). ¹H NMR (400 MHz, DMSO-d₆) δ 9.38 (s, 1H), 8.63 (d, J = 1.77 Hz, 1H), 8.32 (d, J = 8.59 Hz, 1H), 8.19 (dd, J = 2.02, 8.84 Hz, 1H), 1.30 (s, 9H); LCMS (m/z): 410 (M+H⁺).

Step 3: 6-(tert-butylsulfonyl)-4-chloro-3-deuteroquinoline. A solution of 6-(tert-butylsulfonyl)-4-chloro-3-iodoquinoline (750 mg, 1.831 mmol) in THF (22 mL) was cooled to -78°C. n-Butyllithium (1.831 ml, 2.56 mmol) was slowly added and the reaction mixture was stirred for 20min, followed by addition of methanol-d₄ (315 µL). The reaction was allowed to warm to room temperature for 10 minutes and the residue was evaporated to near dryness. The residue was purified via Isco CombiFlash (30% to 100% in EtOAc in Hexanes; 40g silica gel cartridge column). The fractions were evaporated *in vacuo* to give 3-deutero-6-(tert-butylsulfonyl)-4-chloroquinoline as a yellow solid. MS (m/z) 285.0 (M+H⁺); ¹H NMR (DMSO-d₆) δ: 9.08 (s, 1H), 8.59 - 8.69 (m, 1H), 8.36 (d, J = 8.8 Hz, 1H), 8.18 (dd, J = 8.8, 2.0 Hz, 1H), 1.32 (s, 9H).

### Example 1

### 6-[(1,1-dimethylethyl)sulfonyl]-N-(4,5-dimethyl-1H-pyrazol-3-yl)-4-quinolinamine

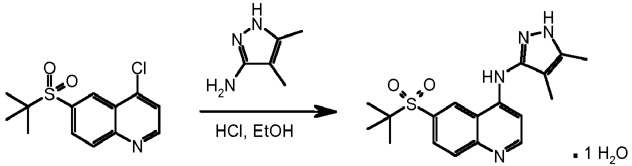

4-Chloro-6-[(1,1-dimethylethyl)sulfonyl]quinoline (500 mg, 1.8 mmol) and 4,5-dimethyl-1*H*-pyrazol-3-amine (196 mg, 1.8 mmol; alternate name: 3,4-dimethyl-1*H-*pyrazol-5-amine) were taken up in ethanol (5.87 mL)with a drop of HCl and heated to 80°C overnight. After cooling to rt, the solution was diluted with ether (12 mL) and filtered. The solid precipitate was taken up in MeOH (heterogeneous mixture) and stirred with 5 equivalents of MP-carbonate resin (3.21 mmol/g, 550 mg) for 20 minutes. After filtering off the beads, the filtrate was concentrated and taken up in 4 mL acetonitrile and 2 mL water and sonicated for 1 minute. The solid precipitate was collected by filtration to provide 475 mg (75%) of the desired product, isolated as a crystalline monohydrate. ¹H NMR (400 MHz, DMSO-*d*₆) δ ppm: 12.31 (s, 1H), 9.40 (s, 1H), 9.06 (s, 1H), 8.53 (d, *J =* 5.3 Hz, 1H), 7.91 - 8.06 (m, 2H), 6.60 (d, *J* = 5.3 Hz, 1H), 2.22 (s, 3H), 1.81 (s, 3H), 1.33 (s, 9H); MS (m/z) 359.2 (M+H⁺).

The following compounds were made in the same manner with some variations in time, solvent (EtOH or iPrOH), and temperature. Please note that some compounds were free-based by basic SPE cartridges. In cases where sulfoxide was isolated, the sulfoxide enantiomers were separated by chiral SFC methods.

| Ex # | Structure | Name | MS (M+H)⁺ | NMR |
|---|---|---|---|---|
| 2 | 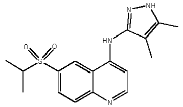 | *N*-(4,5-dimethyl-1*H-*pyrazol-3-yl)-6-(isopropylsulfonyl) quinolin-4-amine | 345 | ¹H NMR (DMSO-*d*₆) δ: 12.30 (s, 1H), 9.32 (s, 1H), 9.08 (d, *J* = 1.5 Hz, 1H), 8.52 (d, *J* = 5.3 Hz, 1H), 7.95 - 8.06 (m, 2H), 6.63 (d, *J =* 5.1 Hz, 1H), 3.53 (spt, *J* = 6.8 Hz, 1H), 2.21 (s, 3H), 1.81 (s, 3H), 1.23 (d, 6H) |
| 3 | 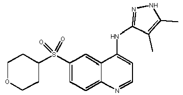 | *N*-(4,5-dimethyl-1*H-*pyrazol-3-yl)-6-((tetrahydro-2H-pyran-4-yl)sulfonyl)quinolin-4-amine | 387 | ¹H NMR (DMF-*d*₇) δ: 12.30 (s, 1H), 9.34 (s, 1H), 9.07 (d, *J* = 1.3 Hz, 1H), 8.52 (d, *J* = 5.6 Hz, 1H), 7.94 - 8.07 (m, 2H), 6.62 (d, *J* = 5.3 Hz, 1H), 3.92 (dd, *J* = 11.2, 3.7 Hz, 2H), 3.61 (tt, *J* = 11.9, 3.7 Hz, 1H), 3.25 - 3.34 (m, 2H), 2.21 (s, 3H), 1.74 - 1.83 (m, 5H), 1.57 - 1.69 (m, 2H) |
| 4 | 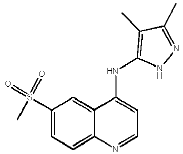 | *N*-(3,4-dimethyl-1*H-*pyrazol-5-yl)-6-(methylsulfonyl)-4-quinolinamine hydrochloride | 317 | ¹H NMR (400 MHz, DMSO-*d*₆) δ:14.64 (br. s., 1 H), 12.71 (br. s., 1 H), 11.14 (br. s., 1 H), 9.44 (s, 1 H), 8.63 (d, *J* = 6.8 Hz, 1 H), 8.44 (dd, *J =* 8.8, 1.8 Hz, 1 H), 8.21 (d, *J* = 9.1 Hz, 1 H), 6.87 (d, *J* = 7.1 Hz, 1 H), 3.40 (s, 3 H), 2.25 (s, 3 H), 1.87 (s, 3 H) |
| 5 | 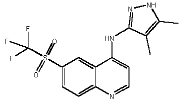 | *N*-(4,5-dimethyl-1*H-*pyrazol-3-yl)-6-[(trifluoromethyl) sulfonyl]-4-quinolinamine | 371 | ¹H NMR (METHANOL-*d*₄) δ: 9.32 (d, *J* = 1.7 Hz, 1H), 8.56 (d, *J* = 5.7 Hz, 1H), 8.22 (dd, *J* = 9.1, 1.7 Hz, 1H), 8.15 (d, *J* = 9.1 Hz, 1H), 6.69 (d, *J* = 5.7 Hz, 1H), 2.31 (s, 3H), 1.92 (s, 3H) |
| 6 | 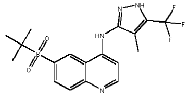 | 6-[(1,1-dimethylethyl)sulfonyl]-*N*-[4-methyl-5-(trifluoromethyl)-1*H-*pyrazol-3-yl]-4-quinolinamine | 413 | ¹H NMR (400 MHz, DMSO-*d*₆) δ: 13.74 (br. s., 1H), 9.53 - 9.84 (m, 1H), 8.92 - 9.22 (m, 1H), 8.47 - 8.77 (m, 1H), 7.86 - 8.27 (m, 2H), 6.18 - 6.58 (m, 1H), 1.96 (s, 3H), 1.32 (s, 9H) |
| 7 | 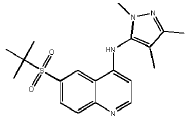 | 6-[(1,1-dimethylethyl)sulfonyl]-*N*-(1,3,4-trimethyl-1*H-*pyrazol-5-yl)-4-quinolinamine | 373 | ¹H NMR (400 MHz, DMSO-*d*₆) δ: 9.45 (s, 1H), 9.04 (s, 1H), 8.58 (d, *J* = 5.05 Hz, 1H), 7.93 - 8.15 (m, 2H), 6.19 (d, *J* = 4.80 Hz, 1H), 3.54 (s, 3H), 2.14 (s, 3H), 1.77 (s, 3H), 1.32 (s, 9H) |
| 8 | 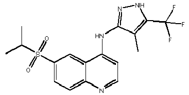 | 6-[(1-methylethyl)sulfonyl]-*N-*[4-methyl-5-(trifluoromethyl)-1*H-*pyrazol-3-yl]-4-quinolinamine | 399 | ¹H NMR (400 MHz, DMSO-*d*₆) δ: 13.59 - 14.00 (m, 1H), 9.03 - 9.26 (m, 1H), 8.48 - 8.70 (m, 1H), 8.02 - 8.30 (m, 2H), 6.49 (br. s., 1H), 3.50 - 3.63 (m, 2H), 1.99 (s, 3H), 1.24 (d, *J* = 7.03 Hz, 6H) |
| 9 | 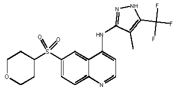 | *N*-(4-methyl-5-(trifluoromethyl)-1*H-*pyrazol-3-yl)-6-((tetrahydro-2*H*-pyran-4-yl)sulfonyl)quinolin-4-amine | 441 | ¹H NMR (400 MHz, DMSO-*d*₆) δ: 13.72 (br. s., 1H), 9.99 (d, 1H), 9.04 (br. s., 1H), 8.38 - 8.71 (m, 1H), 7.98 - 8.18 (m, 2H), 6.25 - 6.43 (m, 1H), 3.93 (dd, *J* = 3.66, 11.24 Hz, 2H), 3.51 - 3.72 (m, 1H), 3.26 - 3.31 (m, 2H), 1.90 - 2.02 (m, 3H), 1.74 - 1.85 (m, 2H), 1.62 (qd, *J* = 4.67, 12.25 Hz, 2H) |
| 10 | 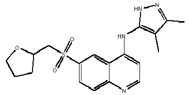 | *N*-(3,4-dimethyl-1*H-*pyrazol-5-yl)-6-(((tetrahydrofuran-2-yl)methyl)sulfonyl) quinolin-4-amine | 387 | ¹H NMR (CHLOROFORM-*d*) δ: 9.14 - 9.45 (m, 1H), 8.34 - 8.57 (m, 2H), 8.20 (d, *J* = 8.3 Hz, 1H), 6.64 (d, *J* = 4.0 Hz, 1H), 4.31 - 4.47 (m, 1H), 3.60 - 3.84 (m, 2H), 2.13 - 2.27 (m, 3H), 2.07 (s, 3H), 1.84 - 1.98 (m, 2H), 1.79 (s, 3H), 1.63 - 1.74 (m, 1H) |
| 11 | 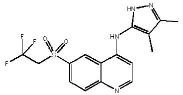 | *N*-(3,4-dimethyl-1*H-*pyrazol-5-yl)-6-[(2,2,2-trifluoroethyl)sulfonyl]-4-quinolinamine | 385 | ¹H NMR (METHANOL-*d*₄) δ: 9.21 (d, *J*= 1.8 Hz, 1H), 8.51 (d, *J*= 5.8 Hz, 1H), 8.28 (dd, *J*= 9.0, 1.8 Hz, 1H), 8.10 (d, *J* = 9.0 Hz, 1H), 6.75 (d, *J*= 5.8 Hz, 1H), 4.63 (q, *J* = 9.6 Hz, 2H), 2.32 (s, 3H), 1.93 (s, 3H) |
| 12 | 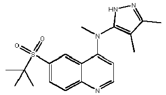 | 6-(tert-butylsulfonyl)-*N-*(4,5-dimethyl-1H-pyrazol-3-yl)-N-methylquinolin-4-amine | 373 | ¹H NMR (400 MHz, DMSO-d₆) δ 12.14 (s, 1H), 8.80 (d, J = 5.31 Hz, 1H), 7.94 - 8.07 (m, 2H), 7.83 (dd, J = 2.02, 8.84 Hz, 1H), 7.15 (d, J = 5.56 Hz, 1H), 3.33 (s, 3H), 2.08 (s, 3H), 1.65 (s, 3H), 1.09 (s, 9H) |
| 13 | 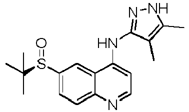 | (*R*)-6-(*tert-*butylsulfinyl)-*N*-(4,5-dimethyl-1H-pyrazol-3-yl)quinolin-4-amine | 343 | ¹H NMR (400 MHz, DMSO-d6) δ ppm 12.28 (s, 1 H), 9.06 (s, 1 H), 8.68 (d, J=1.5 Hz, 1 H), 8.46 (d, J=5.3 Hz, 1 H), 7.99 (d, J=8.8 Hz, 1 H), 7.84 (dd, J=8.8, 1.8 Hz, 1 H), 6.58 (d, J=5.3 Hz, 1 H), 2.21 (s, 3 H), 1.79 (s, 3 H), 1.15 (s, 9 H) |
| 14 | 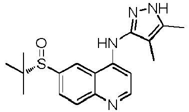 | (*S*)-6-(tert-butylsulfinyl)-*N*-(4,5-dimethyl-1H-pyrazol-3-yl)quinolin-4-amine | 343 | ¹H NMR (400 MHz, DMSO-d6) δ ppm 12.28 (s, 1 H), 9.06 (s, 1 H), 8.68 (d, J=1.5 Hz, 1H), 8.46 (d, J=5.3 Hz, 1 H), 7.99 (d, J=8.8 Hz, 1 H), 7.84 (dd, J=8.8, 1.8 Hz, 1 H), 6.58 (d, J=5.3 Hz, 1 H), 2.21 (s, 3 H), 1.79 (s, 3 H), 1.15 (s, 9 H) |
| 15 | 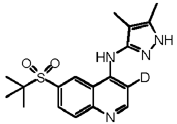 | 6-(*tert*-butylsulfonyl)-*N-*(4,5-dimethyl-1H-pyrazol-3-yl)-3-dueteroquinolin-4-amine | 360 | ¹H NMR (DMSO-d₆) δ: 12.21 - 12.35 (m, 1H), 9.34 - 9.47 (m, 1H), 8.98 - 9.11 (m, 1H), 8.52 (s, 1H), 8.01 (d, J = 8.7 Hz, 1H), 7.94 (d, J = 8.7 Hz, 1H), 2.21 (s, 3H), 1.80 (s, 3H), 1.32 (s, 9H) |

### Example 16

### N-(4,5-dimethyl-1H-pyrazol-3-yl)-6-((4-fluorotetrahydro-2H-pyran-4-yl)sulfonyl)quinolin-4-amine

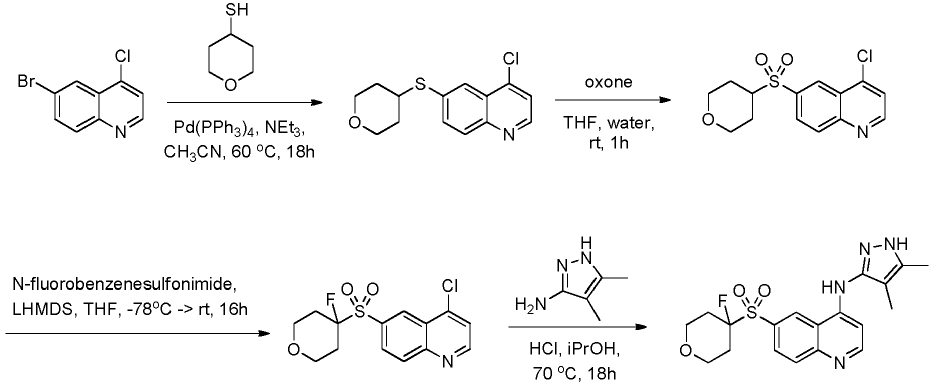

Step 1: 4-chloro-6-((tetrahydro-2*H*-pyran-4-yl)thio)quinoline: 6-Bromo-4-chloroquinoline ( 5 g, 20.6 mmol), acetonitrile (54 mL) and tetrakis(triphenylphosphonium) palladium(0) (1.2 g, 1.0 mmol) were added to a RB flask and purged with nitrogen for 10 min. Tetrahydro-2H-pyran-4-thiol (2.9 g, 24.9 mmol) and triethylamine (4.3 mL, 31 mmol) were added and the reaction was heated under nitrogen at 60 °C for 16 h. The reaction was partitioned between EtOAc and satd. aqueous NaHCO₃. The aqueous layer was extracted with EtOAc (1x) and the combined organics were dry-loaded onto silica gel and purified via column chromatography (ISCO-Rf, 120g, 0-80% EtOAc/hexane) to yield 4-chloro-6-((tetrahydro-2H-pyran-4-yl)thio)quinoline (1.75 g, 6.25 mmol, 30.3 % yield). ¹H NMR (400 MHz, DMSO-*d*₆) δ ppm 8.80 (d, *J* = 4.8 Hz, 1 H), 8.06 (d, *J* = 9.6 Hz, 2 H), 7.88 (d, *J* = 8.8 Hz, 1 H), 7.70 - 7.82 (m, 1 H), 3.86 (dt, *J* = 11.6, 3.5 Hz, 2 H), 3.71 - 3.82 (m, 1 H), 3.41 - 3.50 (m, 2 H), 1.94 (d, *J =* 11.9 Hz, 2 H), 1.50 - 1.65 (m, 2 H). MS (m/z) 280(M+H⁺).

Step 2: 4-chloro-6-((tetrahydro-2*H*-pyran-4-yl)sulfonyl)quinoline: A mixture of 4-chloro-6-((tetrahydro-2*H*-pyran-4-yl)thio)quinoline (6.25 g, 22.3 mmol) and oxone (17.9 g, 29.0 mmol) in THF (105 mL) and water (26.3 mL) was stirred at rt for 1h. The reaction was partitioned between EtOAc and satd. NaHCO₃ (aq), extracted with EtOAc (1x), and the combined organic extracts were washed with brine (1x), dried over magnesium sulfate, filtered and concentrated to dryness to afford 4-chloro-6-((tetrahydro-2H-pyran-4-yl)sulfonyl)quinoline (6.32 g, 18.24 mmol, 82% yield). ¹H NMR (400 MHz, DMSO-*d*₆) δ ppm 9.07 (d, *J* = 4.8 Hz, 1 H), 8.67 (d, *J* = 2.0 Hz, 1 H), 8.38 (d, *J* = 8.8 Hz, 1 H), 8.23 (dd, *J* = 8.8, 2.0 Hz, 1 H), 8.00 (d, *J* = 4.8 Hz, 1 H), 3.91 (dd, *J* = 11.2, 3.7 Hz, 2 H), 3.78 (tt, *J* = 12.0, 3.8 Hz, 1 H), 3.17 - 3.39 (m, 2 H), 1.77 (dd, *J* = 12.4, 1.8 Hz, 2 H), 1.55 - 1.70 (m, 2 H). MS (m/z) 312 (M+H⁺).

Step 3: 4-chloro-6-((4-fluorotetrahydro-2*H*-pyran-4-yl)sulfonyl)quinoline: To an oven dried round bottom flask was added 4-chloro-6-((tetrahydro-2*H*-pyran-4-yl)sulfonyl)quinoline (500 mg, 1.6 mmol) and THF (18 mL). The solution was cooled to - 78 °C and LiHMDS (4.81 mL, 4.81 mmol) was added. After 15 min, N-fluorobenzenesulfonimide (2.0 g, 6.4 mmol) in THF (10 mL) was added and the reaction was allowed to warm to rt over 16h. The reaction was partitioned between satd. aq. NaHCO₃ and DCM, extracted with DCM (1x), dry-loaded onto silica gel and purified via column chromatography (Biotage SP-1, 0-100% EtOAc/hexane, 25g column) to afford 4-chloro-6-((4-fluorotetrahydro-2H-pyran-4-yl)sulfonyl)quinoline (136 mg, 0.412 mmol, 25.7 % yield). ¹H NMR (400 MHz, DMSO-*d*₆) δ ppm 9.11 (d, *J* = 4.5 Hz, 1 H), 8.71 (d, *J* = 2.0 Hz, 1 H), 8.42 (d, *J* = 8.8 Hz, 1 H), 8.24 (dd, *J* = 8.8, 1.3 Hz, 1 H), 8.03 (d, *J* = 4.5 Hz, 1 H), 3.94 (dd, *J* = 11.6, 5.6 Hz, 2 H), 3.44 (td, *J* = 11.9, 1.8 Hz, 2 H), 2.08 - 2.43 (m, 2 H), 1.83 (t, 2 H). MS (m/z) 330 (M+H⁺).

Step 4: *N*-(4,5-dimethyl-1*H*-pyrazol-3-yl)-6-((4-fluorotetrahydro-2*H*-pyran-4-yl)sulfonyl)quinolin-4-amine: To a solution of 4-chloro-6-((4-fluorotetrahydro-2*H*-pyran-4-yl)sulfonyl)quinoline (65 mg, 0.197 mmol) and 4,5-dimethyl-1H-pyrazol-3-amine (54.8 mg, 0.493 mmol) in isopropanol (986 µl) was added 1 drop of conc. HCl. The reaction was stirred at 70 °C for 18 h. The reaction was concentrated to dryness and partitioned between DCM and satd. aq. NaHCO₃. The aqueous layer was extracted with DCM (1x) and the combined organic extracts were dry-loaded onto silica gel and purified via chromatography (Biotage SP-1, 10g, 0-20% isopropanol containing 10% NH₄OH/EtOAc). The desired fractions were concentrated to dryness to afford *N*-(4,5-dimethyl-1*H*-pyrazol-3-yl)-6-((4-fluorotetrahydro-2*H*-pyran-4-yl)sulfonyl)quinolin-4-amine (39 mg, 0.091 mmol, 46.0 % yield). ¹H NMR (400 MHz, DMSO-*d*₆) δ ppm 12.31 (s, 1 H), 9.47 (s, 1 H), 9.15 (d, *J* = 1.8 Hz, 1 H), 8.54 (d, *J* = 5.3 Hz, 1 H), 7.94 - 8.11 (m, 2 H), 6.63 (d, 1 H), 3.95 (dd, *J* = 11.5, 5.2 Hz, 2 H), 3.38 - 3.52 (m, 2 H), 2.16 - 2.39 (m, 5 H), 1.76 - 1.88 (m, 5 H). MS (m/z) 405 (M+H⁺).

The following compound was made in the same manner using methyl iodide as the alkylating agent in step 3:

| Ex # | Structure | Name | MS (M+H)⁺ | NMR |
|---|---|---|---|---|
| 17 | 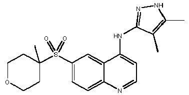 | *N*-(4,5-dimethyl-1*H-*pyrazol-3-yl)-6-((4-methyltetrahydro-2H-pyran-4-yl)sulfonyl)quinolin-4-amine | 401 | ¹H NMR (400 MHz, DMSO-*d*₆) δ ppm 12.31 (s, 1 H), 9.42 (s, 1 H), 9.04 (d, *J* = 1.8 Hz, 1 H), 8.52 (d, *J* = 5.3 Hz, 1H), 7.78 - 8.16 (m, 2 H), 6.59 (d, *J* = 5.6 Hz, 1 H), 3.74 - 3.88 (m, 2 H), 3.37-3.51 (m, 2 H), 2.21 (s, 3 H), 2.06 - 2.18 (m, 2 H), 1.80 (s, 3 H), 1.33 - 1.50 (m, 5 H) |

### Example 18

### N-(4,5-dimethyl-1H-pyrazol-3-yl)-6-(tetrahydro-3-furanylsulfonyl)-4-quinolinamine

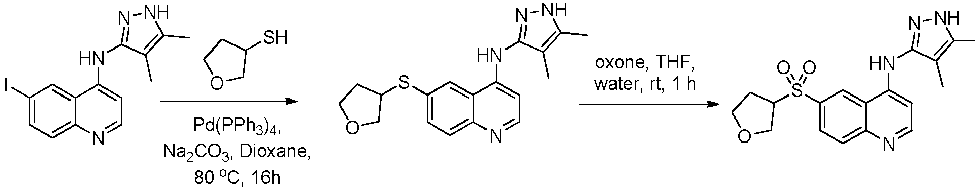

### Step 1: N-(4,5-dimethyl-1H-pyrazol-3-yl)-6-(tetrahydro-3-furanylthio)-4-quinolinamine:

Method A: *N*-(4,5-dimethyl-1*H*-pyrazol-3-yl)-6-iodo-4-quinolinamine (620 mg, 1.7 mmol), sodium carbonate (630 mg, 6.0 mmol), tetrakis(triphenylphosphonium)palladium(0) (295 mg, 0.255 mmol) and 1,4-dioxane (11.3 mL) were combined and purged with nitrogen for 10 min. Tetrahydro-3-furanthiol (177 mg, 1.70 mmol) was added and the reaction was heated at 80 °C for 16 h. The reaction was partitioned between EtOAc and a solution of aqueous sodium thiosulfate/ NaHCO₃ (5:1, 2M). The aqueous layer was extracted with EtOAc (1x) and the combined organic extracts were dry-loaded onto silica. The crude product was purified via flash column chromatography (ISCO-Rf) (40 g, 0-15% MeOH/DCM) to afford *N*-(4,5-dimethyl-1*H-*pyrazol-3-yl)-6-(tetrahydro-3-furanylthio)-4-quinolinamine (135 mg, 0.397 mmol, 23.3 % yield). ¹H NMR (400 MHz, DMSO-*d*₆) δ ppm 12.24 (s, 1 H), 8.79 (s, 1 H), 8.25 - 8.44 (m, 2 H), 7.78 (d, *J* = 8.8 Hz, 1 H), 7.62 (dd, *J* = 8.7, 1.9 Hz, 1 H), 6.51 (d, *J* = 5.1 Hz, 1 H), 4.15 - 4.24 (m, 1 H), 4.09 - 4.15 (m, 1 H), 3.82 - 3.91 (m, 1 H), 3.74 - 3.82 (m, 1 H), 3.60 (dd, *J* = 9.0, 4.7 Hz, 1 H), 2.35 - 2.46 (m, 1 H), 2.20 (s, 3 H), 1.81 - 1.89 (m, 1 H), 1.79 (s, 3 H). MS (m/z) 341 (M+H⁺).

Alternatively, the Pd catalyzed coupling can be achieved by Method B (see table below): A mixture of quinoline (1 eq.), thiol (1 eq.), potassium tert-butoxide (3 eq.), (oxydi-2,1-phenylene)bis-(diphenylphosphine) (0.1 eq.) and bis(dibenzylidineacetone)palladium (0.1 eq.) in DMF (0.15 M) was heated at 100 °C in a sealed, nitrogen-purged vial for 2 h. The reaction was poured into EtOAc and washed 2x with satd. ammonium chloride and satd. NaHCO₃ followed by brine. The organics were dried over MgSO₄ and the solvent was removed *in vacuo* followed by chromatographic purification.

Step 2: *N*-(4,5-dimethyl-1*H*-pyrazol-3-yl)-6-(tetrahydro-3-furanylsulfonyl)-4-quinolinamine: A mixture of *N*-(4,5-dimethyl-1*H*-pyrazol-3-yl)-6-(tetrahydro-3-furanylthio)-4-quinolinamine (130 mg, 0.382 mmol), oxone (258 mg, 0.420 mmol), THF (2 mL) and water (0.60 mL) was stirred at rt for 1 h. The reaction was partitioned between EtOAc and satd. aqueous NaHCO₃. The aqueous layer was extracted with EtOAc (1x) and the combined organic extracts were washed with brine, dried over magnesium sulfate, filtered, dry-loaded onto silica gel and purified via column chromatography (ISCO-Rf, 12g, 0-10% MeOH/DCM) to afford *N*-(4,5-dimethyl-1*H*-pyrazol-3-yl)-6-(tetrahydro-3-furanylsulfonyl)-4-quinolinamine (110 mg, 0.295 mmol, 77 % yield). ¹H NMR (400 MHz, DMSO-*d*₆) δ ppm 14.36 (br. s., 1 H), 11.24 (br. s., 1 H), 9.41 (br. s., 1 H), 8.65 (d, *J* = 6.8 Hz, 1 H), 8.42 (d, *J* = 8.8 Hz, 1 H), 8.18 (d, *J* = 8.8 Hz, 1 H), 6.88 (d, *J* = 6.8 Hz, 1 H), 4.31 (d, *J* = 8.8 Hz, 1 H), 4.10 - 4.22 (m, 1 H), 3.74 - 3.93 (m, 2 H), 3.67 (d, *J* = 7.3 Hz, 1 H), 2.12 - 2.37 (m, 5 H), 1.88 (s, 3 H). MS (m/z) 373 (M+H⁺).

The following compound was made in the same manner from a commercial thiol:

| Ex # | Structure | Name | MS (M+H) ⁺ | NMR | Method |
|---|---|---|---|---|---|
| 19 | 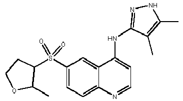 | *N*-(4,5-dimethyl-1*H-*pyrazol-3-yl)-6-[(2-methyltetrahydro-3-furanyl)sulfonyl]-4-quinolinamine | 387 | ¹H NMR (400 MHz, METHANOL-*d*₄) δ ppm 9.21 - 9.34 (m, 1 H), 8.53 (d, *J* = 7.1 Hz, 1 H), 8.47 (dd, *J* = 9.0, 1.9 Hz, 1 H), 8.15 (d, *J* = 9.1 Hz, 1 H), 6.91 - 7.02 (m, 1 H), 4.33 (quin, *J* = 6.7 Hz, 1 H), 4.02 - 4.19 (m, 2 H), 3.69 (q, *J* = 8.3 Hz, 1 H), 2.35 - 2.49 (m, 1 H), 2.34 (s, 3 H), 2.13 (dddd, *J* = 12.9, 9.0, 7.5, 3.8 Hz, 1H), 1.98 (s, 3 H), 1.65 (d, *J* = 6.6 Hz, 3 H) | A |
| 20 | 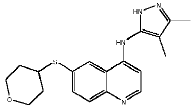 | *N*-(3,4-dimethyl-1*H-*pyrazol-5-yl)-6-(tetrahydro-2H-pyran-4-ylthio)-4-quinolinamine | 355.3 | ¹H NMR (CHLOROFORM-*d*) δ: 8.55 (d, *J* =5.3 Hz, 1H), 8.07 (s, 1H), 7.93 - 8.04 (m, 2H), 7.72 (dd, *J* = 8.6, 1.8 Hz, 1H), 6.92 (d, *J* = 5.3 Hz, 2H), 3.94 - 4.03 (m, 1H), 3.36 - 3.54 (m, 3H), 2.31 (s, 3H), 1.94 - 2.01 (m, 1H), 1.94 (s, 3H), 1.66 - 1.81 (m, 2H), 1.25 - 1.31 (m, 1H), 0.94 (m, 1H) | B |
| 21 | 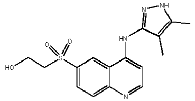 | 2-({4-[(4,5-dimethyl-1*H*-pyrazol-3-yl)amino]-6-quinolinyl} sulfonyl) ethanol | 347.3 | ¹H NMR (METHANOL-*d*₄) δ: 9.06 (d, *J* = 1.8 Hz, 1H), 8.48 (d, *J* = 5.6 Hz, 1H), 8.10 - 8.22 (m, 1H), 8.06 (d, *J* = 8.8 Hz, 1H), 6.64 (d, *J* = 4.8 Hz, 1H), 3.95 (t, *J* = 6.1 Hz, 2H), 3.56 (t, *J* = 6.1 Hz, 2H), 2.31 (s, 3H), 1.91 (s, 3H) | B |

### Example 22

### Ethyl 3-({6-[(1,1-dimethylethyl)sulfonyl]-4-quinolinyl}amino)-4-methyl-1H-pyrazole-5-carboxylate

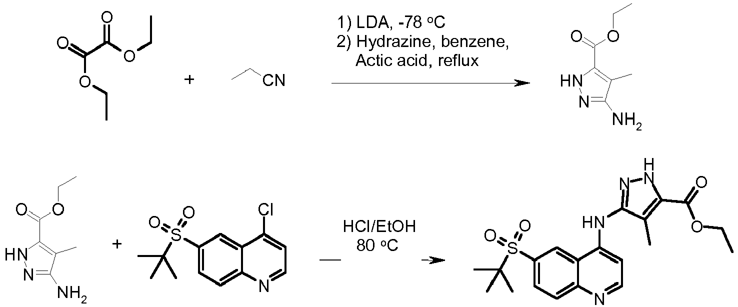

Step 1: Ethyl 3-amino-4-methyl-1*H*-pyrazole-5-carboxylate. To a stirred solution ofpropionitrile (1 g, 18.16 mmol) in THF (40 mL) cooled to -78 °C was added LDA in Hep/THF/EthylPh (10.9 mL, 21.8 mmol) dropwise. The reaction mixture was stirred for 1 hr, then added to a solution of diethyl oxalate (2.65 g, 18.2 mmol) in THF (40 mL) cooled at -78 °C. The resulting solution was stirred at -78 °C for 2 hrs, allowed warm to 0 °C, and quenched by addition of aqueous NH₄Cl, followed by 3N HCl to pH=5. The two resultant layers were separated and the aqueous layer was extracted with EtOAc (2 x 100 mL). The extracts were combined, washed with brine, dried over MgSO₄, and filtered. The solution was partially concentrated resulting in a yellow precipitate was filtered away. The remaining solution was further concentrated to give a brown oil. The residue oil and hydrazine (1.140 mL, 36.3 mmol) were dissolved in acetic acid (3 mL) and benzene (100 mL) and refluxed for 16 hrs using Dean Stark trap (1.5 mL of water was collected). The reaction was cooled to room temperature, and the solution was decanted (some precipitates formed on the bottom of the flask) and solvent was removed via rotovap. Brine (20 mL) was added to the mixture which was then extracted with EtOAc (3 x 70 mL). The combined extracts were washed with water, dried over (MgSO₄), filtered, and concentrated to give a colorless oil. The precipitate from the reaction was partitioned between EtOAc and saturated sodium bicarbonate, organic was washed with brine, dried over MgSO₄, filtered, and combined with the oil above. The solvent was removed via rotovap to give a white solid ethyl 3-amino-4-methyl-1H-pyrazole-5-carboxylate (1.92 g, 11.35 mmol, 62.5 % yield) as the desired product. ). ¹H NMR (Chloroform-*d*) δ: 4.37 (q, *J* = 7.1 Hz, 2H), 2.15 (s, 3H), 1.38 (t, *J*= 7.2 Hz, 3H); MS (m/z) 170 (M+H⁺).

Step 2: Ethyl 3-({6-[(1,1-dimethylethyl)sulfonyl]-4-quinolinyl}amino)-4-methyl-1*H*-pyrazole-5-carboxylate. 6-(tert-Butylsulfonyl)-4-chloroquinoline (500 mg, 1.76 mmol) and ethyl 3-amino-4-methyl-1H-pyrazole-5-carboxylate (328 mg, 1.94 mmol) were dissolved in EtOH with two drops of HCl (4M in dioxane) added. The reaction mixture was heated at 80 °C for 6 hrs, then cooled to room temperature and partitioned between EtOAc and saturated sodium bicarbonate. The organic layer was washed with brine, dried over MgSO₄, filtered, and concentrated. The residue solid was washed with DCM to give the desired product ethyl 3-((6-(tert-butylsulfonyl)quinolin-4-yl)amino)-4-methyl-1H-pyrazole-5-carboxylate (525 mg, 1.26 mmol, 71.5 % yield) as a yellow solid. ¹H NMR (DMSO-*d*₆) δ: 13.70 (br. s., 1H), 9.44 - 9.69 (m, 1H), 9.06 (s, 1H), 8.45 - 8.65 (m, 1H), 7.89 - 8.14 (m, 2H), 6.52 (br. s., 1H), 4.22 - 4.44 (m, 2H), 2.09 (s, 3H), 1.35 (s, 3H), 1.32 (s, 9H); MS (m/z) 417 (M+H⁺).

### Example 23

### 3-({6-[(1,1-Dimethylethyl)sulfonyl]-4-quinolinyl}amino)-4-methyl-1H-pyrazol-5-yl]methanol

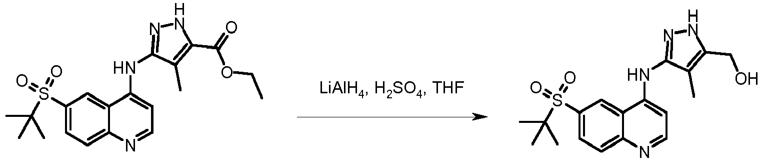

A solution of 1M LiAlH₄ (in THF, 0.960 mL, 0.960 mmol) in THF (0.5 mL) was cooled to -78 °C, a solution of sulfuric acid (0.026 mL, 0.48 mmol) in THF (0.5 ml) was added. The mixture was allowed to warm up to room temperature, then added to a solution of ethyl 3-((6-(tert-butylsulfonyl)quinolin-4-yl)amino)-4-methyl-1H-pyrazole-5-carboxylate (100 mg, 0.240 mmol) in THF (2 mL) (heating was requited for dissolution of the carboxylate) at room temperature and a red suspension was obtained. After stirring at room temperature for 30 min, the reaction mixture was quenched with saturated Na₂SO₄ (0.3 mL), then 2N NaOH (0.5 mL), and stirred for 10 min. The solid was filtered, and the filtrate was concentrated and purified on a Gilson HPLC, using 5-50% ACN/water/TFA (0.1%), 30 min gradient. Fractions containing the desired product were combined and partitioned between EtOAc and saturated NaHCO₃. The organic layer was separated, washed with brine, dried over MgSO₄, filtered, and concentrated. The residue was dried under high vacuum for 16 hrs to give (3-((6-(tert-butylsulfonyl)quinolin-4-yl)amino)-4-methyl-1H-pyrazol-5-yl)methanol (32 mg, 0.085 mmol, 35.6 % yield) as a white solid. ¹HNMR (DMSO-*d*₆) δ: 12.52 (s, 1H), 9.43 (s, 1H), 9.07 (d, *J* = 1.8 Hz, 1H), 8.48 - 8.59 (m, 1H), 7.90 - 8.08 (m, 2H), 6.52 - 6.65 (m, 1H), 5.24 (br. s., 1H), 4.49 (s, 2H), 1.86 (s, 3H), 1.32 (s, 9H); MS (m/z) 375 (M+H⁺).

### Example 24

### [3-({6-[(1,1-Dimethylethyl)sulfonyl]-4-quinolinyl}amino)-5-methyl-1H-pyrazol-4-yl]methanol

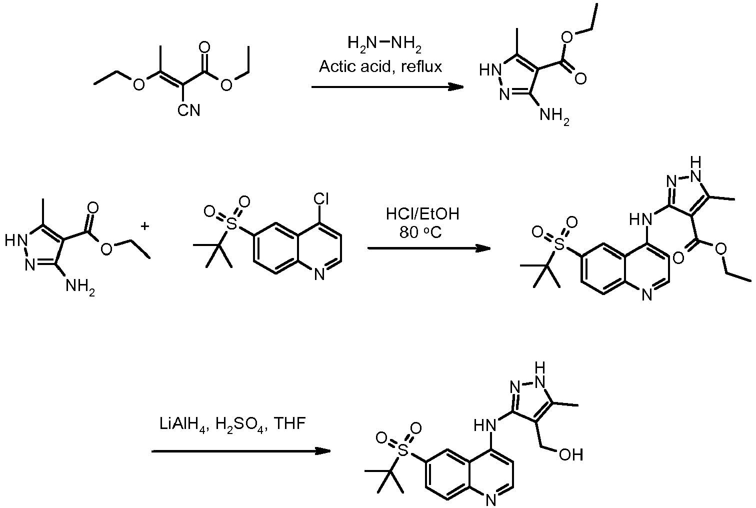

Step 1: Ethyl 3-amino-5-methyl-1*H*-pyrazole-4-carboxylate. (E)-Ethyl 2-cyano-3-ethoxybut-2-enoate (2 g, 10.92 mmol) was dissolved in acetic acid (20 mL) and hydrazine (0.857 mL, 27.3 mmol) was added. The reaction mixture was heated at 110 °C for 30 min. The solvent was removed *in vacuo.* Brine (20 mL) was added and the mixture was then extracted with CHCl₃ (3 x 20 mL). The combined extracts were washed with water, dried over MgSO₄, filtered, and concentrated to give a colorless oil ethyl 3-amino-5-methyl-1H-pyrazole-4-carboxylate (1.9 g, 11.23 mmol, 103 % yield). ¹H NMR (Chloroform-*d*) δ: 4.31 (q, 2H), 2.44 (s, 3H), 1.30 - 1.47 (m, 3H); MS (m/z) 170 (M+H⁺).

Step 2: Ethyl 3-({6-[(1,1-dimethylethyl)sulfonyl]-4-quinolinyl}amino)-5-methyl-1*H*-pyrazole-4-carboxylate. 6-(*t*-Butylsulfonyl)-4-chloroquinoline (200 mg, 0.71 mmol) and ethyl 3-amino-5-methyl-1H-pyrazole-4-carboxylate (131 mg, 0.78 mmol) were dissolved in EtOH (2 mL) with a drop of HCl (4M in dioxane). The reaction mixture was heated at 80 °C for 16 hrs. The resulting precipitate was filtered and air dried to give a pale solid ethyl 3-((6-(tert-butylsulfonyl)quinolin-4-yl)amino)-5-methyl-1H-pyrazole-4-carboxylate, hydrochloride (190 mg, 0.419 mmol, 59.5 % yield). ¹H NMR (DMSO-*d*₆) δ: 13.56 (s, 1H), 11.08 - 11.29 (m, 1H), 9.15 (s, 1H), 8.78 (d, *J* = 6.8 Hz, 1H), 8.26 - 8.41 (m, 2H), 7.40 - 7.53 (m, 1H), 4.17 (d, *J* = 7.1 Hz, 2H), 2.54 (s, 3H), 1.33 (s, 9H), 1.09 (t, *J* = 7.2 Hz, 3H); MS (m/z) 417 (M+H⁺).

Step 3: [3-({6-[(1,1-Dimethylethyl)sulfonyl]-4-quinolinyl}amino)-5-methyl-1H-pyrazol-4-yl]methanol. A solution of 1M LiAlH₄ (in THF, 0.44 mL, 0.88 mmol) in THF (0.5 mL) was cooled to -78 °C, and a solution of sulfuric acid (0.024 mL, 0.44 mmol) in THF (0.5 mL) was added. The mixture was allowed to warm up to room temperature, and was then added to a suspension of ethyl 3-((6-(tert-butylsulfonyl)quinolin-4-yl)amino)-5-methyl-1H-pyrazole-4-carboxylate hydrochloride (100 mg, 0.221 mmol) in THF (1 mL) at room temperature. A red suspension was obtained. The reaction was stirred for 10 min, quenched with saturated Na₂SO₄ (0.3 mL), then 2N NaOH (0.5 mL) and stirred for 10 min. The solid was filtered, and the filtrate was concentrated and purified by prep-TLC to afford [3-({6-[(1,1-dimethylethyl)sulfonyl]-4-quinolinyl}amino)-5-methyl-1H-pyrazol-4-yl]methanol (8 mg, 0.021 mmol, 10% yield). ¹H NMR (Chloroform-*d*) δ: 8.94 (s, 1H), 8.63 - 8.72 (m, 2H), 8.11 - 8.19 (m, 1H), 8.02 - 8.08 (m, 1H), 7.45 - 7.52 (m, 1H), 4.61 (br. s., 2H), 2.27 (br. s., 3H), 1.41 (s, 9H); MS (m/z) 375 (M+H⁺).

### Example 25

### 3-({6-[(1,1-dimethylethyl)sulfonyl]-4-quinolinyl}amino)-5-methyl-1H-pyrazole-4-carboxylic acid

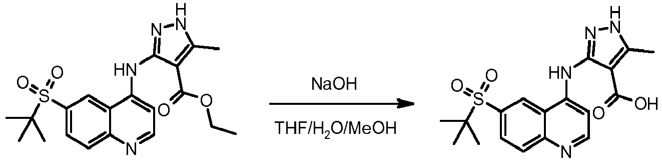

Ethyl 3-((6-(tert-butylsulfonyl)quinolin-4-yl)amino)-5-methyl-1H-pyrazole-4-carboxylate (100 mg, 0.240 mmol) was suspended in a mixture of MeOH (1 mL) and tetrahydrofuran (THF) (1 mL). 2N NaOH (0.25 mL) was added to above mixture and a clear solution was obtained. After heating at 60 °C for 13 hrs, the reaction was not complete as judged by LCMS. NaOH (19.21 mg, 0.48 mmol) was added and heating was continued for 14 hrs at 60 °C. The reaction was concentrated and neutralized with 1N HCl to pH∼6-7. The precipitate was filtered and air dried to give a yellow solid 3-((6-(tert-butylsulfonyl)quinolin-4-yl)amino)-5-methyl-1H-pyrazole-4-carboxylic acid (100 mg, 0.26 mmol, 107 % yield). ¹H NMR (DMSO-*d*₆) δ: 12.54 (s, 1H), 11.56 - 11.96 (m, 1H), 8.78 (d, *J* = 5.3 Hz, 1H), 8.65 (d, *J* = 1.5 Hz, 1H), 8.26 (d, *J* = 5.3 Hz, 1H), 8.08 - 8.13 (m, 1H), 8.02 (d, *J* = 1.8 Hz, 1H), 2.47 (s, 3H), 1.30 (s, 9H); MS (m/z) 389 (M+H⁺).

### Examples 26 and 27

### (R)-N-(3,4-dimethyl-1H-pyrazol-5-yl)-6-((tetrahydro-2H-pyran-4-yl)sulfinyl)quinolin-4-amine and (S)-N-(3,4-dimethyl-1H-pyrazol-5-yl)-6-((tetrahydro-2H-pyran-4-yl)sulfinyl)quinolin-4-amine

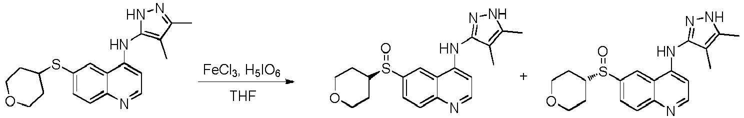

A mixture of N-(3,4-dimethyl-1H-pyrazol-5-yl)-6-(tetrahydro-2H-pyran-4-ylthio)-4-quinolinamine (635 mg, 1.79 mmol) and iron (III) chloride (8.7 mg, 0.054 mmol) was stirred in THF (60 mL). Periodic acid (449 mg, 1.97 mmol) was added and stirred at room temperature for 30min at which point a solution of 1:1 10% sodium bicarbonate:sodium bisulfate (30mL) was added. The product was extracted with EtOAc followed by DCM. The pooled organic layers were dried over MgSO₄, filtered and concentrated *in vacuo.* The residue was purified via Isco CombiFlash (DCM to 10% MeOH in DCM; 80g silica gel cartridge column) and via Gilson reverse phase chromatography (6% to 60% 0.1% TFA in MeCN in 0.1% TFA in water; 5um 30x150 mm Waters Sunfire column) to remove any traces of sulfone. The resulting impure sulfoxide product was purified via column chromatography (0 to 100% 10%NH₄OH in IPA/ EA, 40g silica gel cartridge column) which was again followed by another Gilson reverse phase purification using the above conditions to give desired N-(3,4-dimethyl-1H-pyrazol-5-yl)-6-(tetrahydro-2H-pyran-4-ylsulfinyl)-4-quinolinamine as a white solid (200 mg, 30.1% yield). MS (m/z) 371.2 (M+H⁺); ¹H NMR (CHLOROFORM-d) δ: 12.58 (br. s., 1H), 10.20 (br. s., 1H), 8.92 - 9.05 (m, 1H), 8.54 (d, J = 5.6 Hz, 1H), 8.14 (d, J = 8.6 Hz, 1H), 7.56 (d, J = 8.8 Hz, 1H), 6.47 (d, J = 5.6 Hz, 1H), 4.09 - 4.24 (m, 1H), 3.93 - 4.09 (m, 1H), 3.21 - 3.50 (m, 2H), 2.87 - 3.05 (m, 1H), 2.36 (s, 3H), 1.81 - 2.01 (m, 3H), 1.76 (s, 3H), 1.54 (m 1H). The sulfoxide enantiomers were separated via chiral reverse phase HPLC to yield 40.6 and 45.7 mg of each resolved enantiomer (Chiralpak OD-H column, 4 mL/min MeOH).

### Pharmaceutical Compositions

### Example A

Tablets are prepared using conventional methods and are formulated as follows:

| Ingredient | Amount per tablet |
|---|---|
| Compound of Example 1 | 5mg |
| Microcrystalline cellulose | 100mg |
| Lactose | 100mg |
| Sodium starch glycollate | 30mg |
| Magnesium stearate | 2mg |
| Total | 237mg |

### Example B

Capsules are prepared using conventional methods and are formulated as follows:

| Ingredient | Amount per tablet |
|---|---|
| Compound of Example 3 | 15mg |
| Dried starch | 178mg |
| Magnesium stearate | 2mg |
| Total | 195mg |

### Biological Assay:

A fluorescent polarization based binding assay was developed to quantitate interaction of novel test compounds at the ATP binding pocket of RIPK2, by competition with a fluorescently labeled ATP competitive ligand. Full length FLAG His tagged RIPK2 was purified from a Baculovirus expression system and was used at a final assay concentration of twice the KDapparent. A fluorescent labeled ligand (5-({[2-({[3-({4-[(5-hydroxy-2-methylphenyl)amino]-2-pyrimidinyl}amino)phenyl]carbonyl}amino)ethyl] amino}carbonyl)-2-(6-hydroxy-3-oxo-3H-xanthen-9-yl)benzoic acid, prepared as described below) was used at a final assay concentration of 5nM. Both the enzyme and ligand were prepared in solutions in 50mM HEPES pH7.5, 150mM NaCl, 10mM MgCl2, 1mM DTT, and 1mM CHAPS. Test compounds were prepared in 100% DMSO and 100nL was dispensed to individual wells of a multiwell plate. Next, 5ul RIPK2 was added to the test compounds at twice the final assay concentration, and incubated at rt for 10 minutes. Following the incubation, 5ul of the fluorescent labeled ligand solution, was added to each reaction, at twice the final assay concentration, and incubated at rt for at least 10 minutes. Finally, samples were read on an instrument capable of measuring fluorescent polarization. Test compound inhibition was expressed as percent (%) inhibition of internal assay controls.

For concentration/dose response experiments, normalized data were fit and pIC₅₀s determined using conventional techniques. The pIC₅₀s are averaged to determine a mean value, for a minimum of 2 experiments..

As determined using the above method, the compounds of Examples 1-27 exhibited a pIC₅₀ between 5.0 and 9.0 e.g., for example, the compound of Example 1 inhibited RIP2 kinase in the above method with a mean pIC₅₀ of 8.2.

### FLAG His tagged RIPK2 Preparation:

Full-length human RIPK2 (receptor-interacting serine-threonine kinase 2) cDNA was purchased from Invitrogen (Carlsbad, California, USA, Clone ID:IOH6368, RIPK2-pENTR 221). Gateway^{®} LR cloning was used to site-specifically recombine RIPK2 downstream to an N-terminal FLAG-6His contained within the destination vector pDEST8-FLAG-His6 according to the protocol described by Invitrogen. Transfection into *Spodoptera frugiperda*(Sf9) insect cells was performed using Cellfectin® (Invitrogen), according to the manufacturer's protocol.

Sf9 cells were grown in Excell 420 (SAFC Biosciences, Lenexa, Kansas, US; Andover, Hampshire UK) growth media at 27°C, 80 rpm in shake flask until of a sufficient volume to inoculate a bioreactor. The cells were grown in a 50 litre working volume bioreactor (Applikon, Foster City, California, US; Schiedam, Netherlands) at 27°C, 30% dissolved oxygen and an agitation rate of 60-140 rpm until the required volume was achieved with a cell concentration of approximately 3.7xe6 cells/mL. The insect cells were infected with Baculovirus at a multiplicity of infection (MOI) of 12.7. The cultivation was continued for a 43 hour expression phase. The infected cells were removed from the growth media by centrifugation at 2500 g using a Viafuge (Carr) continuous centrifuge at a flow rate of 80 litres/hour. The cell pellet was immediately frozen and subsequently supplied for purification.

9.83 x 10¹⁰ Insect cells were re-suspended in 1.4 L lysis buffer (50mM Tris (pH 8.0), 150mM NaCl, 0.5mM NaF, 0.1% Triton X-100, 1mL/litre Protease Inhibitor Cocktail Set III (available from EMD Group; CalBiochem/Merck Biosciences, Gibbstown, New Jersey, US; Damstadt, Germany) and processed by dounce homogenization on ice. The suspension was then clarified by centrifugation at 47,900g for 2 hours, at 4°C. The lysate was decanted from the insoluble pellet and loaded at a linear flow rate of 16 cm/h onto a 55 mL FLAG-M2 affinity column (2.6 x 10.4 cm) that had been pre-equilibrated with 10 column volumes buffer A (50mM Tris (pH 8.0), 150mM NaCl, 0.5mM NaF, 1mL/litre Protease Inhibitor Cocktail Set III). The column was then washed with 15 column volumes buffer A, and eluted with 6 column volumes buffer B (buffer A + 150µg/mL 3X FLAG peptide) at a linear flow rate of 57 cm/h. Fractions identified by SDS-PAGE as containing protein of interest were dialyzed to remove the 3X FLAG peptide from the preparation against 5 L of Buffer A (not containing the Protease Inhibitor Cocktail) overnight, using 10 kDa MWCO SnakeSkin Pleated Dialysis Tubing. The purification process yielded 11.3 mg of total protein, with the RIPK2 present at 40% purity by gel densitometry scanning, and identity confirmed by peptide mass fingerprinting. The main contaminating proteins in the preparation were identified as lower molecular weight degraded species of RIPK2.

### Fluorescent Ligand Preparation:

### 2-Methyl-5-(2-propen-1-yloxy)aniline:

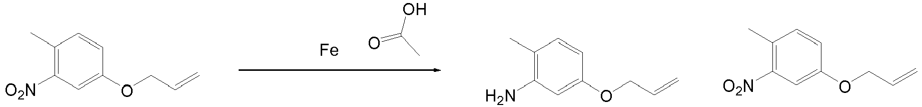

1-Methyl-2-nitro-4-(2-propen-1-yloxy)benzene (25.2 g, 130 mmol) was dissolved in ethanol (280 mL), water (28 mL), and acetic acid (5.6 mL, 98 mmol). Iron (29.1 g, 522 mmol) was added in six portions. The reaction was stirred for 72 hours, and then additional acetic acid (5.6 mL, 98 mmol) and 4 eq. of iron were added. The mixture was filtered through celite rinsing with EtOH and water and the filtrates were concentrated to remove EtOH. Diethylether (300 mL) was added along with 100 mL of 2 N HCl. The layers were separated and the ether layer was extracted with 2x100 mL of 2 N HCl. The acidic aqueous layer was slowly made pH 9 with NaOH pellets, and then dichloromethane (300 mL) was added. The resulting emulsion was filtered using a Buchner funnel. The layers were separated and the aqueous layer extracted with DCM (2 X 100 mL). The combined extracts were dried over MgSO₄, filtered, and concentrated to a dark red oil (15.2 g). The crude material was purified via flash chromatography using a 120 g silica cartridge eluting with 5-15% EtOAc/hexanes for 30 min then 15-30% EtOAc/hexanes for 10 min. to give the titled compound as a red oil. MS (m/z) ¹H NMR (400 MHz, CHLOROFORM-d) δ ppm 2.23 (s, 3 H) 4.51 (dt, *J =* 5.29, 1.51 Hz, 2 H) 5.29 (dd, *J* = 10.45, 1.38 Hz, 1 H) 5.38 - 5.46 (m, 1 H) 5.99 - 6.12 (m, 1 H) 6.01 - 6.10 (m, 1 H) 6.46 (dd, *J* = 8.31, 2.52 Hz, 1 H) 6.56 (d, *J* = 2.52 Hz, 1 H) 7.01 (d, *J* = 8.56 Hz, 1 H); MS 164 (M+H⁺).

### 2-Chloro-N-[2-methyl-5-(2-propen-1-yloxy)phenyl]-4-pyrimidinamine:

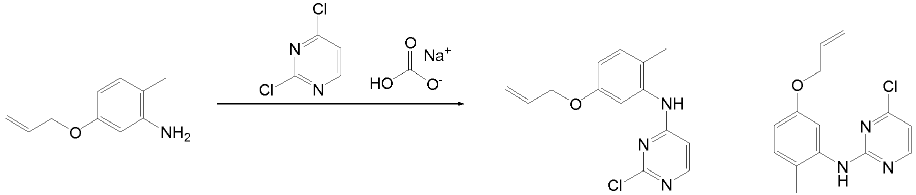

2-Methyl-5-(2-propen-1-yloxy)aniline (11.8 g, 72.3 mmol) was dissolved in *tert*-butanol (103 mL) and 2,4-dichloropyrimidine (10.77 g, 72.3 mmol) was added followed by NaHCO₃ (18.22 g, 217 mmol). The reaction was heated at 80°C for 17 hrs then additional 1,4-dichloropyrimidine (5.38 g, 36.6 mmol) was added and the reaction was stirred for 6 days. Additional 2,4-dichloropyrimidine (2.69 g, 17.8 mmol) was added and the reaction stirred for 2 days. The reaction was cooled to room temp diluting with EtOAc (200 mL) and water (200 mL). The layers were separated and the aqueous layer extracted with EtOAc (2 X 100 mL). The combined extracts were washed with brine (100 mL), dried over Na₂SO₄, filtered, and concentrated. The crude material was purified via flash chromatography using a 330 g silica cartridge eluting with 1-20% EtOAc/hexanes for 30 min then 20% EtOAc/hexanes for 50 min to give the titled compound (15.1 g). ¹H NMR (400 MHz, CHLOROFORM-*d*) δ ppm 2.20 (s, 3 H) 4.54 (d, *J* = 5.29 Hz, 2 H) 5.32 (dd, *J* = 10.45, 1.38 Hz, 1 H) 5.42 (dd, *J* = 17.37, 1.51 Hz, 1 H) 5.99 - 6.12 (m, 1 H) 6.35 (d, *J* = 5.79 Hz, 1 H) 6.83 (dd, *J* = 8.44, 2.64 Hz, 1 H) 6.89 (d, *J* = 2.52 Hz, 6 H) 7.14 (br. s., 6 H) 7.21 (d, *J* = 8.56 Hz, 7 H) 8.10 (d, *J* = 5.79 Hz, 6 H); MS (m/z) 276 (M+H⁺).

### 3-[(4-{[2-Methyl-5-(2-propen-1-yloxy)phenyl]amino}-2-pyrimidinyl)amino]benzoic acid:

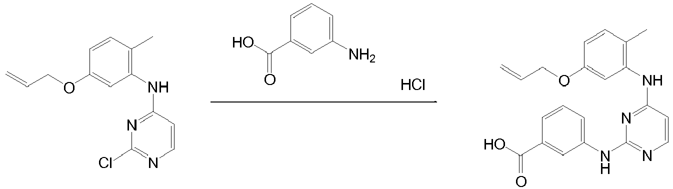

2-Chloro-N-[2-methyl-5-(2-propen-1-yloxy)phenyl]-4-pyrimidinamine (8 g, 29.0 mmol), 3-aminobenzoic acid (3.98 g, 29.0 mmol), and HCl (14.51 mL, 29.0 mmol) were dissolved in acetone (58.0 mL) and water (58.0 mL). The reaction was heated to 60°C for 48 hrs. The reaction was cooled to rt passing air over it and a solid crashed out. Water (150 mL) was added and the solid was filtered washing with 3 X 50 mL water. The solid was dried in the vacuum funnel overnight affording the desired compound (10.9 g). ¹H NMR (400 MHz, METHANOL-*d*₄) δ ppm 2.21 (s, 3 H) 4.47 (d, *J* = 5.04 Hz, 2 H) 5.24 (dd, *J* = 10.58, 1.51 Hz, 1 H) 5.37 (dd, *J =* 17.25, 1.64 Hz, 1 H) 5.97 - 6.09 (m, 4 H) 6.29 - 6.39 (m, 1 H) 6.89 (dd, *J* = 8.44, 2.64 Hz, 4 H) 6.96 (d, *J* = 2.77 Hz, 1 H) 7.23 (d, *J =* 8.56 Hz, 1 H) 7.34 - 7.41 (m, 1 H) 7.75 - 7.79 (m, 1 H) 7.81 (s, 1 H) 7.85 (d, *J* = 7.30 Hz, 3 H) 7.98 - 8.09 (m, 3 H); MS (m/z) 377 (M+H⁺).

### 1,1-Dimethylethyl {2-[({3-[(4-{[2-methyl-5-(2-propen-1-yloxy)phenyl]amino}-2-pyrimidinyl)amino]phenyl}carbonyl)amino]ethyl}carbamate:

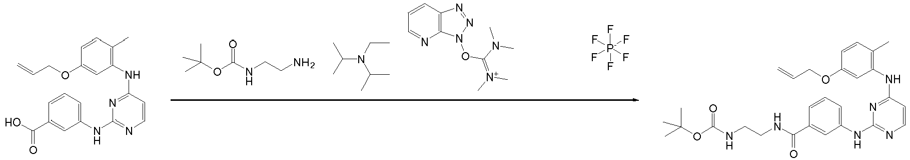

A solution of 3-[(4-{[2-methyl-5-(2-propen-1-yloxy)phenyl]amino}-2-pyrimidinyl)amino]benzoic acid (6.83 g, 18.15 mmol) and DIEA (9.51 mL, 54.4 mmol) in *N,N*-Dimethylformamide (DMF) (51.8 mL). was treated with N-(2-aminoethyl) carbamic acid tert-butyl ester (3.20 g, 19.96 mmol) and HATU (8.28 g, 21.77 mmol). EtOAc/Et₂O (400 mL, 1:1) was added and the layers separated. The organic layer was washed with water (3 X 300 mL) and brine (100 mL), dried over Na₂SO₄, filtered, and concentrated to give the titled compound (8.3 g). ¹H NMR (400 MHz, DMSO-*d*₆) δ ppm 1.38 (s, 9 H) 2.15 (s, 3 H) 3.09 (q, *J* = 6.19 Hz, 2 H) 3.27 (q, *J* = 6.19 Hz, 2 H) 4.51 (d, *J* = 5.27 Hz, 2 H) 5.24 (dd, *J* = 10.54, 1.51 Hz, 1 H) 5.37 (dd, *J* = 17.32, 1.76 Hz, 1 H) 6.02 (m, *J =* 17.29, 10.51, 5.18, 5.18 Hz, 1 H) 6.13 (d, *J* = 5.77 Hz, 1 H) 6.73 (dd, *J* = 8.41, 2.63 Hz, 1 H) 6.90 (t, *J* = 5.65 Hz, 1 H) 7.09 (d, *J* = 2.51 Hz, 1 H) 7.15 (d, *J* = 8.28 Hz, 1 H) 7.17 - 7.22 (m, 1 H) 7.28 (d, *J* = 7.78 Hz, 1 H) 7.94 - 7.99 (m, 2 H) 7.99 - 8.05 (m, 2 H) 8.26 (t, *J* = 5.65 Hz, 1 H) 8.66 (s, 1 H) 9.17 (s, 1 H); MS (m/z) 519 (M+H⁺).

### 1,1-Dimethylethyl [2-({[3-({4-[(5-hydroxy-2-methylphenyl)amino]-2-pyrimidinyl}amino)phenyl]carbonyl}amino)ethyl]carbamate:

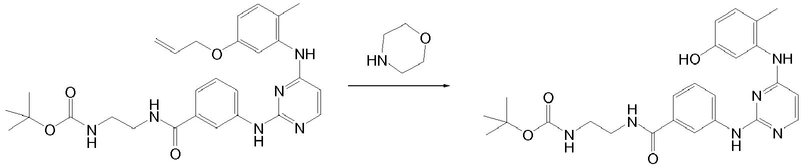

1,1-Dimethylethyl {2-[({3-[(4-{[2-methyl-5-(2-propen-1-yloxy)phenyl]amino}-2-pyrimidinyl)amino]phenyl}carbonyl)amino]ethyl}carbamate (5.5 g, 10.61 mmol) and morpholine (1.016 mL, 11.67 mmol) were dissolved in *N,N-*dimethylformamide (DMF) (42.4 mL). The atmosphere was exchanged for nitrogen and then it was treated with tetrakistriphenylphosphine palladium (0) (1.226 g, 1.061 mmol). The reaction was heated to 80 °C for 3 hrs. The reaction was diluted with EtOAc (250 mL) and washed with water (3 X 200 mL) then brine (100 mL). The organic layer was dried over Na₂SO₄, filtered, and concentrated to about 50 mL and let stand overnight. A solid formed and to the suspension was added 50 mL ether. The solid was filtered washing with ether to give the desired product as an orange solid (4.75 g). ¹H NMR (400 MHz, METHANOL-*d*₄) δ ppm 1.42 (s, 9 H) 2.17 (s, 3 H) 3.29 (t, *J* = 6.04 Hz, 2 H) 3.46 (t, *J* = 6.17 Hz, 2 H) 6.04 (d, *J* = 6.04 Hz, 1 H) 6.65 (dd, *J* = 8.31, 2.52 Hz, 1 H) 6.87 (d, *J* = 2.52 Hz, 1 H) 7.09 (d, *J =* 8.31 Hz, 1 H) 7.27 - 7.33 (m, 1 H) 7.35 - 7.41 (m, 1 H) 7.53 - 7.61 (m, 1 H) 7.62 - 7.70 (m, 2 H) 7.75 (d, *J* = 8.06 Hz, 1 H) 7.91 (d, *J* = 6.04 Hz, 1 H) 8.11 (s, 1 H); MS (m/z) 479 (M+H⁺).

### N-(2-Aminoethyl)-3-({4-[(5-hydroxy-2-methylphenyl)amino]-2-pyrimidinyl} amino)benzamide:

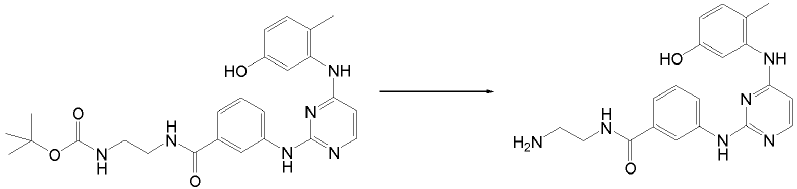

1,1-Dimethylethyl [2-({[3-({4-[(5-hydroxy-2-methylphenyl)amino]-2-pyrimidinyl}amino)phenyl]carbonyl}amino)ethyl]carbamate (4.75 g, 8.93 mmol) (contaminated with tetrakis or related entities) was dissolved in dichloromethane (DCM) (28.6 mL) and trifluoroacetic acid (TFA) (7.15 mL). The reaction concentrated to give the desired product as the TFA salt containing the same impurities going into the reaction (6.5 g) MS (m/z) 379 (M+H⁺).

### 5-({[3-({4-[(5-Hydroxy-2-methylphenyl)amino]-2-pyrimidinyl}amino)phenyl]carbonyl}amino)ethyl]amino}carbonyl)-2-(6-hydroxy-3-oxo-3H-xanthen-9-yl)benzoic acid:

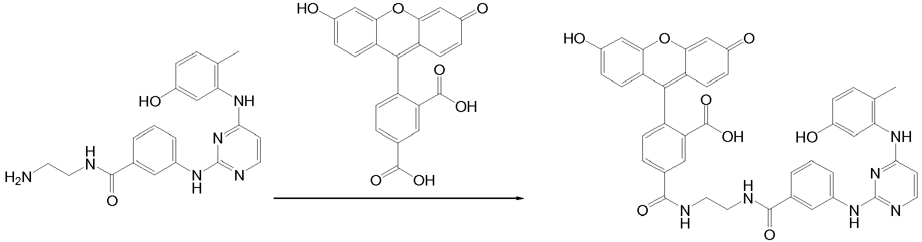

To a suspension of *N*-(2-aminoethyl)-3-({4-[(5-hydroxy-2-methylphenyl)amino]-2-pyrimidinyl}amino)benzamide(1 g, 1.319 mmol) in *N,N-*dimethylformamide (DMF) (13.19 mL) was added 5-FAM (5-carboxyfluorescein single isomer) (0.397 g, 1.055 mmol), triethylamine (0.919 mL, 6.60 mmol), EDC (0.506 g, 2.64 mmol), and HOBT (0.202 g, 1.319 mmol). The reaction was stirred overnight then the pH was adjusted to 3 with 2 N HCl. The solution was extracted with EtOAc (100 mL) and the organic layer washed with water (1 X 50 mL), dried over Na₂SO₄, filtered, and concentrated to give the titled compound. MS (m/z) 737 (M+H⁺).

### Biological in vivo Assay

The efficacy of RIP2 inhibitors may also be evaluated *in vivo* in rodents. Intraperitoneal (*i.p.*) or intravenous (*i.v.*) administration of L18-MDP in mice has been shown to induce an inflammatory response through activation of the NOD2 signaling pathway (Rosenweig, H. L., et al. 2008. Journal of Leukocyte Biology 84:529-536). The level of the inflammatory response in the L18-MDP treated mice/rats is monitored using conventional techniques by measuring increases in cytokine levels (IL8, TNFα, IL6 and IL-1β) in serum and/or peritoneal lavage fluid and by measuring neutrophil influx into the peritoneal space (when L18-MDP is dosed *i.p.*). Inhibition of the L18-MDP induced inflammatory response in treated rodents may be shown by orally pre-dosing with selected compounds of this invention, then measuring and comparing cytokine levels (IL8, TNFα, IL6 and IL-1β) in serum and/or peritoneal lavage fluid and neutrophil influx into the peritoneal space (when L18-MDP is dosed *i.p.*) using conventional techniques.

For example, rats were orally pre-dosed with the compound of Example 1 at 10 mg/kg (8 rats) and the compound Example 3 at 10 mg/kg (8 rats) and with prednisolone (8 rats, used as a positive control), followed by dosing with L18-MDP (50 µg/rat) 0.25 hours/minutes after pre-dosing. Combined cytokine levels (IL8, TNFα, IL6 and IL-1β) in whole blood samples taken from the rats in this study were measured using an antibody based detection (Meso-Scale Discovery platform). The combined cytokine response was calculated as the averaged response for the 4 cytokines measured relative to the response observed in the vehicle-treated mice, and are depicted in the figures as the mean ± standard error of the mean (n=8 rats /group).

## Claims

1. A compound according to Formula (I): 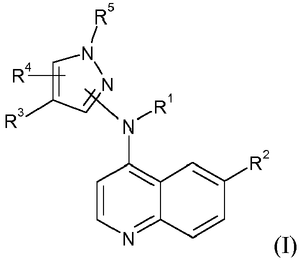 wherein:
R¹ is H, -SO₂(C₁-C₄)alkyl, -CO(C₁-C₄)alkyl, or (C₁-C₄)alkyl;
R² is - SR^{a}, -SOR^{a}, or -SO₂R^{a}, wherein R^{a} is (C₁-C₆)alkyl, halo(C₁-C₆)alkyl, (C₃-C₇)cycloalkyl, 4-7 membered heterocycloalkyl, aryl, or heteroaryl, wherein:
said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄)alkyl, (C₃-C₇)cycloalkyl, phenyl, 5-6 membered heteroaryl, 9-10 membered heteroaryl, 4-7 membered heterocycloalkyl and (phenyl)(C₁-C₄ alkyl)amino-, wherein said (C₃-C₇)cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl and (C₁-C₄)alkoxy,
said (C₃-C₇)cycloalkyl or 4-7 membered heterocycloalkyl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl-, oxo and (C₁-C₄)alkoxy, and
said aryl is an aromatic, monovalent monocyclic or bicyclic hydrocarbon radical containing from 6 to 10 carbon ring atoms, which may be fused to one or more cycloalkyl rings; and said heteroaryl is an aromatic monovalent monocyclic or bicyclic radical, containing 5 to 10 ring atoms, including 1 to 4 heteroatoms selected from nitrogen, oxygen and sulfur, and wherein said aryl or heteroaryl is optionally substituted by 1-3 groups each independently selected from halogen, -CF₃, hydroxyl, amino, ((C₁-C₄)alkyl)amino-, ((C₁-C₄)alkyl)((C₁-C₄)alkyl)amino-, (C₁-C₄)alkyl, phenyl(C₁-C₄)alkyl-, hydroxy(C₁-C₄)alkyl-and (C₁-C₄)alkoxy;
R³ is methyl, hydroxymethyl, trifluoromethyl, carboxy, or -CO₂(C₁-C₄)alkyl;
R⁴ is H, methyl, hydroxymethyl, trifluoromethyl, carboxy, or -CO₂(C₁-C₄)alkyl;
R⁵ is H or (C₁-C₃)alkyl;
or a salt thereof, or hydrate thereof

2. The compound, salt, or hydrate according to claim 1, wherein:
R² is -SR^{a} or -SO₂R^{a}, wherein R^{a} is (C₁-C₆)alkyl, (C₃-C₇)cycloalkyl, 4-7 membered heterocycloalkyl, aryl, or heteroaryl, wherein:
said (C₁-C₆)alkyl is optionally substituted by one or two groups each independently selected from cyano, hydroxyl, (C₁-C₆)alkoxy, (C₁-C₆)alkoxy(C₂-C₆)alkoxy, -CO₂H, -CO₂(C₁-C₄)alkyl, -SO₂(C₁-C₄ alkyl), (C₃-C₇)cycloalkyl, phenyl, 5-6 membered heteroaryl, 9-10 membered heteroaryl, 4-7 membered heterocycloalkyl and (phenyl)(C₁-C₄ alkyl)amino-, wherein said (C₃-C₇)cycloalkyl, phenyl, (phenyl)(C₁-C₄ alkyl)amino-, 5-6 membered heteroaryl, 9-10 membered heteroaryl or 4-7 membered heterocycloalkyl is optionally substituted by 1-3 groups each independently selected from halogen, -CF₃, (C₁-C₄)alkyl, hydroxy(C₁-C₄)alkyl and (C₁-C₄)alkoxy,
said (C₃-C₇)cycloalkyl or 4-7 membered heterocycloalkyl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl-, oxo and (C₁-C₄)alkoxy, and
said aryl is an aromatic, monovalent monocyclic or bicyclic hydrocarbon radical containing from 6 to 10 carbon ring atoms, which may be fused to one or more cycloalkyl rings; and said heteroaryl is an aromatic monovalent monocyclic or bicyclic radical, containing 5 to 10 ring atoms, including 1 to 4 heteroatoms selected from nitrogen, oxygen and sulfur, and wherein said aryl or heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy;
R³ is methyl or trifluoromethyl;
R⁴ is H, methyl or trifluoromethyl; and
R⁵ is H or (C₁-C₃)alkyl.

3. The compound, salt, or hydrate according to claim 1 or claim 2, wherein R¹ is H.

4. The compound, salt, or hydrate according to any one of claims 1-3, wherein R² is -SO₂R^{a}.

5. The compound, salt, or hydrate according to any one of claims 1-4, wherein R^{a} is (C₁-C₆)alkyl, wherein said (C₁-C₆)alkyl is optionally substituted by one or two groups each independently selected from hydroxyl, (C₁-C₄)alkoxy, -CO₂(C₁-C₄)alkyl, -SO₂(C₁-C₄)alkyl, and a (C₃-C₆)cycloalkyl, phenyl, 4-6-membered heterocycloalkyl, 5-6-membered heteroaryl, or 9-10-membered heteroaryl, where said (C₃-C₆)cycloalkyl, phenyl, 4-6-membered heterocycloalkyl, 5-6-membered heteroaryl, or 9-10-membered heteroaryl is optionally substituted by 1-3 groups each independently selected from halogen, -CF₃, hydroxyl, amino, (C₁-C₄)alkyl, phenyl(C₁-C₄)alkyl-, hydroxy(C₁-C₄)alkyl- and (C₁-C₄)alkoxy.

6. The compound, salt, or hydrate according to any one of claims 1-5, wherein R^{a} is -CH₃, -CF₃, -CH₂CF₃, -CH(CH₃)₂, -C(CH₃)₃, -CH₂CH₂OH, (4-fluoro)-tetrahydro-2H-pyran-4-yl, (4-methyl)-tetrahydro-2H-pyran-4-yl, -CH₂-tetrahydro-2H-furan-2-yl, tetrahydro-2H-furan-3-yl, 2-methyl-tetrahydro-2H-furan-3-yl or tetrahydro-2H-pyran-4-yl.

7. The compound, salt, or hydrate according to any one of claims 1-6, wherein R⁵ is H or methyl.

8. The compound, salt, or hydrate according to any one of claims 1-7, wherein R³ and R⁴ are both methyl and R⁵ is H.

9. The compound, salt, or hydrate according to claim 1, wherein R¹ is H or -CH₃; R² is -SR^{a}, -SOR^{a}, or -SO₂R^{a}; wherein R^{a} is (C₁-C₄)alkyl, halo(C₁-C₄)alkyl, hydroxy(C₁-C₄)alkyl, 5-6 membered heterocycloalkyl, or -(C₁-C₂)alkyl-(5-6 membered heterocycloalkyl), wherein any of said heterocycloalkyl contains one heteroatom selected from N, O and S; R³ is methyl, hydroxymethyl or carboxy and R⁴ is H, methyl or trifluoromethyl, or R³ is methyl and R⁴ is H, methyl, trifluoromethyl, hydroxymethyl- or ethyl-carboxy-; and R⁵ is H or (C₁-C₃)alkyl.

10. A compound according to claim 1 which is:
6-[(1,1-dimethylethyl)sulfonyl]-*N*-(4,5-dimethyl-1*H*-pyrazol-3-yl)-4-quinolinamine,
*N*-(4,5-dimethyl-1*H*-pyrazol-3-yl)-6-(isopropylsulfonyl) quinolin-4-amine,
*N*-(4,5-dimethyl-1*H*-pyrazol-3-yl)-6-((tetrahydro-2H-pyran-4-yl)sulfonyl)quinolin-4-amine,
*N*-(3,4-dimethyl-1*H*-pyrazol-5-yl)-6-(methylsulfonyl)-4-quinolinamine,
*N*-(4,5-dimethyl-1*H*-pyrazol-3-yl)-6-[(trifluoromethyl) sulfonyl]-4-quinolinamine,
6-[(1,1-dimethylethyl)sulfonyl]-*N*-[4-methyl-5-(trifluoromethyl)-1*H*-pyrazol-3-yl]-4-quinolinamine,
6-[(1,1-dimethylethyl)sulfonyl]-*N*-(1,3,4-trimethyl-1*H*-pyrazol-5-yl)-4-quinolinamine,
6-[(1-methylethyl)sulfonyl]-*N-*[4-methyl-5-(trifluoromethyl)-1*H-*pyrazol-3-yl]-4-quinolinamine,
*N*-(4-methyl-5-(trifluoromethyl)-1*H*-pyrazol-3-yl)-6-((tetrahydro-2*H*-pyran-4-yl)sulfonyl)quinolin-4-amine,
*N*-(3,4-dimethyl-1*H*-pyrazol-5-yl)-6-(((tetrahydrofuran-2-yl)methyl)sulfonyl) quinolin-4-amine,
*N*-(3,4-dimethyl-1*H*-pyrazol-5-yl)-6-[(2,2,2-trifluoroethyl)sulfonyl]-4-quinolinamine,
6-(tert-butylsulfonyl)-*N*-(4,5-dimethyl-1H-pyrazol-3-yl)-N-methylquinolin-4-amine,
(*R*)-6-(tert-butylsulfinyl)-*N*-(4,5-dimethyl-1H-pyrazol-3-yl)quinolin-4-amine,
(*S*)-6-(tert-butylsulfinyl)-*N*-(4,5-dimethyl-1H-pyrazol-3-yl)quinolin-4-amine,
*N*-(4,5-dimethyl-1H-pyrazol-3-yl)-6-((4-fluorotetrahydro-2*H*-pyran-4-yl)sulfonyl)quinolin-4-amine,
*N*-(4,5-dimethyl-1*H*-pyrazol-3-yl)-6-((4-methyltetrahydro-2H-pyran-4-yl)sulfonyl)quinolin-4-amine,
*N*-(4,5-dimethyl-1*H*-pyrazol-3-yl)-6-(tetrahydro-3-furanylsulfonyl)-4-quinolinamine,
*N*-(4,5-dimethyl-1*H*-pyrazol-3-yl)-6-[(2-methyltetrahydro-3-furanyl)sulfonyl]-4-quinolinamine,
*N*-(3,4-dimethyl-1*H*-pyrazol-5-yl)-6-(tetrahydro-2H-pyran-4-ylthio)-4-quinolinamine,
2-({4-[(4,5-dimethyl-1*H*-pyrazol-3-yl)amino]-6-quinolinyl}sulfonyl) ethanol,
ethyl 3-({6-[(1,1-dimethylethyl)sulfonyl]-4-quinolinyl}amino)-4-methyl-1*H*-pyrazole-5-carboxylate,
3-({6-[(1,1-Dimethylethyl)sulfonyl]-4-quinolinyl}amino)-4-methyl-1*H*-pyrazol-5-yl]methanol,
[3-({6-[(1,1-Dimethylethyl)sulfonyl]-4-quinolinyl}amino)-5-methyl-1*H*-pyrazol-4-yl]methanol,
3-({6-[(1,1-dimethylethyl)sulfonyl]-4-quinolinyl}amino)-5-methyl-1*H*-pyrazole-4-carboxylic acid,
(*R*)-*N*-(3,4-dimethyl-1H-pyrazol-5-yl)-6-((tetrahydro-2H-pyran-4-yl)sulfinyl)quinolin-4-amine, and
(*S*)-*N*-(3,4-dimethyl-1H-pyrazol-5-yl)-6-((tetrahydro-2H-pyran-4-yl)sulfinyl)quinolin-4-amine,
or a salt thereof, or hydrate thereof.

11. A pharmaceutical composition comprising the compound, salt, or hydrate according to any one of claims 1-10, wherein the salt is a pharmaceutically acceptable salt, and one or more pharmaceutically acceptable excipients.

12. A compound, salt or hydrate according to any one of claims 1-10, wherein the salt is a pharmaceutically acceptable salt, for use in therapy.

13. A compound, salt, or hydrate according to any one of claims 1-10, wherein the salt is a pharmaceutically acceptable salt, for use in the treatment of a disease mediated by inhibition of RIP2 kinase, wherein the disease mediated by inhibition of RIP2 kinase is selected from uveitis, interleukin-1 converting enzyme associated fever syndrome, dermatitis, acute lung injury, type 2 diabetes mellitus, arthritis, rheumatoid arthritis, ulcerative colitis, Crohn's disease, early-onset and extra-intestinal inflammatory bowel disease, prevention of ischemia reperfusion injury in solid organ transplant, non-alcohol steatohepatitis, alcohol steatohepatitis, autoimmune hepatitis, asthma, graft versus host disease, systemic lupus erythematosus, multiple sclerosis, sarcoidosis, Blau syndrome/early-onset sarcoidosis, Wegner's granulomatosis, and interstitial pulmonary disease.

14. The use of a compound, salt, or hydrate according to any one of claims 1-10, wherein the salt is a pharmaceutically acceptable salt, in the manufacture of a medicament for use in the treatment of a disease mediated by inhibition of RIP2 kinase, wherein the disease mediated by inhibition of RIP2 kinase is selected from uveitis, interleukin-1 converting enzyme associated fever syndrome, dermatitis, acute lung injury, type 2 diabetes mellitus, arthritis, rheumatoid arthritis, ulcerative colitis, Crohn's disease, early-onset and extra-intestinal inflammatory bowel disease, prevention of ischemia reperfusion injury in solid organ transplant, non-alcohol steatohepatitis, alcohol steatohepatitis, autoimmune hepatitis, asthma, graft versus host disease, systemic lupus erythematosus, multiple sclerosis, sarcoidosis, Blau syndrome/early-onset sarcoidosis, Wegner's granulomatosis, and interstitial pulmonary disease.

## Patentansprüche

1. Verbindung gemäss Formel (I): 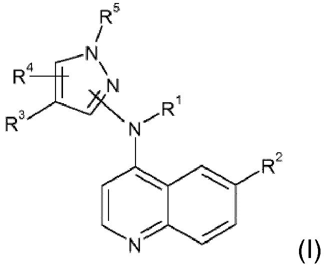 worin:
R¹ H, -SO₂(C₁₋₄)-Alkyl, -CO(C₁₋₄)-Alkyl oder (C₁₋₄)-Alkyl ist;
R² -SR^{a}, -SOR^{a} oder -SO₂R^{a} ist, worin R^{a} (C₁₋₆)-Alkyl, Halogen-(C₁₋₆)-alkyl, (C₃₋₇)-Cycloalkyl, 4- bis 7-gliedriges Heterocycloalkyl, Aryl oder Heteroaryl ist, wobei:
das (C₁₋₆)-Alkyl gegebenenfalls mit einer oder zwei Gruppe(n) substituiert ist, die jeweils unabhängig aus Cyano, Hydroxyl, (C₁₋₆)-Alkoxy, (C₁₋₆)-Alkoxy-(C₂₋₆)-alkoxy, -CO₂H, -CO₂(C₁₋₄)-Alkyl, -SO₂(C₁₋₄)-Alkyl, (C₃₋₇)-Cycloalkyl, Phenyl, 5- bis 6-gliedrigem Heteroaryl, 9- bis 10-gliedrigem Heteroaryl, 4- bis 7-gliedrigem Heterocycloalkyl und (Phenyl)(C₁₋₄-alkyl)-amino- ausgewählt ist/sind, wobei das (C₃₋₇)-Cycloalkyl, Phenyl, (Phenyl)(C₁₋₄-alkyl)-amino-, 5- bis 6-gliedrige Heteroaryl, 9- bis 10-gliedrige Heteroaryl oder 4- bis 7-gliedrige Heterocycloalkyl gegebenenfalls mit 1 bis 3 Gruppe(n) substituiert ist, die unabhängig aus Halogen, -CF₃, Hydroxyl, Amino, ((C₁₋₄)-Alkyl)amino-, ((C₁₋₄)-Alky) ((C₁₋₄)-alkyl)-amino-, (C₁₋₄)-Alkyl, Phenyl-(C₁₋₄)-alkyl-, Hydroxy-(C₁₋₄)-alkyl und (C₁₋₄)-Alkoxy ausgewählt ist/sind,
das (C₃₋₇)-Cycloalkyl oder 4- bis 7-gliedrige Heterocycloalkyl gegebenenfalls mit 1 bis 3 Gruppe(n) substituiert ist, die jeweils unabhängig aus Halogen, -CF₃, Hydroxyl, Amino, (C₁₋₄)-Alkyl)amino-, ((C₁₋₄)-Alkyl)((C₁₋₄)-alkyl)amino-, (C₁₋₄)-Alkyl, Phenyl-(C₁₋₄)alkyl-, Hydroy-(C₁₋₄)alkyl-, Oxo und (C₁₋₄)-Alkoxy ausgewählt ist/sind, und
das Aryl ein aromatischer, monovalenter monocyclischer oder bicyclischer Kohlenwasserstoffrest mit 6 bis 10 Kohlenstoff-Ringatomen ist, der an einen oder mehrere Cycloalkylring(e) kondensiert sein kann; und das Heteroaryl ein aromatischer monovalenter monocyclischer oder bicyclischer Rest mit 5 bis 10 Ringatomen, einschliesslich 1 bis 4 Heteroatomen, ausgewählt aus Stickstoff, Sauerstoff und Schwefel, ist, und wobei das Aryl oder Heteroaryl gegebenenfalls mit 1 bis 3 Gruppe(n) substituiert ist, die jeweils unabhängig aus Halogen, -CF₃, Hydroxyl, Amino, ((C₁₋₄)-Alkyl)amino-, ((C₁₋₄)-Alkyl)((C₁₋₄)-alkyl)amino-, (C₁₋₄)-Alkyl, Phenyl-(C₁₋₄)alkyl, Hydroxy-(C₁₋₄)alkyl- und (C₁₋₄)-Alkoxy ausgewählt ist/sind;
R³ Methyl, Hydroxymethyl, Trifluormethyl, Carboxy oder -CO₂(C₁₋₄)-Alkyl ist;
R⁴ H, Methyl, Hydroxymethyl, Trifluormethyl, Carboxy oder -CO₂(C₁₋₄)-Alkyl ist;
R⁵ H oder (C₁₋₃)-Alkyl ist;
oder ein Salz davon oder ein Hydrat davon.

2. Verbindung, Salz oder Hydrat gemäss Anspruch 1, worin:
R² -SR^{a} oder -SO₂R^{a} ist, worin R^{a} (C₁₋₆)-Alkyl, (C₃₋₇)-Cycloalkyl, 4- bis 7-gliedriges Heterocycloalkyl, Aryl oder Heteroaryl ist, wobei:
das (C₁₋₆)-Alkyl gegebenenfalls mit einer oder zwei Gruppe(n) substituiert ist, die jeweils unabhängig aus Cyano, Hydroxyl, (C₁₋₆)-Alkoxy, (C₁₋₆)-Alkoxy-(C₂₋₆)-alkoxy, -CO₂H, -CO₂(C₁₋₄)-Alkyl, -SO₂(C₁₋₄)-Alkyl, (C₃₋₇)-Cycloalkyl, Phenyl, 5- bis 6-gliedrigem Heteroaryl, 9- bis 10-gliedrigem Heteroaryl, 4- bis 7-gliedrigem Heterocycloalkyl und (Phenyl)(C₁₋₄-alkyl)-amino- ausgewählt ist/sind, wobei das (C₃₋₇)-Cycloalkyl, Phenyl, (Phenyl)(C₁₋₄-alkyl)-amino-, 5- bis 6-gliedrige Heteroaryl, 9- bis 10-gliedrige Heteroaryl oder 4- bis 7-gliedrige Heterocycloalkyl gegebenenfalls mit 1 bis 3 Gruppe(n) substituiert ist, die unabhängig aus Halogen, -CF₃, (C₁₋₄)-Alkyl, Hydroxy-(C₁₋₄)-alkyl und (C₁₋₄)-Alkoxy ausgewählt ist/sind,
das (C₃₋₇)-Cycloalkyl oder 4- bis 7-gliedrige Heterocycloalkyl gegebenenfalls mit 1 bis 3 Gruppe(n) substituiert ist, die jeweils unabhängig aus Halogen, -CF₃, Hydroxyl, Amino, (C₁₋₄)-Alkyl, Phenyl-(C₁₋₄)alkyl-, Hydroxy-(C₁₋₄)alkyl-, Oxo und (C₁₋₄)-Alkoxy ausgewählt ist/sind, und
das Aryl ein aromatischer, monovalenter monocyclischer oder bicyclischer Kohlenwasserstoffrest mit 6 bis 10 Kohlenstoff-Ringatomen ist, der an einen oder mehrere Cycloalkylring(e) kondensiert sein kann; und das Heteroaryl ein aromatischer monovalenter monocyclischer oder bicyclischer Rest mit 5 bis 10 Ringatomen, einschliesslich 1 bis 4 Heteroatomen, ausgewählt aus Stickstoff, Sauerstoff und Schwefel, ist, und wobei das Aryl oder Heteroaryl gegebenenfalls mit 1 bis 3 Gruppe(n) substituiert ist, die jeweils unabhängig aus Halogen, -CF₃, Hydroxyl, Amino, (C₁₋₄)-Alkyl, Phenyl-(C₁₋₄)alkyl, Hydroxy-(C₁₋₄)alkyl- und (C₁₋₄)-Alkoxy ausgewählt ist/sind;
R³ Methyl oder Trifluormethyl ist;
R⁴ H, Methyl oder Trifluormethyl ist; und
R⁵ H oder (C₁₋₃)-Alkyl ist.

3. Verbindung, Salz oder Hydrat gemäss Anspruch 1 oder Anspruch 2, worin R¹ H ist.

4. Verbindung, Salz oder Hydrat gemäss irgendeinem der Ansprüche 1 bis 3, worin R² -SO₂R^{a} ist.

5. Verbindung, Salz oder Hydrat gemäss irgendeinem der Ansprüche 1 bis 4, worin R^{a} (C₁₋₆)-Alkyl, wobei das (C₁₋₆)-Alkyl gegebenenfalls mit einer oder zwei Gruppe(n) substituiert ist, die jeweils unabhängig aus Hydroxyl, (C₁₋₄)-Alkoxy, -CO₂(C₁₋₄)-Alkyl, -SO₂(C₁₋₄)-Alkyl und (C₃₋₆)-Cycloalkyl ausgewählt ist/sind, Phenyl, 4- bis 6-gliedriges Heterocycloalkyl, 5- bis 6-gliedriges Heteroaryl oder 9- bis 10-gliedriges Heteroaryl ist, wobei das (C₃₋₆)-Cycloalkyl, Phenyl, 4-bis 6-gliedrige Heterocycloalkyl, 5- bis 6-gliedrige Heteroaryl oder 9- bis 10-gliedrige Heteroaryl gegebenenfalls mit 1 bis 3 Gruppe(n) substituiert ist, die jeweils unabhängig aus Halogen, -CF₃, Hydroxyl, Amino, (C₁₋₄)-Alkyl, Phenyl-(C₁₋₄)alkyl-, Hydroxy-(C₁₋₄)alkyl- und (C₁₋₄)-Alkoxy ausgewählt ist/sind.

6. Verbindung, Salz oder Hydrat gemäss irgendeinem der Ansprüche 1 bis 5, worin R^{a} -CH₃, -CF₃, -CH₂CF₃, -CH(CH₃)₂, -C(CH₃)₃, -CH₂CH₂OH, (4-Fluor)-tetrahydro-2H-pyran-4-yl, (4-Methyl)-tetrahydro-2H-pyran-4-yl, -CH₂-Tetrahydro-2H-furan-2-yl, Tetrahydro-2H-furan-3-yl, 2-Methyl-tetrahydro-2H-furan-3-yl oder Tetrahydro-2H-pyran-4-yl ist.

7. Verbindung, Salz oder Hydrat gemäss irgendeinem der Ansprüche 1 bis 6, worin R⁵ H oder Methyl ist.

8. Verbindung, Salz oder Hydrat gemäss irgendeinem der Ansprüche 1 bis 7, worin R³ und R⁴ beide Methyl sind und R⁵ H ist.

9. Verbindung, Salz oder Hydrat gemäss Anspruch 1, worin R¹ H oder -CH₃ ist; R² -SR^{a}, -SOR^{a} oder -SO₂R^{a} ist, worin R^{a} (C₁₋₄)-Alkyl, Halogen-(C₁₋₄)-alkyl, Hydroxy-(C₁₋₄)-alkyl, 5- bis 6-gliedriges Heterocycloalkyl oder -(C₁₋₂)-Alkyl-(5- bis 6-gliedriges Heterocycloalkyl) ist, wobei jedes Heterocycloalkyl ein Heteroatom, ausgewählt aus N, O und S, enthält; R³ Methyl, Hydroxymethyl oder Carboxy ist und R⁴ H, Methyl oder Trifluormethyl ist oder R³ Methyl ist und R⁴ H, Methyl, Trifluormethyl, Hydroxymethyl- oder Ethyl-carboxy- ist, und R⁵ H oder (C₁₋₃)-Alkyl ist.

10. Verbindung gemäss Anspruch 1, die
6-[(1,1-Dimethylethyl)sulfonyl]-N-(4,5-dimethyl-1H-pyrazol-3-yl)-4-chinolinamin,
N-(4,5-Dimethyl-1H-pyrazol-3-yl)-6-(isopropylsulfonyl)-chinolin-4-amin,
N-(4,5-Dimethyl-1H-pyrazol-3-yl)-6-((tetrahydro-2H-pyran-4-yl)sulfonyl)chinolin-4-amin,
N-(3,4-Dimethyl-1H-pyrazol-5-yl)-6-(methylsulfonyl)-4-chinolinamin,
N-(4,5-Dimethyl-1H-pyrazol-3-yl)-6-[(trifluormethyl)-sulfonyl]-4-chinolinamin,
6-[(1,1-Dimethylethyl)sulfonyl]-N-[4-methyl-5-(trifluormethyl)-1H-pyrazol-3-yl]-4-chinolinamin,
6-[(1,1-Dimethylethyl)sulfonyl]-N-(1,3,4-trimethyl-1H-pyrazol-5-yl)-4-chinolinamin,
6-[(1-Methylethyl)sulfonyl]-N-[4-methyl-5-(trifluormethyl)-1H-pyrazol-3-yl]-4-chinolinamin,
N-(4-Methyl-5-(trifluormethyl)-1H-pyrazol-3-yl)-6-((tetrahydro-2H-pyran-4-yl)sulfonyl)chinolin-4-amin,
N-(3,4-Dimethyl-1H-pyrazol-5-yl)-6-(((tetrahydrofuran-2-yl)methyl)sulfonyl)chinolin-4-amin,
N-(3,4-Dimethyl-1H-pyrazol-5-yl)-6-[(2,2,2-trifluorethyl)sulfonyl]-4-chinolinamin,
6-(tert-Butylsulfonyl)-N-(4,5-dimethyl-1H-pyrazol-3-yl)-N-methylchinolin-4-amin,
(R)-6-(tert-Butylsulfinyl)-N-(4,5-dimethyl-1H-pyrazol-3-yl)chinolin-4-amin,
(S)-6-(tert-Butylsulfinyl)-N-(4,5-dimethyl-1H-pyrazol-3-yl)chinolin-4-amin,
N-(4,5-Dimethyl-1H-pyrazol-3-yl)-6-((4-fluortetrahydro-2H-pyran-4-yl)sulfonyl)chinolin-4-amin,
N-(4,5-Dimethyl-1H-pyrazol-3-yl)-6-((4-methyltetrahydro-2H-pyran-4-yl)sulfonyl)chinolin-4-amin,
N-(4,5-Dimethyl-1H-pyrazol-3-yl)-6-(tetrahydro-3-furanylsulfonyl)-4-chinolinamin,
N-(4,5-Dimethyl-1H-pyrazol-3-yl)-6-[(2-methyltetrahydro-3-furanyl)sulfonyl]-4-chinolinamin,
N-(3,4-Dimethyl-1H-pyrazol-5-yl)-6-(tetrahydro-2H-pyran-4-ylthio)-4-chinolinamin,
2-({4-[(4,5-Dimethyl-1H-pyrazol-3-yl)amino]-6-chinolinyl}sulfonyl)ethanol,
Ethyl-3-({6-[(1,1-dimethylethyl)sulfonyl]-4-chinolinyl}-amino)-4-methyl-1H-pyrazol-5-carboxylat,
3-({6-[(1,1-Dimethylethyl)sulfonyl]-4-chinolinyl}amino)-4-methyl-1H-pyrazol-5-yl]methanol,
[3-({6-[(1,1-Dimethylethyl)sulfonyl]-4-chinolinyl}-amino)-5-methyl-1H-pyrazol-4-yl]methanol,
3-({6-[(1,1-Dimethylethyl)sulfonyl]-4-chinolinyl}amino)-5-methyl-1H-pyrazol-4-carbonsäure,
(R)-N-(3,4-Dimethyl-1H-pyrazol-5-yl)-6-((tetrahydro-2H-pyran-4-yl)sulfinyl)chinolin-4-amin und
(S)-N-(3,4-Dimethyl-1H-pyrazol-5-yl)-6-((tetrahydro-2H-pyran-4-yl)sulfinyl)chinolin-4-amin
oder ein Salz davon oder ein Hydrat davon ist.

11. Pharmazeutische Zusammensetzung, die eine Verbindung, ein Salz oder ein Hydrat gemäss irgendeinem der Ansprüche 1 bis 10, wobei das Salz ein pharmazeutisch annehmbares Salz ist, und einen oder mehrere pharmazeutisch annehmbare(n) Hilfsstoff(e) umfasst.

12. Verbindung, Salz oder Hydrat gemäss irgendeinem der Ansprüche 1 bis 10, wobei das Salz ein pharmazeutisch annehmbares Salz zur Verwendung in der Therapie ist.

13. Verbindung, Salz oder Hydrat gemäss irgendeinem der Ansprüche 1 bis 10, wobei das Salz ein pharmazeutisch annehmbares Salz zur Verwendung bei der Behandlung einer Erkrankung ist, die durch Inhibierung von RIP2-Kinase vermittelt wird, wobei die durch RIP2-Kinase vermittelte Erkrankung aus Uveitis, mit Interleukin-1-Umwandlungsenzym assoziiertem Fiebersyndrom, Dermatitis, akuter Lungenschädigung, Diabetes mellitus Typ 2, Arthritis, rheumatoider Arthritis, Colitis ulcerosa, Morbus Crohn, früh einsetzender und extraintestinaler entzündlicher Darmerkrankung, Prävention von ischämischen Reperfusionssschäden im Bereich der Transplantation fester Organe, nicht-alkoholischer Steatohepatitis, alkoholischer Steatohepatitis, Autoimmun-Hepatitis, Asthma, Transplantat-gegen-Wirt-Erkrankung, systemischem Lupus erythematosus, Multipler Sklerose, Sarkoidose, Blau-Syndrom/früh einsetzender Sarkoidose, Wegner-Granulomatose und interstitieller Lungenerkrankung ausgewählt ist.

14. Verwendung einer Verbindung, eines Salzes oder eines Hydrats gemäss irgendeinem der Ansprüche 1 bis 10, wobei das Salz ein pharmazeutisch annehmbares Salz ist, bei der Herstellung eines Arzneimittels zur Verwendung bei der Behandlung einer Erkrankung, die durch Inhibierung von RIP2-Kinase vermittelt wird, wobei die durch RIP2-Kinase vermittelte Erkrankung aus Uveitis, mit Interleukin-1-Umwandlungsenzym assoziiertem Fiebersyndrom, Dermatitis, akuter Lungenschädigung, Diabetes mellitus Typ 2, Arthritis, rheumatoider Arthritis, Colitis ulcerosa, Morbus Crohn, früh einsetzender und extraintestinaler entzündlicher Darmerkrankung, Prävention von ischämischen Reperfusionssschäden im Bereich der Transplantation fester Organe, nicht-alkoholischer Steatohepatitis, alkoholischer Steatohepatitis, Autoimmun-Hepatitis, Asthma, Transplantat-gegen-Wirt-Erkrankung, systemischem Lupus erythematosus, Multipler Sklerose, Sarkoidose, Blau-Syndrom/früh einsetzender Sarkoidose, Wegner-Granulomatose und interstitieller Lungenerkrankung ausgewählt ist.

## Revendications

1. Composé de formule (I) : 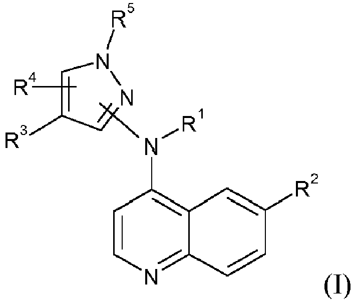 dans laquelle :
R¹ est H, un groupe -SO₂(alkyle en C₁₋C₄), - CO(alkyle en C₁₋C₄), ou alkyle en C₁-C₄ ;
R² est -SR^{a}, -SOR^{a}, ou -SO₂R^{a}, dans lequel R^{a} est un groupe alkyle en C₁-C₆, halogéno(alkyle en C₁₋C₆), cycloalkyle en C₃-C₇, hétérocycloalkyle à 4 à 7 chaînons, aryle, ou hétéroaryle, dans lequel :
ledit groupe alkyle en C₁-C₆ est facultativement substitué par un ou deux groupes chacun indépendamment sélectionnés parmi les groupes cyano, hydroxyle, (alcoxy en C₁₋C₆), (alcoxy en C₁₋C₆) (alcoxy en C₂₋C₆), - CO₂H, -CO₂(alkyle en C₁₋C₄), -SO₂(alkyle en C₁₋C₄), cycloalkyle en C₃-C₇, phényle, hétéroaryle à 5 à 6 chaînons, hétéroaryle à 9 à 10 chaînons, hétérocycloalkyle à 4 à 7 chaînons et (phényl)(alkyl en C₁₋C₄)amino-, dans lequel ledit groupe cycloalkyle en C₃-C₇, phényle, (phényl)(alkyl en C₁₋C₄)amino-, hétéroaryle à 5 à 6 chaînons, hétéroaryle à 9 à 10 chaînons ou hétérocycloalkyle à 4 à 7 chaînons est facultativement substitué par 1 à 3 groupes chacun indépendamment sélectionnés parmi les groupes halogène, -CF₃, hydroxyle, amino, (alkyl en C₁₋C₄)amino-, (alkyl en C₁₋C₄) (alkyl en C₁₋C₄) amino-, alkyle en C₁₋C₄, phényl (alkyle en C₁₋C₄)-, hydroxy (alkyle en C₁₋C₄) et alcoxy en C₁₋C₄,
ledit groupe cycloalkyle en C₃-C₇ ou hétérocycloalkyle à 4 à 7 chaînons est facultativement substitué par 1 à 3 groupes chacun indépendamment sélectionnés parmi les groupes halogène, -CF₃, hydroxyle, amino, (alkyl en C₁₋C₄)amino-, (alkyl en C₁₋C₄)(alkyl en C₁₋C₄)amino-, alkyle en C₁₋C₄, phényl(alkyle en C₁₋C₄)-, hydroxy (alkyle en C₁₋C₄)-, oxo et alcoxy en C₁-C₄, et
ledit groupe aryle est un radical hydrocarboné monocyclique ou bicyclique, monovalent, aromatique, contenant de 6 à 10 atomes de carbone cycliques, qui peut être fusionné à un ou plusieurs cycles cycloalkyle ; et ledit groupe hétéroaryle est un radical monocyclique ou bicyclique monovalent contenant de 5 à 10 atomes cycliques, comprenant de 1 à 4 hétéroatomes sélectionnés parmi les atomes d'azote, d'oxygène et de soufre, et dans lequel ledit groupe aryle ou hétéroaryle est facultativement substitué par 1 à 3 groupes chacun indépendamment sélectionnés parmi les groupes halogène, -CF₃, hydroxyle, amino, (alkyl en C₁-C₄) amino-, (alkyl en C₁₋C₄) (alkyl en C₁-C₄) amino-, alkyle en C₁-C₄, phényl(alkyle en C₁₋C₄)-, hydroxy (alkyle en C₁₋C₄) - et alcoxy en C₁₋C₄ ;
R³ est un groupe méthyle, hydroxyméthyle, trifluorométhyle, carboxy, ou -CO₂(alkyle en C₁₋C₄) ;
R⁴ est H, un groupe méthyle, hydroxyméthyle, trifluorométhyle, carboxy, ou -CO₂(alkyle en C₁₋C₄) ;
R⁵ est H ou un groupe alkyle en C₁₋C₃ ;
ou un sel ou un hydrate, de ceux-ci.

2. Composé, sel ou hydrate selon la revendication 1, dans lequel :
R² est -SR^{a}, ou -SO₂R^{a}, dans lequel R^{a} est un groupe alkyle en C₁₋C₆, cycloalkyle en C₃-C₇, hétérocycloalkyle à 4 à 7 chaînons, aryle, ou hétéroaryle, dans lequel :
ledit groupe alkyle en C₁₋C₆ est facultativement substitué par un ou deux groupes chacun indépendamment sélectionnés parmi un groupe cyano, hydroxyle, alcoxy en C₁₋C₆, (alcoxy en C₁₋C₆) (alcoxy en C₂₋C₆), -CO₂H, - CO₂(alkyle en C₁₋C₄), -SO₂(alkyle en C₁₋C₄), cycloalkyle en C₃-C₇, phényle, hétéroaryle à 5 à 6 chaînons, hétéroaryle à 9 à 10 chaînons, hétérocycloalkyle à 4 à 7 chaînons et (phényl)(alkyl en C₁₋C₄)amino-, dans lequel ledit groupe cycloalkyle en C₃-C₇, phényle, (phényl)(alkyl en C₁₋C₄)amino-, hétéroaryle à 5 à 6 chaînons, hétéroaryle à 9 à 10 chaînons ou hétérocycloalkyle à 4 à 7 chaînons est facultativement substitué par 1 à 3 groupes chacun indépendamment sélectionnés parmi les groupes halogène, -CF₃, alkyle en C₁-C₄, hydroxy (alkyle en C₁-C₄) et alcoxy en C₁₋C₄,
ledit groupe cycloalkyle en C₃-C₇ ou hétérocycloalkyle à 4 à 7 chaînons est facultativement substitué par 1 à 3 groupes chacun indépendamment sélectionnés parmi les groupes halogène, -CF₃, hydroxyle, amino, alkyle en C₁-C₄, phényl(alkyle en C₁₋C₄)-, hydroxy (alkyle en C₁₋C₄)-, oxo et alcoxy en C₁₋C₄, et
ledit groupe aryle est un radical hydrocarboné monocyclique ou bicyclique monovalent, aromatique, contenant de 6 à 10 atomes de carbone cycliques, qui peut être fusionné à un ou plusieurs cycles cycloalkyle ; et ledit groupe hétéroaryle est un radical monocyclique ou bicyclique monovalent contenant de 5 à 10 atomes cycliques, comprenant de 1 à 4 hétéroatomes sélectionnés parmi les atomes d'azote, d'oxygène et de soufre, et dans lequel ledit groupe aryle ou hétéroaryle est facultativement substitué par 1 à 3 groupes chacun indépendamment sélectionnés parmi les groupes halogène, -CF₃, hydroxyl, amino, alkyle en C₁-C₄, phényl(alkyle en C₁₋C₄)-, hydroxy (alkyle en C₁₋C₄) - et alcoxy en C₁₋C₄ ;
R³ est un groupe méthyle ou trifluorométhyle ;
R⁴ est H, un groupe méthyle ou trifluorométhyle ;
R⁵ est H ou un groupe alkyle en C₁₋C₃.

3. Composé, sel ou hydrate selon la revendication 1 ou la revendication 2, dans lequel R¹ est H.

4. Composé, sel ou hydrate selon l'une quelconque des revendications 1 à 3, dans lequel R² est -SO₂R^{a}.

5. Composé, sel ou hydrate selon l'une quelconque des revendications 1 à 4, dans lequel R^{a} est un groupe alkyle en C₁₋C₆, dans lequel ledit groupe alkyle en C₁₋C₆ est facultativement substitué par un ou deux groupes chacun indépendamment sélectionnés parmi des groupes hydroxyle, alcoxy en C₁₋C₄, -CO₂ (alkyle en C₁₋C₄), - SO₂(alkyle en C₁₋C₄), et cycloalkyle en C₃₋C₆, phényle, hétérocycloalkyle à 4 à 6 chaînons, hétéroaryle à 5 à 6 chaînons, ou hétéroaryle à 9 à 10, dans lequel ledit groupe cycloalkyle en C₃-C₆, phényle, hétérocycloalkyle à 4 à 6 chaînons, hétéroaryle à 5 à 6, ou hétéroaryle à 9 à 10 chaînons est facultativement substitué par 1 à 3 groupes chacun indépendamment sélectionnés parmi les groupes halogène, -CF₃, hydroxyle, amino, alkyle en C₁-C₄, phényl(alkyle en C₁₋C₄)-, hydroxy (alkyle en C₁₋C₄) - et alcoxy en C₁₋C₄.

6. Composé, sel ou hydrate selon l'une quelconque des revendications 1 à 5, dans lequel R^{a} est -CH₃, -CF₃, -CH₂CF₃, -CH(CH₃)₂, -C(CH₃)₃, -CH₂CH₂OH, (4-fluoro)-tétrahydro-2H-pyran-4-yl, (4-méthyl)-tétrahydro-2H-pyran-4-yl, -CH₂-tétrahydro-2H-furan-2-yl, tétrahydro-2H-furan-3-yl, 2-méthyl-tétrahydro-2H-furan-3-yl ou tétrahydro-2H-pyran-4-yl.

7. Composé, sel ou hydrate selon l'une quelconque des revendications 1 à 6, dans lequel R⁵ est H ou un groupe méthyle.

8. Composé, sel ou hydrate selon l'une quelconque des revendications 1 à 7, dans lequel R³ et R⁴ sont tous les deux un groupe méthyle et R⁵ est H.

9. Composé, sel ou hydrate selon la revendication 1, dans lequel R¹ est H ou -CH₃ ; R² est -SR^{a}, -SOR^{a}, ou -SO₂R^{a} ; dans lequel R^{a} est un groupe alkyle en C₁₋C₄, halogéno(alkyle en C₁₋C₄), hydroxy (alkyle en C₁₋C₄), hétérocycloalkyle à 5 à 6 chaînons, ou -(alkyl en C₁-C₂)-(hétérocycloalkyle à 5 à 6 chaînons), dans lequel l'un quelconque desdits groupes hétérocycloalkyle contient un hétéroatome sélectionné parmi N, O et S ; R³ est un groupe méthyle, hydroxyméthyle ou carboxy et R⁴ est H, un groupe méthyle ou trifluorométhyle, ou R³ est un groupe méthyle et R⁴ est H, un groupe méthyle, trifluorométhyle, hydroxyméthyl- ou éthyl-carboxy- ; et R⁵ est H ou un groupe alkyle en C₁₋C₃.

10. Composé selon la revendication 1, qui est
la 6-[(1,1-diméthyléthyl)sulfonyl]-N-(4,5-diméthyl-1*H*-pyrazol-3-yl)-4-quinoléinamine,
la *N*-(4,5-diméthyl-1*H*-pyrazol-3-yl)-6-(isopropylsulfonyl)quinoléin-4-amine,
la *N*-(4,5-diméthyl-1*H*-pyrazol-3-yl)-6-((tétrahydro-2H-pyran-4-yl)sulfonyl)quinoléin-4-amine,
la N-(3,4-diméthyl-1*H*-pyrazol-5-yl)-6-(méthylsulfonyl)-4-quinoléinamine,
la N-(4,5-diméthyl-1*H*-pyrazol-3-yl)-6-[(trifluorométhyl)sulfonyl]-4-quinoléinamine,
la 6-[(1,1-diméthyléthyl)sulfonyl]-N-[4-méthyl-5-(trifluorométhyl)-1*H*-pyrazol-3-yl]-4-quinoléinamine,
la 6-[(1,1-diméthyléthyl)sulfonyl]-N-(1,3,4-triméthyl-1*H*-pyrazol-5-yl)-4-quinoléinamine,
la 6-[(1-méthyléthyl)sulfonyl]-N-[4-méthyl-5-(trifluorométhyl)-1*H*-pyrazol-3-yl]-4-quinoléinamine,
la *N*-(4-méthyl-5-(trifluorométhyl)-1*H*-pyrazol-3-yl)-6-((tétrahydro-2*H*-pyran-4-yl)sulfonyl)quinoléin-4-amine,
la *N*-(3,4-diméthyl-1H-pyrazol-5-yl)-6-(((tétrahydrofuran-2-yl)méthyl)sulfonyl)quinoléin-4-amine,
la N-(3,4-diméthyl-1*H*-pyrazol-5-yl)-6-[(2,2,2-trifluoroéthyl)sulfonyl]-4-quinoléinamine,
la 6-(tert-butylsulfonyl)-*N*-(4,5-diméthyl-1H-pyrazol-3-yl)-N-méthylquinoléin-4-amine,
la (*R*)-6-(tert-butylsulfinyl)-*N*-(4,5-diméthyl-1H-pyrazol-3-yl)quinoléin-4-amine,
la (*S*)-6-(tert-butylsulfinyl)-*N*-(4,5-diméthyl-1H-pyrazol-3-yl)quinoléin-4-amine,
la *N*-(4,5-diméthyl-1H-pyrazol-3-yl)-6-((4-fluorotétrahydro-2H-pyran-4-yl)-sulfonyl)quinoléin-4-amine,
la *N*-(4,5-diméthyl-1*H*-pyrazol-3-yl)-6-((4-méthyltétrahydro-2H-pyran-4-yl)sulfonyl)quinoléin-4-amine,
la *N*-(4,5-diméthyl-1*H*-pyrazol-3-yl)-6-(tétrahydro-3-furanylsulfonyl)-4-quinoléinamine,
la *N*-(4,5-diméthyl-1*H*-pyrazol-3-yl)-6-[(2-méthyltétrahydro-3-furanyl)sulfonyl]-4-quinoléinamine,
la *N*-(3,4-diméthyl-1*H*-pyrazol-5-yl)-6-(tétrahydro-2H-pyran-4-ylthio)-4-quinoléinamine,
le 2-({4-[(4,5-diméthyl-1*H*-pyrazol-3-yl)amino]-6-quinoléinyl}sulfonyl)éthanol,
le 3-({6-[(1,1-diméthyléthyl)sulfonyl]-4-quinoléinyl}amino)-4-méthyl-1*H*-pyrazole-5-carboxylate d'éthyle,
le 3-({6-[(1,1-diméthyléthyl)sulfonyl]-4-quinoléinyl}amino)-4-méthyl-1*H*-pyrazol-5-yl]méthanol,
le [3-({6-[(1,1-diméthyléthyl)sulfonyl]-4-quinoléinyl}amino)-5-méthyl-1*H*-pyrazol-4-yl]méthanol,
l'acide 3-({6-[(1,1-diméthyléthyl)sulfonyl]-4-quinoléinyl}amino)-5-méthyl-1*H*-pyrazole-4-carboxylique,
la (*R*)-*N*-(3,4-diméthyl-1H-pyrazol-5-yl)-6-((tétrahydro-2H-pyran-4-yl)sulfinyl)quinoléin-4-amine, et
la (*S*)-*N*-(3,4-diméthyl-1H-pyrazol-5-yl)-6-((tétrahydro-2H-pyran-4-yl)sulfinyl)quinoléin-4-amine,
ou un sel de celui-ci ou un hydrate de celui-ci.

11. Composition pharmaceutique comprenant le composé, le sel, ou l'hydrate selon l'une quelconque des revendications 1 à 10, dans lequel le sel est un sel pharmaceutiquement acceptable, et un ou plusieurs excipients pharmaceutiquement acceptables.

12. Composé, sel ou hydrate selon l'une quelconque des revendications 1 à 10, dans lequel le sel est un sel pharmaceutiquement acceptable, pour son utilisation en thérapie.

13. Composé, sel ou hydrate selon l'une quelconque des revendications 1 à 10, dans lequel le sel est un sel pharmaceutiquement acceptable, pour son utilisation dans le traitement d'une maladie médiée par l'inhibition de la kinase RIP2, dans lequel la maladie médiée par l'inhibition de la kinase RIP2 est sélectionnée parmi l'uvéite, le syndrome de fièvre associé à l'enzyme de conversion de l'interleukine 1, la dermite, une lésion pulmonaire aiguë, le diabète sucré de type 2, l'arthrite, la polyarthrite rhumatoïde, la recto-colite hémorragique, la maladie de Crohn, une affection abdominale inflammatoire extra-intestinale d'apparition précoce, la prévention d'une lésion d'ischémie reperfusion dans une transplantation d'organe solide, la stéatose hépatique non alcoolique, la stéatose hépatique alcoolique, l'hépatite auto-immune, l'asthme, la maladie du greffon contre l'hôte, le lupus érythémateux systémique, la sclérose en plaques, la sarcoïdose, le syndrome de Blau/la sarcoïdose à début précoce, la granulomatose de Wegner, et une maladie pulmonaire interstitielle.

14. Utilisation d'un composé, d'un sel ou d'un hydrate selon l'une quelconque des revendications 1 à 10, dans laquelle le sel est un sel pharmaceutiquement acceptable, dans la fabrication d'un médicament pour son utilisation dans le traitement d'une maladie médiée par l'inhibition de la kinase RIP2, dans laquelle la maladie médiée par l'inhibition de la kinase RIP2 est sélectionnée parmi l'uvéite, le syndrome de fièvre associé à l'enzyme de conversion de l'interleukine 1, la dermite, une lésion pulmonaire aiguë, le diabète sucré de type 2, l'arthrite, la polyarthrite rhumatoïde, la recto-colite hémorragique, la maladie de Crohn, une affection abdominale inflammatoire extra-intestinale d'apparition précoce, la prévention d'une lésion d'ischémie reperfusion dans une transplantation d'organe solide, la stéatose hépatique non alcoolique, la stéatose hépatique alcoolique, l'hépatite auto-immune, l'asthme, la maladie du greffon contre l'hôte, le lupus érythémateux systémique, la sclérose en plaques, la sarcoïdose, le syndrome de Blau/la sarcoïdose à début précoce, la granulomatose de Wegner, et une maladie pulmonaire interstitielle.
